(19) Europäisches Patentamt
European Patent Office
Office européen des brevets

(11) EP 4 394 351 B1

(12) EUROPEAN PATENT SPECIFICATION

(45) Date of publication and mention of the grant of the patent:
**11.03.2026 Bulletin 2026/11**

(21) Application number: **23218059.6**

(22) Date of filing: **19.12.2023**

(51) International Patent Classification (IPC):
***G01N 15/14*** (2024.01) ***G01N 15/01*** (2024.01)
***G01N 21/64*** (2006.01) ***G01N 33/49*** (2006.01)

(52) Cooperative Patent Classification (CPC):
**G01N 15/1459; G01N 15/01; G01N 15/14;**
**G01N 33/575;** G01N 2015/016; G01N 2015/1006;
G01N 2015/1486

(54) **BONE MARROW ASPIRATE ANALYSIS METHOD, SAMPLE ANALYZER, AND COMPUTER PROGRAM**

VERFAHREN ZUR ANALYSE VON KNOCHENMARKASPIRAT, PROBENANALYSATOR UND COMPUTERPROGRAMM

PROCÉDÉ D’ANALYSE D’ASPIRAT DE MOELLE OSSEUSE, ANALYSEUR D’ÉCHANTILLONS ET PROGRAMME INFORMATIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.12.2022 JP 2022212357**

(43) Date of publication of application:
**03.07.2024 Bulletin 2024/27**

(73) Proprietor: **SYSMEX CORPORATION**
**Kobe-shi**
**Hyogo 651-0073 (JP)**

(72) Inventors:
- **Hayashi, Fumiaki**
  **Kobe-shi, Hyogo, 651-0073 (JP)**
- **Yoshimoto, Michiko**
  **Kobe-shi, Hyogo, 651-0073 (JP)**
- **Oguni, Shinichiro**
  **Kobe-shi, Hyogo, 651-0073 (JP)**
- **Nagai, Takaaki**
  **Kobe-shi, Hyogo, 651-0073 (JP)**
- **Nagai, Yuya**
  **Kobe-shi, Hyogo, 650-0047 (JP)**
- **Maruoka, Hayato**
  **Kobe-shi, Hyogo, 650-0047 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 520 926**
**EP-A1- 2 703 813**
**EP-A1- 3 654 014**
**EP-A2- 2 267 453**
**US-A1- 2017 074 863**
**US-A1- 2018 136 110**
**US-A1- 2022 291 200**

- **YUSUKE MORI ET AL: "Automation of bone marrow aspirate examination using the XE-2100 automated hematology analyzer", CYTOMETRY PART B CLINICAL CYTOMETRY, WILEY-LISS, HOBOKEN, NJ, US, no. 1, 29 December 2003 (2003-12-29), pages 25 - 31, XP072331665, ISSN: 1552-4949, DOI: 10.1002/CYTO.B.10070**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

CROSS REFERENCE TO RELATED APPLICATIONS

This application claims priority from prior Japanese Patent Application No. 2022-212357, filed on December 28, 2022, entitled "BONE MARROW ASPIRATE ANALYSIS METHOD, SAMPLE ANALYZER, AND COMPUTER PROGRAM.

FIELD OF THE INVENTION

The present invention relates to a bone marrow aspirate analysis method. The present invention relates to a sample analyzer. The present invention relates to a computer program for analyzing bone marrow aspirate.

BACKGROUND OF THE INVENTION

Automation of Bone Marrow Aspirate Examination Using the XE-2100 Automated Hematology Analyzer, by Mori Y., et al., Cytometry Part B (Clinical Cytometry), 2003, vol. 58B, pp. 25-31 (Non-Patent Literature 1), indicates that a method for testing bone marrow aspirate with use of an automated hematology analyzer XE-2100 (SYSMEX CORPORATION) was examined. Non-Patent Literature 1 indicates that pretreatment of bone marrow aspirate is performed by using Stromatolyser IM (hereinafter, also referred to as "IM reagent") as a reagent for measuring immature cells. The IM reagent causes damage to a cell membrane and thus, a nucleus is exposed. However, damage to immature cells is small as compared with damage to mature leukocytes. Therefore, when a cell in the bone marrow aspirate treated by the IM reagent is stained with a reagent for staining nucleic acid, the exposed nucleus of the mature leukocyte is strongly stained and the fluorescence intensity becomes high. Meanwhile, since a nucleic acid-staining dye does not easily permeate an immature cell such as myeloblast and immature granulocyte, the fluorescence intensity becomes low (see FIG. 1). According to Non-Patent Literature 1, myelocytes and mature leukocytes are distinguished from each other and are each counted by XE-2100, by utilizing such difference in stainability.

EP 3 654 014 A1 discloses a bone marrow fluid analysis method and corresponding sample analyzer, for determining diagnosis and therapeutic effects for blood diseases such as hematopoietic disorder and hematopoietic tumor.

There is room for improving the bone marrow aspirate analysis methods disclosed in Non-Patent Literature 1 and EP 3 654 014 A1 from the viewpoint of screening accuracy for hematopoietic tumors. An object of the present invention is to provide a bone marrow aspirate analysis method, a sample analyzer, and a computer program that have enhanced screening accuracy for hematopoietic tumors.

SUMMARY OF THE INVENTION

The inventors of the present invention have paid attention to the fact that the number of cells having high proliferative capacity and high protein production capacity is increased in bone marrow aspirate of a hematopoietic tumor patient, and these cells contain relatively large amounts of nucleic acid. The inventors of the present invention have obtained an idea that cells in bone marrow aspirate are stained by using a fluorescent dye that can stain nucleic acid, and are measured by flow cytometry, and detection of bone marrow aspirate suspected of a hematopoietic tumor is performed based on the obtained fluorescence signal information. The inventors of the present invention have found, through analysis of bone marrow aspirates having no abnormality and bone marrow aspirates having hematopoietic tumors or the symptoms thereof, that bone marrow aspirates having hematopoietic tumors and the other bone marrow aspirates can be distinguished from each other according to a value based on the number of particles for which the fluorescence signal information indicates a threshold value or more. Furthermore, the inventors of the present invention have found that the determination result preferably correlates with a bone marrow aspirate determination result based on microscopy.

The present invention provides a bone marrow aspirate analysis method that includes: measuring a sample containing bone marrow aspirate and a fluorescent dye that can stain nucleic acid, by flow cytometry, and obtaining optical information including fluorescence signal information for a particle in the sample; counting particles for which the fluorescence signal information indicates a threshold value or more, as target cells; and obtaining a screening index for a hematopoietic tumor based on a number of the target cells.

The present invention provides a sample analyzer that includes: a sample preparation unit configured to prepare a sample containing bone marrow aspirate, and a fluorescent dye that can stain nucleic acid; a detector configured to obtain optical information including fluorescence signal information for a particle in the sample; and a controller configured to count particles for which the fluorescence signal information indicates a threshold value or more, as target cells, and the controller obtains a screening index for a hematopoietic tumor based on a number of the target cells.

The present invention provides a computer program for analyzing bone marrow aspirate and causing a computer to execute: obtaining optical information including fluorescence signal information for a particle in a sample containing the bone marrow aspirate and a fluorescent dye that can stain nucleic acid; counting particles for which the fluorescence signal information indicates a threshold value or more, as target cells, based on the optical information; and obtaining a screening index for a hematopoietic tumor based on a number of the target cells.

The present invention can provide information for supporting, for example, screening for a hematopoietic tumor and determination as to whether or not bone marrow aspirate is abnormal.

BRIEF DESCRIPTION OF THE DRAWINGS

FIG. 1 is a schematic diagram illustrating a scattergram obtained by measuring a sample containing bone marrow aspirate and a fluorescent dye, by using a flow cytometer (FCM);
FIG. 2 is a schematic diagram illustrating a scattergram obtained by measuring, by the FCM, a control sample containing peripheral blood of a healthy individual and a fluorescent dye;
FIG. 3A is a schematic diagram illustrating a scattergram obtained by measuring, by the FCM, a sample containing bone marrow aspirate and a fluorescent dye;
FIG. 3B is a schematic diagram illustrating a scattergram obtained by measuring, by the FCM, a control sample containing peripheral blood of a healthy individual and a fluorescent dye;
FIG. 4A is a schematic diagram illustrating a scattergram obtained by measuring, by the FCM, a sample containing bone marrow aspirate and a fluorescent dye;
FIG. 4B is a schematic diagram illustrating a scattergram obtained by measuring, by the FCM, a control sample containing peripheral blood of a healthy individual and a fluorescent dye;
FIG. 4C is a schematic diagram illustrating a scattergram obtained by measuring, by the FCM, a control sample containing peripheral blood of a healthy individual and a fluorescent dye;
FIG. 5A is a schematic diagram illustrating a scattergram obtained by measuring, by the FCM, a sample containing bone marrow aspirate and a fluorescent dye;
FIG. 5B is a schematic diagram illustrating a scattergram obtained by measuring, by the FCM, a control sample containing peripheral blood of a healthy individual and a fluorescent dye;
FIG. 6 is a schematic diagram illustrating a configuration of a sample analyzer according to an embodiment;
FIG. 7 is a perspective view of a configuration of a flow cell;
FIG. 8 is a block diagram illustrating a configuration of an analysis unit;
FIG. 9 is a flow chart showing a flow of an operation performed by the sample analyzer according to the embodiment;
FIG. 10 is a flow chart showing a procedure of a sample preparation process;
FIG. 11A is a flow chart showing a procedure of a measurement data analyzing process;
FIG. 11B is a flow chart showing a procedure of a measurement data analyzing process;
FIG. 11C is a flow chart showing of a procedure of a measurement data analyzing process;
FIG. 11D is a flow chart showing a procedure of a measurement data analyzing process;
FIG. 11E is a flow chart showing a procedure of a measurement data analyzing process;
FIG. 12A is a flow chart showing a procedure of a determination process based on a first ratio;
FIG. 12B is a flow chart showing a procedure of a determination process based on a second ratio;
FIG. 12C is a flow chart showing a procedure of a determination process based on a third ratio;
FIG. 12D is a flow chart showing a procedure of a determination process based on a fifth ratio;
FIG. 12E is a flow chart showing a procedure of a determination process based on a sixth ratio;
FIG. 13 illustrates an example of a scattergram obtained by measuring, by the FCM, a sample containing bone marrow aspirate and a fluorescent dye;
FIG. 14 shows a graph obtained by plotting the first ratio obtained by the analyzer and a ratio obtained by microscopic examination;
FIG. 15 illustrates an example of a scattergram obtained by measuring, by the FCM, a sample containing bone marrow aspirate and a fluorescent dye;
FIG. 16 shows a graph obtained by plotting the second ratio obtained by the analyzer and a ratio obtained by microscopic examination;
FIG. 17 illustrates an example of a scattergram obtained by measuring, by the FCM, a sample containing bone marrow aspirate and a fluorescent dye;
FIG. 18 illustrates an example of a histogram based on forward scattered light intensities of particles included in a third region;
FIG. 19 shows a graph obtained by plotting the third ratio obtained by the analyzer and a ratio obtained by microscopic

examination; and
FIG. 20 illustrates an example of a scattergram obtained by measuring, by the FCM, a sample containing bone marrow aspirate and a fluorescent dye.

DETAILED DESCRIPTION

[1. Bone marrow aspirate analysis method]

In a bone marrow aspirate analysis method (hereinafter, also referred to as "analysis method of the present embodiment") of the present embodiment, a sample containing bone marrow aspirate and a fluorescent dye that can stain nucleic acid is firstly measured by flow cytometry, and optical information including fluorescence signal information about a particle in the sample is obtained.

(Bone marrow aspirate, and cells contained in the bone marrow aspirate)

The "bone marrow aspirate" refers to bone marrow aspirate collected from a subject through bone marrow aspiration or bone marrow biopsy, and a sample containing the bone marrow aspirate. When bone marrow aspirate collected from a subject contains solid contaminants that could be an obstacle for cell measurement, such as spicules and aggregated blood cells, the bone marrow aspirate may be filtered by using a mesh or the like. A chelating agent and/or an anticoagulant agent may be added to the bone marrow aspirate as necessary. Examples of the chelating agent include ethylene diamine tetraacetic acid (EDTA) salt. Examples of the anticoagulant agent include heparin, citric acid, and citrate.
The bone marrow aspirate contains various nucleated cells. Examples of the nucleated cells in the bone marrow aspirate include leukocytes, erythroblast cells, megakaryoblast, promegakaryocyte, bone marrow megakaryocyte, mature megakaryocyte, and plasmacyte (plasma cells). Examples of the "leukocytes" include myeloblast, promyelocyte, myelocyte, metamyelocyte, monoblast, promonocyte, band neutrophil, segmented neutrophil, immature eosinophil, immature basophil, monocyte, eosinophil, basophil, and lymphocyte. Band neutrophils are mature neutrophils, and segmented neutrophils are more mature neutrophils than band neutrophils. The "erythroblast cells" are also referred to as nucleated erythrocytes, and examples thereof include proerythroblast, basophilic erythroblast, polychromatic erythroblast, and orthochromatic erythroblast.
Hereinafter, band neutrophil and segmented neutrophil are collectively referred to also as "neutrophil". Monocyte, lymphocyte, neutrophil, eosinophil, and basophil are collectively referred to also as "mature leukocyte". Monocyte and lymphocyte are collectively referred to also as "mononuclear leukocyte". Neutrophil, eosinophil, and basophil are collectively referred to also as "mature granulocyte".
Leukocytes and erythroblast cells may include tumor cells that appear and increase due to various kinds of hematopoietic tumors such as leukemia and malignant lymphoma. For example, the number of blast cells is increased in acute leukemia, and the number of plasmacytes is increased in plasmacytoma (also called myeloma). The "blast cell" refers to myeloblast, monoblast, and promonocyte.
In the present specification, promyelocyte and myelocyte are also referred to as "immature granulocyte in an early differentiation stage", and metamyelocyte is also referred to as "immature granulocyte in a late differentiation stage". Proerythroblast and basophilic erythroblast are also referred to as "erythroblast in an early differentiation stage", and polychromatic erythroblast and orthochromatic erythroblast are also referred to as "erythroblast in a late differentiation stage". Megakaryoblast, promegakaryocyte, and bone marrow megakaryocyte are also referred to as "megakaryocytic cell". An amount of nucleic acid, in particular, an amount of ribonucleic acid contained in immature granulocyte in an early differentiation stage, erythroblast in an early differentiation stage, a blast cell, a megakaryocytic cell, and plasmacyte, is greater than an amount thereof contained in another nucleated cell. Furthermore, as described above, in hematopoietic tumors, the numbers of blast cells and plasmacytes are increased in bone marrow. In the analysis method of the present embodiment, the above-described sample is measured by an FCM and optical information including fluorescence signal information is obtained in order to selectively detect a nucleated cell having a large amount of nucleic acid as described above, in bone marrow aspirate.
"Particle in sample" refers to a tangible component in the sample. Particles include not only cells but also acellular particles such as remainders of lysed erythrocytes (hereinafter, also referred to as "erythrocyte ghost"), agglutinated platelets, and lipid particles.

(Fluorescent dye)

The "fluorescent dye that can stain nucleic acid" refers to a fluorescent substance that can stain nucleic acid in a cell. Hereinafter, the fluorescent dye that can stain nucleic acid may be simply referred to also as "fluorescent dye". The fluorescent dye is preferably a fluorescent substance that can stain ribonucleic acid (RNA) in a cell. Examples of the

fluorescent dye include propidium iodide, ethidium bromide, ethidium-acridine heterodimer, ethidium diazide, ethidium homodimer-1, ethidium homodimer-2, ethidium monoazide, trimethylenebis[[3-[[4-[[(3-methylbenzothiazol-3-ium)-2-yl] methylene]-1,4-dihydroquinolin]-1-yl]propyl]dimethylaminium]·tetraiodide (TOTO-1), 4-[(3-methylbenzothiazol-2(3H)-ylidene)methyl]-1-[3-(trimethylaminio)propyl]quinolinium·diiodide (TO-PRO-1), N,N,N',N'-tetramethyl-N,N'-bis [3-[4-[3-[(3-methylbenzothiazol-3-ium)-2-yl]-2-propenylidene]-1,4-dihydroquinolin-1-yl]propyl]-1,3-propanediaminium·-tetraiodide (TOTO-3), 2-[3-[[1-[3-(trimethylaminio)propyl]-1,4-dihydroquinolin]-4-ylidene]-1-propenyl]-3-methylbenzothiazol-3-ium·diiodide (TOPRO-3), fluorescent dyes represented by the following formula (I), and combinations thereof. One kind of the fluorescent dye may be contained in the sample, or two or more kinds thereof may be contained therein.

[Chem. 1]

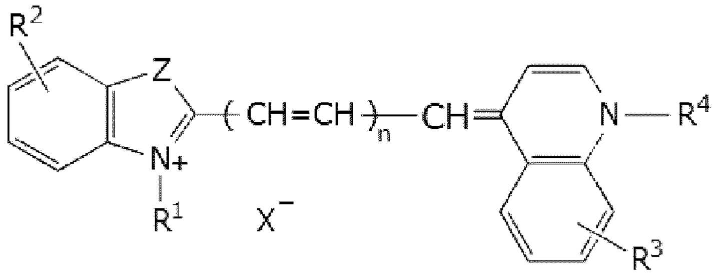

(I)

In Formula (I), $R^1$ and $R^4$ each represent a hydrogen atom, a methyl group, an ethyl group, or a C6 to C18 alkyl group. When one of $R^1$ and $R^4$ is a C6 to C18 alkyl group, the other thereof is a hydrogen atom, a methyl group, or an ethyl group. $R^2$ and $R^3$ are the same or different from each other, and each represent a methyl group, an ethyl group, a methoxy group, or an ethoxy group. Z represents a sulfur atom, an oxygen atom, or a carbon atom having a methyl group. n represents 0, 1, 2, or 3. $X^-$ represents an anion.

In formula (I), the C6 to C18 alkyl group may be linear or branched. Among C6 to C18 alkyl groups, a C6, C8, or C10 alkyl group is preferable.

In formula (I), examples of a substituent of the benzyl group of $R^1$ and $R^4$ include C1 to C20 alkyl groups, C2 to C20 alkenyl groups, and C2 to C20 alkynyl groups. Among them, a methyl group or an ethyl group is particularly preferable.

In formula (I), examples of the alkenyl group of $R^2$ and $R^3$ include C2 to C20 alkenyl groups. Examples of the alkoxy group of $R^2$ and $R^3$ include C1 to C20 alkoxy groups. Among them, a methoxy group or an ethoxy group is particularly preferable.

In formula (I), examples of the anion $X^-$ include halogen ions such as $F^-$, $Cl^-$, $Br^-$, and $I^-$, $CF_3SO_3^-$, $BF_4^-$, and $ClO_4^-$.

As the fluorescent dye represented by above-described formula (I), a fluorescent dye represented by the following formula (II) is preferable.

[Chem. 2]

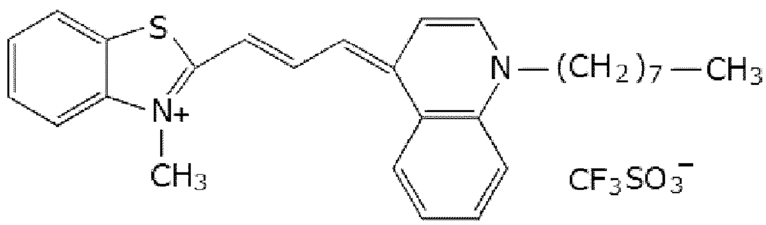

(II)

(Staining reagent)

In the analysis method of the present embodiment, a staining reagent containing a solution of the fluorescent dye is preferably used. A solvent is not particularly limited as long as the above-described fluorescent dye can be dissolved. Examples of the solvent include water, organic solvents, and mixtures thereof. As the organic solvent, a solvent that can be mixed with water is preferable, and examples thereof include C1 to C6 alcohols, ethylene glycol, diethylene glycol, polyethylene glycol, and dimethyl sulfoxide (DMSO). A concentration of the fluorescent dye in the staining reagent can be determined according to a kind of the fluorescent dye as appropriate, and is, for example, 0.01 mg/L or more. The concentration of the fluorescent dye in the staining reagent is preferably 0.1 mg/L or more and more preferably 0.2 mg/L or more. The concentration of the fluorescent dye in the staining reagent is 100 mg/L or less, preferably 90 mg/L or less, and more preferably 80 mg/L or less.

A commercially available staining reagent that contains the fluorescent dye alone may be used, and examples thereof include Fluorocell WDF (SYSMEX CORPORATION) and Stromatolyser 4DS (SYSMEX CORPORATION).

(Surfactant)

In the analysis method of the present embodiment, preferably, the sample further contains a cationic surfactant. The cationic surfactant can lyse erythrocytes in the bone marrow aspirate, and can damage a cell membrane of a nucleated cell to such a degree that the fluorescent dye can permeate the cell. Examples of the cationic surfactant include a quaternary-ammonium-salt surfactant, a pyridinium salt surfactant, and a combination thereof. One kind of the cationic surfactant may be contained in the sample, or two or more kinds thereof may be contained therein. As the quaternary-ammonium-salt surfactant, for example, a surfactant which is represented by the following formula (III) and in which the total number of carbon atoms is 9 to 30 is preferable.

[Chem. 3]

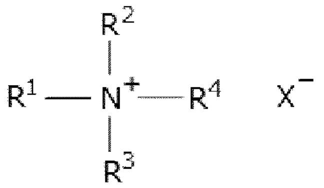

(III)

In formula (III), $R^1$ represents a C6 to C18 alkyl group or alkenyl group, $R^2$ and $R^3$ are the same or different from each other, and each represent a C1 to C4 alkyl group or alkenyl group, $R^4$ represents a C1 to C4 alkyl group or alkenyl group, or a benzyl group, and $X^-$ represents a halogen ion.
In formula (III), R’ preferably represents a C6, C8, C10, *C12,* or C14 alkyl group or alkenyl group, and particularly preferably represents a linear alkyl group. More specifically, $R^1$ represents an octyl group, a decyl group, or a dodecyl group. $R^2$ and $R^3$ are the same or different from each other, and, preferably, each represent a methyl group, an ethyl group, or a propyl group. $R^4$ preferably represents a methyl group, an ethyl group, or a propyl group.
As the pyridinium salt surfactant, for example, a surfactant represented by the following formula (IV) is used.

[Chem. 4]

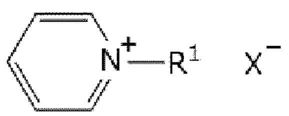

(IV)

In formula (IV), $R^1$ represents a C6 to C18 alkyl group or alkenyl group, and $X^-$ represents a halogen ion.
In formula (IV), $R^1$ preferably represents a C6, C8, C10, *C12,* or C14 alkyl group or alkenyl group, and particularly preferably represents a linear alkyl group. More specifically, $R^1$ represents an octyl group, a decyl group, or a dodecyl group.

(Hemolytic reagent)

In the analysis method of the present embodiment, a hemolytic reagent containing a solution of the cationic surfactant is preferably used. A solvent is not particularly limited as long as the cationic surfactant can be dissolved. Examples of the solvent include water, organic solvents, and mixtures thereof. As the organic solvent, a solvent that can be mixed with water is preferable, and examples thereof include C1 to C6 alcohols, ethylene glycol, diethylene glycol, polyethylene glycol, and DMSO. A concentration of the cationic surfactant in the hemolytic reagent can be determined according to a kind of the cationic surfactant as appropriate, and is, for example, 10 ppm or more. The concentration of the cationic surfactant is preferably 400 ppm or more, more preferably 500 ppm or more, and even more preferably 600 ppm or more. The concentration of the cationic surfactant in the hemolytic reagent is 10000 ppm or less. The concentration of the cationic surfactant in the hemolytic reagent is preferably 1000 ppm or less, more preferably 800 ppm or less, and even more preferably 700 ppm or less.
The sample and the hemolytic reagent preferably contain nonionic surfactants in addition to the above-described cationic surfactants. In a case where the cationic surfactant and a nonionic surfactant are contained, a nucleated cell can be inhibited from being excessively damaged by the cationic surfactant. The nonionic surfactant is, for example, a nonionic

surfactant represented by the following formula (V).

$$R^1\text{-}R^2\text{-}(CH_2CH_2O)_n\text{-}H \qquad (V)$$

(in formula (V), $R^1$ represents an alkyl group, alkenyl group, or alkynyl group in which the number of carbon atoms is 8 or more and 25 or less, and $R^2$ represents an oxygen atom or - (COO)-, or is represented by the following formula (VI):

[Chem. 5]

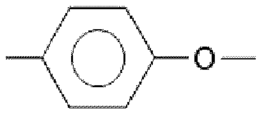

(VI)

n represents a numerical value that is 23 or more and 25 or less, or 30.)

In formula (V), n preferably represents 23 or 25 and more preferably represents 23. In a case where n is 23 or more and 25 or less, a concentration of the nonionic surfactant represented by formula (V) in the hemolytic reagent is 1700 ppm or more and preferably 1750 ppm or more. In a case where n is 23 or more and 25 or less, the concentration of the nonionic surfactant represented by formula (V) in the sample is 2300 ppm or less and preferably 2200 ppm or less.

In a case where n is 30, the concentration of the nonionic surfactant represented by formula (V) in the hemolytic reagent is 1900 ppm or more, preferably 2000 ppm or more, and even more preferably 2100 ppm or more. In a case where n is 30, the concentration of the nonionic surfactant represented by formula (V) in the sample is 2300 ppm or less and preferably 2200 ppm.

Specific examples of the nonionic surfactant represented by formula (V) include polyoxyethylene alkyl ether, polyoxyethylene sterol, polyoxyethylene castor oil, polyoxyethylene sorbitol fatty acid ester, polyoxyethylene alkylamine, polyoxyethylene polyoxypropylene alkyl ether, and combinations thereof. Among them, polyoxyethylene alkyl ether is preferable. Examples of the polyoxyethylene alkyl ether include polyoxyethylene (23) cetyl ether, polyoxyethylene (25) cetyl ether, and polyoxyethylene (30) cetyl ether. Polyoxyethylene (23) cetyl ether, polyoxyethylene (25) cetyl ether, and a combination thereof are more preferable, and polyoxyethylene (23) cetyl ether is even more preferable. One kind of the nonionic surfactant may be contained in the sample and the hemolytic reagent, or two or more kinds thereof may be contained therein. The hemolytic reagent may further contain a nonionic surfactant other than the nonionic surfactants represented by formula (V).

The sample and the hemolytic reagent may contain buffer substances for making a pH constant. Examples of the buffer substance include inorganic acid salts, organic acid salts, Good's buffers, and combinations thereof. Examples of the inorganic acid salt include phosphates, borates, and combinations thereof. Examples of the organic acid salt include citrates, malates, and combinations thereof. Examples of Good's buffers include MES, Bis-Tris, ADA, PIPES, Bis-Tris-Propane, ACES, MOPS, MOPSO, BES, TES, HEPES, HEPPS, Tricine, Tris, Bicine, TAPS, and combinations thereof.

The sample and the hemolytic reagent may further contain aromatic organic acids. In the present specification, the "aromatic organic acid" refers to an acid having at least one aromatic ring in the molecule, and a salt thereof. Examples of the aromatic organic acid include aromatic carboxylic acid and aromatic sulfonic acid. Examples of the aromatic carboxylic acid include phthalic acid, benzoic acid, salicylic acid, hippuric acid, salts thereof, and combinations thereof. Examples of the aromatic sulfonic acid include p-aminobenzenesulfonic acid, benzenesulfonic acid, salts thereof, and combinations thereof. One kind of the aromatic organic acid may be contained in the sample and the hemolytic reagent, or two or more kinds thereof may be contained therein. The aromatic organic acid may exhibit a buffering effect. In a case where the aromatic organic acid exhibiting a buffering effect is used, a buffer is discretionarily added, and the aromatic organic acid and the above-described buffer may be used in combination.

A concentration of the aromatic organic acid in the hemolytic reagent is 20 mM or higher and preferably 25 mM or higher. The concentration of the aromatic organic acid in the hemolytic reagent is 50 mM or less and preferably 45 mM or less.

The pH of the hemolytic reagent is, but is not particularly limited to, for example, 5.5 or higher. The pH of the hemolytic reagent is preferably 5.7 or higher and more preferably 5.9 or higher. The pH of the hemolytic reagent is, for example, 7.2 or less, preferably 6.9 or less, and more preferably 6.6 or less. A known base (sodium hydroxide or the like) or acid (hydrochloric acid or the like) can be used for adjusting the pH.

An osmotic pressure of the hemolytic reagent is, but is not particularly limited to, preferably 150 mOsm/kg or less, more preferably 130 mOsm/kg or less, and most preferably 110 mOsm/kg or less from the viewpoint of erythrocyte hemolyzing efficiency. An appropriate osmotic pressure adjusting agent may be added for adjusting the osmotic pressure. Examples of the osmotic pressure adjusting agent include sugars, amino acids, organic solvents, sodium chloride, and combinations thereof.

As the hemolytic reagent, a commercially available hemolytic reagent containing the cationic surfactant may be used. Examples of the hemolytic reagent include Lysercell WDF (SYSMEX CORPORATION) and Lysercell WDFII (SYSMEX CORPORATION).

(Sample)

The sample can be prepared by mixing the bone marrow aspirate, and the staining reagent containing the fluorescent dye. Preferably, the sample is prepared by mixing the bone marrow aspirate, the staining reagent containing the fluorescent dye, and the hemolytic reagent containing the cationic surfactant. In a case where the hemolytic reagent is used, a nucleated cell in the bone marrow aspirate is in a state of being stainable with the fluorescent dye by the effect of the cationic surfactant. The state of being stainable with the fluorescent dye refers to a state where a cell membrane of a cell has been damaged to such a degree that the fluorescent dye can permeate the cell. In a case where erythrocytes are mixed in the bone marrow aspirate, the erythrocytes are lysed by the effect of the cationic surfactant. The cell membranes of erythroblast cells are also damaged by the effect of the cationic surfactant, similarly to the erythrocytes. However, the cell nucleus of the erythroblast cell is maintained. Therefore, the erythroblast cell is in a state of being stainable with the fluorescent dye.

Through the damaged cell membrane of a nucleated cell, the fluorescent dye enters the cell and can stain nucleic acid in the cell nucleus. The stained nucleated cell can emit fluorescence, and fluorescence signal information can be obtained by FCM measurement. As described above, immature granulocytes in an early differentiation stage, erythroblasts in an early differentiation stage, blast cells, megakaryocytic cells, and plasmacytes each contain a large amount of nucleic acid, and are thus strongly stained, and the emitted fluorescence has a high intensity. As compared with these nucleated cells, immature granulocytes in a late differentiation stage, erythroblasts in a late differentiation stage, mature leukocytes, and mature megakaryocytes are weakly stained. Cells and particles such as erythrocytes, erythrocyte ghost, and lipid particles which have no nucleuses, are hardly stained.

In a case where one reagent containing the fluorescent dye and the cationic surfactant is used, a mixing ratio between the bone marrow aspirate and the reagent is generally 1:5 to 500 and preferably 1:10 to 100 by volume ratio. In a case where the staining reagent containing the fluorescent dye and the hemolytic reagent containing the cationic surfactant are used, the mixing ratio among the bone marrow aspirate, the staining reagent, and the hemolytic reagent is generally 1:1 to 10:5 to 500 and preferably 1:1 to 5:10 to 100 by volume ratio. After the bone marrow aspirate and each reagent are mixed, the mixture is preferably incubated under a predetermined condition. The predetermined condition is, for example, a condition that an incubation temperature is 15 to 50°C and preferably 30 to 45°C and an incubation time is 5 to 120 seconds and preferably 5 to 30 seconds. The sample may be prepared by hand or may be prepared by an analyzer having a sample preparation part.

(FCM measurement)

In the FCM measurement, light is applied to the sample, and optical information including fluorescence signal information is obtained for a particle in the sample. Specifically, firstly, the sample is introduced to a flow cell of the FCM, and, when particles in the sample pass through the flow cell one by one, light is applied to each particle. Light emitted from the individual particle is measured, and the optical information is obtained. The light emitted from the particle is fluorescence derived from the fluorescent dye, and scattered light. The scattered light includes, for example, forward scattered light (for example, scattered light for which a light reception angle is 0° to about 20°) and side scattered light (for example, scattered light for which a light reception angle is about 20° to about 90°) As the optical information, fluorescence signal information and scattered light information are preferably obtained. As the scattered light information, at least one of side scattered light information and forward scattered light information is preferably obtained, and both the side scattered light information and the forward scattered light information are particularly preferably obtained.

The fluorescence signal information and the scattered light information include peak values (heights of pulse peaks), pulse areas, pulse widths, transmittances, Stokes shifts, ratios, change with elapse of time, and values correlating therewith, about a fluorescence signal and a scattered light signal. The fluorescence signal information is not particularly limited as long as the fluorescence signal information represents an amount of the fluorescent dye with which nucleic acid in a nucleated cell is stained. Fluorescence information preferably represents a peak value (hereinafter, also referred to as "fluorescence signal intensity") of a fluorescence signal. The side scattered light information is not particularly limited as long as the side scattered light information represents internal information such as complexity of a cellular structure, granular characteristics, a nuclear structure, and lobularity. The forward scattered light information is not particularly limited as long as the forward scattered light information represents the size of a cell. The side scattered light information preferably represents a peak value (hereinafter, also referred to as "side scattered light intensity") of a side scattered light signal. The forward scattered light information preferably represents a peak value (hereinafter, also referred to as "forward scattered light intensity") of a forward scattered light signal.

A scattergram may be generated based on the optical information obtained by FCM measurement of the bone marrow aspirate. In the scattergram, for example, the individual measured particles are indicated as dots on a two-dimensional plane in which the horizontal axis represents the side scattered light information and the vertical axis represents the fluorescence signal information.

The FCM is not particularly limited, and a commercially available automated blood cell analyzer may be used. Examples of such an analyzer include XR series analyzers manufactured by SYSMEX CORPORATION. A light source of the FCM is not particularly limited, and a light source for which a wavelength is appropriate for excitation of the fluorescent dye, can be selected as appropriate. As the light source, for example, a blue semiconductor laser, a red semiconductor laser, an argon laser, a He-Ne laser, a mercury arc lamp, or the like is used.

In the analysis method of the present embodiment, nucleated cells are preferably counted. In the present specification, the "the number of nucleated cells" refers to the total of the numbers of leukocytes, erythroblast cells, megakaryocytic cells, mature megakaryocytes, and plasmacytes. A method for counting the nucleated cells is a known method, and the number of nucleated cells can be obtained based on the optical information obtained by FCM measurement of the sample. For example, the nucleated cells can be counted by excluding cells and particles such as erythrocytes, erythrocyte ghost, and lipid particles which have no nucleuses, from particles in the sample. Cells and particles having no nucleuses have very low fluorescence signal intensities, and can thus be excluded based on the fluorescence signal intensities. The nucleated cells can be counted by using analysis software mounted to the FCM. Such analysis software is known, and is, for example, Flowjo (Trademark) (available from BD Biosciences).

(First embodiment)

In the analysis method of the present embodiment, particles for which the fluorescence signal information indicates a threshold value or more are counted as target cells. A screening index for a hematopoietic tumor is obtained based on the number of the target cells. An embodiment in which the target cells are counted and the screening index for a hematopoietic tumor is obtained based on the number of the target cells is also referred to as "first embodiment". In the present specification, the "target cells" refer to immature granulocytes in an early differentiation stage, erythroblasts in an early differentiation stage, blast cells, megakaryocytic cells, and plasmacytes. As described above, an amount of nucleic acid in these nucleated cells is greater than an amount of nucleic acid in the other nucleated cells. The threshold value corresponding to the fluorescence signal information is a value that allows the above-described target cells to be distinguished from the other particles. A relationship between the target cells and the threshold value corresponding to the fluorescence signal information will be described below with reference to FIG. 1.

FIG. 1 illustrates an example of a scattergram based on the fluorescence signal information and the scattered light information obtained by FCM measurement of bone marrow aspirate. In FIG. 1, in the scattergram, the horizontal axis represents side scattered light intensities and the vertical axis represents fluorescence signal intensities. In FIG. 1, mature leukocytes are classified into five kinds of subpopulations of a lymphocyte population, a monocyte population, a neutrophil population, an eosinophil population, and a basophil population. However, the present disclosure is not limited thereto. The mature leukocytes may be classified into three kinds of subpopulations of a lymphocyte population, a monocyte population, and a mature granulocyte population, or classified into four kinds of subpopulations of a lymphocyte population, a monocyte population, a neutrophil-and-basophil population, and an eosinophil population. Classification of the cell populations in the scattergram can be performed by analysis software mounted to the FCM. In FIG. 1, a straight line (hereinafter, also referred to as "transverse line") extending across the two-dimensional plane of the scattergram indicates a threshold value corresponding to the fluorescence signal intensity. Immature granulocytes in an early differentiation stage, erythroblasts in an early differentiation stage, blast cells, megakaryocytic cells, and plasmacytes which are target cells each contain a large amount of nucleic acid, and thus indicate high fluorescence signal intensities. Therefore, these cells are distributed so as to form the corresponding populations in a region in which the fluorescence signal intensity is high in the scattergram. The blast cell population and the population of erythroblasts in an early differentiation stage appear such that the regions of both populations overlap each other. Meanwhile, erythroblasts in a late differentiation stage, immature granulocytes in a late differentiation stage, lymphocytes, monocytes, neutrophils, eosinophils, basophils, and immature eosinophils each contain a relatively small amount of nucleic acid, and thus indicate low fluorescence signal intensities. Therefore, these cells are distributed so as to form the corresponding populations in a region in which the fluorescence signal intensity is low in the scattergram. The populations of lymphocytes and monocytes, and the population of erythroblasts in a late differentiation stage appear such that the regions thereof overlap each other. By dividing the two-dimensional plane of the scattergram by the threshold value corresponding to the fluorescence signal intensity like the transverse line in FIG. 1, a cell group of the target cells and a cell group of non-target cells can be divided. The threshold value corresponding to the fluorescence signal information may be a fixed value or may be set so as to be variable for each specimen.

As the threshold value corresponding to the fluorescence signal information, for example, a value that allows 95% or more of mature leukocytes in the bone marrow aspirate to be excluded can be defined. An example of such

threshold value is represented as the transverse line on the scattergram illustrated in FIG. 1. In FIG. 1, for the sake of description, the population of immature granulocytes in an early differentiation stage and the population of immature granulocytes in a late differentiation stage are separately indicated. In an actual scattergram of bone marrow aspirate, these immature granulocyte populations may continuously appear, and may not be clearly separated from each other. The same applies to the population of erythroblasts in an early differentiation stage and the population of erythroblasts in a late differentiation stage. Therefore, the threshold value corresponding to the fluorescence signal information is preferably a fixed value. The FCM performs A/D conversion of analog signals of fluorescence and scattered light at predetermined sampling rates, and generates digital signals. At this time, in a case where the digital signal is expressed by 8 bit data, the fluorescence signal intensity and the side scattered light intensity are each expressed by 256 gradations from 0 to 255 channels. The channel is a unit of an intensity of a digital signal. In a case where the fluorescence intensity is expressed by 256 gradations from 0 to 255 channels, for example, the fluorescence signal intensity of 100 ch can be set as the threshold value that allows 95% or more of mature leukocytes in the bone marrow aspirate to be excluded. The "ch" represents channel. That is, the fluorescence signal intensity of 100 ch can be set as the threshold value corresponding to the fluorescence signal information.

Alternatively, as shown in FIG. 1, for example, the threshold value corresponding to the fluorescence signal information may be defined as a value represented by a transverse line that intersects the monocyte population but does not intersect the lymphocyte population in the sample when distribution of particles in the sample is rendered on the two-dimensional plane based on the fluorescence signal information and the side scattered light information. In this case, the threshold value corresponding to the fluorescence signal information is, for example, a value that satisfies at least the following two conditions:

(1) The value is greater than the maximum value of the fluorescence signal intensity of the lymphocyte population; and
(2) The value is less than the maximum value of the fluorescence signal intensity of the monocyte population.

More preferably, the threshold value corresponding to the fluorescence signal information is a value that also satisfies a condition (3) that the value is greater than the statistical representative value of the monocyte population. In this case, the statistical representative value is, for example, an average value, a median, or a mode. The maximum value in the above-described condition may be a representative value of a partial population in which the fluorescence signal intensity is high, in each cell population, instead of the above-described maximum value. For example, the average value, the median, or the mode of the top 5% of the fluorescence signal intensities in each cell population may be used.

Alternatively, the threshold value corresponding to the fluorescence signal information may be, for example, defined by a ratio of a population Y of nucleated cells in which the fluorescence signal information indicates a certain value or more, to a parent population X of nucleated cells in peripheral blood of a healthy individual. Specifically, the certain value of the fluorescence signal information which allows the ratio to be in a predetermined range, for example, a range of 0.1% or more and 10% or less, more preferably a range of 1% or more and 9% or less, and even more preferably a range of 2.5% or more and 5% or less, may be defined as the threshold value corresponding to the fluorescence signal information. In this definition, the threshold value corresponding to the fluorescence signal information may be determined as follows based on a result of FCM measurement of peripheral blood of a healthy individual (hereinafter, also referred to as "normal blood"). Firstly, a control sample containing the normal blood and the above-described fluorescent dye is measured by the FCM in the same manner as for bone marrow aspirate, and the fluorescence signal information and the side scattered light information are obtained. The normal blood can be obtained and measured by the FCM more easily than bone marrow aspirate, and is thus appropriate as the control sample. The number of the control samples is preferably plural. For example, the number of the control samples to be measured by the FCM is at least 20, and is preferably 20 or more and 40 or less. The plurality of control samples are prepared from a plurality of normal bloods (for example, 20 specimens), respectively. Preferably, the control sample further contains the above-described cationic surfactant. More preferably, the control sample is prepared by mixing peripheral blood of a healthy individual, the above-described staining reagent, and the above-described hemolytic reagent. The fluorescence signal information is preferably a fluorescence signal intensity. The side scattered light information is preferably a scattered light intensity. Subsequently, nucleated cells are sorted based on such optical information, and the number of the nucleated cells is obtained. Furthermore, a scattergram may be generated based on the optical information, and the mature leukocytes may be classified into three to five kinds of subpopulations as described above. Then, a certain fluorescence signal intensity is set as a provisional threshold value. The provisional threshold value can be, for example, selected from values higher than the fluorescence signal intensity of the monocyte population. The fluorescence signal intensity of the cell population on the scattergram can be a statistical representative value of the fluorescence signal intensities of the cells included in the population. Examples of the statistical representative value include an average value, a median, a mode, and quartile points. Such a statistical representative value can be obtained by analysis software mounted to the FCM. After the provisional threshold value is set, cells each having a fluorescence signal intensity that indicates the provisional threshold value or more, are counted for each of the plurality of the measurement samples. A ratio of the cells each having the fluorescence signal intensity that indicates the

provisional threshold value or more, relative to the number of nucleated cells, is calculated. The median of the ratios of the plurality of measurement samples is obtained. The median can be obtained by, for example, known spreadsheet software such as Excel (Registered Trademark). When the median is, for example, 2.5% or more and 5% or less, the provisional threshold value can be set as the threshold value corresponding to the fluorescence signal information in FCM measurement of bone marrow aspirate. Alternatively, after the provisional threshold value is set, the threshold value may be set as follows. For each of the plurality of measurement samples, the first quartile point and the third quartile point of the fluorescence signal intensities of the monocyte population are obtained, and the average of the first quartile points and the average of the third quartile points in measurement of the plurality of specimens are obtained, and an interquartile range is calculated, and an interquartile range (section from the first quartile point to the third quartile point: IQR) including the median is obtained. Any provisional threshold value included in the averaged interquartile range thus set for the monocyte population may be set as a predetermined threshold value corresponding to the fluorescence signal information in FCM measurement of bone marrow aspirate.

The target cells are counted in such a manner that particles for which the fluorescence signal information indicates the threshold value or more are extracted from all the particles in the sample measured by the FCM, and the number thereof is counted. In a case where a scattergram in which the horizontal axis represents the side scattered light information and the vertical axis represents the fluorescence signal information is generated, particles included in a region (hereinafter, also referred to as "first region") in which the fluorescence signal information indicates the threshold value or more are specified as target cells, and the number of the target cells may be counted. For example, in the scattergram in FIG. 1, the first region is a region on the upper side of the two-dimensional plane that is divided by the transverse line that indicates the threshold value corresponding to the fluorescence signal information. The target cells can be counted by, for example, analysis software mounted to the FCM.

Alternatively, the threshold value corresponding to the fluorescence signal information may be determined by specifying the first region by the following process step. Specifically, the lowest fluorescence signal intensity in the first region can be set as the threshold value corresponding to the fluorescence signal information. Each of steps of specifying the first region will be described. Firstly, a control sample containing normal blood and the above-described fluorescent dye is measured by the FCM in the same manner as for bone marrow aspirate, and the fluorescence signal information and the side scattered light information are obtained. The number of the control samples may be one or plural. A plurality of control samples are preferably measured. The fluorescence signal information is preferably a fluorescence signal intensity. The side scattered light information is preferably a scattered light intensity. Subsequently, a scattergram is generated based on the fluorescence signal information and the side scattered light information having been obtained. In the generated scattergram, a region in which 95% or more of mature leukocytes are excluded, and the fluorescence signal intensity is higher than or equal to a fluorescence signal intensity of the monocyte population is specified as the first region. The first region having been thus specified is, for example, a region surrounded by dashed lines in FIG. 2. As shown in FIG. 2, a part of monocytes is allowed to appear in the first region as long as 95% or more of mature leukocytes are excluded from the first region. In a case where the first region specified based on the FCM measurement of the normal blood is applied to the scattergram generated based on FCM measurement of bone marrow aspirate, the first region can include or overlap a region in which the above-described target cells appear. The lowest fluorescence signal intensity indicated by an arrow in FIG. 2 in the first region can be set as the threshold value corresponding to the fluorescence signal information in the FCM measurement of bone marrow aspirate.

In the present specification, the "peripheral blood of a healthy individual" represents peripheral blood which is collected from a healthy individual and has EDTA-2K added thereto and in which the numbers of various blood cells and the hemoglobin concentration satisfy the following criteria.

- ·The number of leukocytes is 2500/μL or more and 18000/μL or less.
- ·An amount of hemoglobin is 10 g/dL or more
- ·The number of platelets is 60000/μL or more and 600000/μL or less.
- ·The number of neutrophils is 1000/μL or more and 11000/μL or less.
- ·The number of lymphocytes is 800/μL or more and 4000/μL or less.
- ·The number of monocytes is 1000/μL or less.
- ·The number of eosinophils is 700/μL or less.
- ·The number of basophils is 200/μL or less.

In the first embodiment, a screening index for a hematopoietic tumor is obtained based on the number of target cells. The hematopoietic tumor is not particularly limited, and is, for example, acute leukemia, plasmacytoma, or mature lymphoma. Among them, acute leukemia rapidly progresses and is a lethal tumor, and is thus particularly significant. The screening index for a hematopoietic tumor represents, for example, a value for indicating whether or not there is onset of a hematopoietic tumor in a subject, indicating whether or not a subject is suspected of having a hematopoietic tumor, or indicating whether or not there is a symptom of a hematopoietic tumor. The number of the target cells may be used, as it is,

as the screening index for a hematopoietic tumor. A ratio of the number of the target cells to the number of nucleated cells is preferably obtained as the screening index for a hematopoietic tumor. Hereinafter, the ratio is also referred to as "first ratio". The number of nucleated cells represents the number that is counted based on the optical information obtained by FCM measurement of the sample, as described above. The first ratio is calculated according to the following Equation (A).

$$\text{(First ratio)}=[(\text{the number of target cells})/(\text{the number of nucleated cells})]\times 100 \quad \cdots(A)$$

The first ratio also includes values based on the value calculated according to Equation (A) as long as tendency of change in the number of the target cells is indicated based on the number of nucleated cells in the sample. Examples of the values based on the value calculated according to Equation (A) include values obtained by using any coefficient and/or constant in Equation (A). A percentage may be indicated as a real number by dividing the value calculated according to Equation (A) by 100. Any constant may be added to or subtracted from the value calculated according to Equation (A).

In the first embodiment, whether or not the bone marrow aspirate is abnormal may be determined according to comparison between the first ratio and a threshold value. In a case where the first ratio is the value calculated according to Equation (A), the threshold value for the first ratio can be, for example, any value between 20 and 40%, preferably any value between 25 and 35%, and more preferably 30%. For example, when the first ratio indicates the threshold value or more in comparison between the first ratio and the threshold value, the bone marrow aspirate is determined to be abnormal. In this case, it is considered that the number of blast cells or plasmacytes is increased in the bone marrow aspirate of a subject as compared with normal bone marrow aspirate. This means that there is onset or suspicion of acute leukemia or plasmacytoma. Meanwhile, when the first ratio is less than the threshold value, it is determined that the bone marrow aspirate is not abnormal. This indicates that the subject is not suspected of having a hematopoietic tumor.

In the first embodiment, in a case where the first ratio indicates the threshold value or more, distinguishable display may be provided. The distinguishable display is preferably made on a screen of a display device that is mounted to the FCM, or connected to the FCM so as to make communication therebetween. Examples of the display device include displays such as liquid crystal displays, plasma displays, and CRT displays. On the screen of the display device, for example, the first ratio itself is displayed as the distinguishable display, and/or distinguishable display indicating that the ratio indicates the threshold value or more is made, in a form that can distinguish characters and numerical values of the distinguishable display from the other characters and numerical values. For example, on the screen of the display device, the value of the first ratio is indicated by a size or color different from those of the other values, or the first ratio is highlighted by an underline, a flag, or the like. Alternatively, for example, a message indicating that the first ratio indicates the threshold value or more is displayed as a pop-up message on the screen of the display device or displayed in a remark area. In a case where the first ratio is less than the threshold value, the first ratio may be displayed in a normal manner. For example, the value of the first ratio may be displayed in the same manner as for the other values on the screen of the display device.

Such distinguishable display has the significance and effect that information for supporting determination as to whether or not the bone marrow aspirate is abnormal can be provided to medical professionals such as a doctor and an examiner who view the display. For example, the medical professional who views the distinguishable display can know that the bone marrow aspirate is a specimen suspected of acute leukemia or plasmacytoma. Furthermore, as necessary, an additional test for the bone marrow aspirate can be performed, or procedures for treatment and hospitalization of the subject or introduction to a special hospital can be started.

In a case where the first ratio indicates the threshold value or more, the distinguishable display may indicate that the bone marrow aspirate is a specimen that needs to be preferentially tested for a hematopoietic tumor. Examples of the test include myelogram, a genetic test, and an antibody panel test. Among them, myelogram is particularly preferable. As described above, in a case where the first ratio indicates the threshold value or more, the subject is suspected of having acute leukemia or plasmacytoma. Such distinguishable display has the significance and effect that, for example, a doctor or an examiner can perform arrangement so as to test the bone marrow aspirate more preferentially than the other specimens.

(Second embodiment)

In acute leukemia, it is known that cancerated blast cells abnormally proliferate. Furthermore, in plasmacytoma, increase in the number of plasmacytes in bone marrow aspirate is found. Each of a blast cell and plasmacyte is a mononuclear cell. In another embodiment, the screening index for a hematopoietic tumor may be obtained based on the number of mononuclear cells among the target cells. This embodiment is also referred to as "second embodiment". Hereinafter, the target cell that is a mononuclear cell is also referred to as "target mononuclear cell". As described above, the target cell is a particle for which the fluorescence signal information indicates a threshold value or more. Therefore, in the second embodiment, in the target cell counting step, mononuclear cells for which the fluorescence signal information

indicates the threshold value or more are counted as the target mononuclear cells. In the present embodiment, the mononuclear cells include erythroblasts in an early differentiation stage, blast cells, plasmacytes, monocytes, lymphocytes, and erythroblasts in a late differentiation stage among nucleated cells. Therefore, the target mononuclear cells are erythroblasts in an early differentiation stage, blast cells, and plasmacytes. Among the nucleated cells, immature granulocytes in an early differentiation stage, megakaryocytic cells, mature megakaryocytes, immature granulocytes in a late differentiation stage, mature granulocytes, immature eosinophils, and immature basophils are polymorphonuclear cells.

In the second embodiment, the target mononuclear cells may be counted based on the fluorescence signal information and the side scattered light information of particles in the sample. Alternatively, after the target cells are counted, the target mononuclear cells may be counted based on the side scattered light information of the target cells.

In the FCM measurement, it is known that side scattered light represents internal information of a nucleus, a granule, and the like in a cell. The mononuclear cell tends to indicate a lower side scattered light intensity than the polymorphonuclear cell. Therefore, the mononuclear cell population and the polymorphonuclear cell population can be divided by an auxiliary line based on the side scattered light information in a scattergram generated based on the fluorescence signal information and the side scattered light information. This will be described with reference to FIG. 3A. In FIG. 3A, as indicated by an oblique line, the auxiliary line based on the side scattered light intensity is drawn in the scattergram shown in FIG. 1. In FIG. 3, the transverse line indicates the threshold value corresponding to the fluorescence signal intensity as in FIG. 1. As shown in FIG. 3A, in a region above the transverse line, the oblique line divides the populations of blast cells and erythroblasts in an early differentiation stage, and the population of immature granulocytes in an early differentiation stage. In a region below the transverse line, the oblique line divides the populations of monocytes and erythroblasts in a late differentiation stage, and the populations of immature granulocytes in a late differentiation stage, neutrophils, and basophils.

As shown in FIG. 3A, the two-dimensional plane of the scattergram is divided into four regions by the oblique line that divides the nucleated cells in the bone marrow aspirate into mononuclear cells and polymorphonuclear cells, and the transverse line which indicates the threshold value corresponding to the fluorescence signal intensity and allows 95% or more of mature leukocytes in the bone marrow aspirate to be excluded. In the second embodiment, particles appearing in a region (region surrounded by dashed lines) above the transverse line and leftward of the oblique line in the scattergram in FIG. 3A are counted. In this region, the mononuclear cells for which the fluorescence signal information indicates the threshold value or more, that is, blast cells, erythroblasts in an early differentiation stage, and plasmacytes are distributed to form the populations, respectively. In the second embodiment, particles included in a region (hereinafter, also referred to as "second region") in which blast cells, erythroblasts in an early differentiation stage, and plasmacytes appear can be specified as the target mononuclear cells based on the fluorescence signal information and the side scattered light information of the particles in the sample. The target mononuclear cells can be counted by, for example, analysis software mounted to the FCM.

In FIG. 3A, for the sake of description, the populations of erythroblasts in an early differentiation stage and blast cells, and the population of immature granulocytes in an early differentiation stage are separately indicated. In an actual scattergram of bone marrow aspirate, these populations may continuously appear and may not be clearly separated from each other. The same applies to the populations of monocytes and erythroblasts in a late differentiation stage, and the populations of immature granulocytes in a late differentiation stage, neutrophils, and basophils. The second region may be a preset region, or may be set so as to be variable for each specimen. For example, the second region may be empirically set by accumulating data of the scattergrams based on the fluorescence signal information and the side scattered light information that are obtained by FCM measurement of normal bone marrow aspirates and bone marrow aspirates of hematopoietic tumors such as acute leukemia. Alternatively, the region may be set according to predetermined coordinates of the scattergram based on the fluorescence signal intensity and the side scattered light intensity. As described above, the FCM performs A/D conversion of analog signals of fluorescence and scattered light at predetermined sampling rates, and generates digital signals. At this time, in a case where the digital signal is expressed by 8 bit data, the ranges of the fluorescence signal intensity and the side scattered light intensity are each a range from 0 to 255 ch in the scattergram. The predetermined coordinates may be the following four coordinates (X:Y)=(0 ch:100 ch), (135 ch:100 ch), (0 ch:255 ch), and (180 ch:255 ch) in the scattergram in which the X-axis represents side scattered light intensities and the Y-axis represents fluorescence signal intensities. The region surrounded by these four coordinates can be set as the second region.

Alternatively, the four coordinates of the second region may be set based on the analysis results of a plurality of control samples each containing normal blood and the above-described fluorescent dye. The number of the control samples is at least 20, and is preferably 20 or more and 40 or less. For example, in FIG. 3B, a region surrounded by a point A, a point B, a point C, and a point D is set as the second region. FIG. 3B shows a scattergram of normal blood in which the X-axis represents side scattered light intensities and the Y-axis represents fluorescence signal intensities. In FIG. 3B, the straight line extending across the two-dimensional plane of the scattergram is a straight line that indicates the threshold value corresponding to the fluorescence signal intensity. As described for the first embodiment, the straight line can be set

based on the analysis result of the control samples. An X coordinate of the point A can be, for example, determined as any value between an average value of medians of the monocyte populations of the respective specimens at the X-axis, and an average value of medians of the neutrophil populations at the X-axis. The point A is a point on the straight line that indicates the predetermined threshold value corresponding to the fluorescence signal intensity, and thus, a Y coordinate of the point A is equal to the predetermined threshold value corresponding to the fluorescence signal intensity. An X coordinate of the point B can be, for example, determined as any value between an average value of medians of the neutrophil populations of the respective specimens at the X-axis, and an average value of medians of the eosinophil populations at the X-axis. A Y coordinate of the point B is the maximum value of the fluorescence signal intensity in the scattergram as shown in FIG. 3B. For the point C, as shown in FIG. 3B, the X coordinate is 0, and the Y coordinate is the maximum value of the fluorescence signal intensity in the scattergram. The point D is a point on the straight line that indicates the predetermined threshold value corresponding to the fluorescence signal intensity, similarly to the point A. Therefore, for the point D, as shown in FIG. 3B, the X coordinate is 0, and the Y coordinate is equal to the predetermined threshold value corresponding to the fluorescence signal intensity. In a case where the second region specified by the four coordinates thus determined is applied to the scattergram generated based on FCM measurement of the bone marrow aspirate, the second region can include or overlap a region in which the above-described target mononuclear cells appear.

Alternatively, the second region may be set based on the analysis results of the control samples each containing normal blood and the above-described fluorescent dye, similarly to the first region. Firstly, as described for the first embodiment, the control samples are measured by the FCM, and the fluorescence signal information and the side scattered light information are obtained. The details of the control sample are as described above. The fluorescence signal information is preferably a fluorescence signal intensity. The side scattered light information is preferably a scattered light intensity. Subsequently, a scattergram is generated based on the fluorescence signal information and the side scattered light information having been obtained. In the generated scattergram, a region in which 95% or more of mature leukocytes are excluded, the fluorescence signal intensity is higher than or equal to the fluorescence signal intensity of the monocyte population, and the side scattered light intensity is less than or equal to the side scattered light intensity of the neutrophil population, is specified as the second region. The fluorescence signal intensity and the side scattered light intensity of the cell population on the scattergram can be statistical representative values of the fluorescence signal intensities and the side scattered light intensities of the cells included in the population. The statistical representative value is as described above. In a case where the second region specified based on FCM measurement of peripheral bloods of healthy individuals is applied to the scattergram generated based on FCM measurement of the bone marrow aspirate, the second region can include or overlap a region in which the above-described target mononuclear cells appear.

In the second embodiment, a screening index for a hematopoietic tumor is obtained based on the number of the target mononuclear cells. The hematopoietic tumor and the screening index therefor are as described above. The number of the target mononuclear cells may be used, as it is, as the screening index for a hematopoietic tumor. A ratio of the number of the target mononuclear cells to the number of nucleated cells is preferably obtained as the screening index for a hematopoietic tumor. Hereinafter, the ratio is also referred to as "second ratio". The number of nucleated cells represents the number that is counted based on the optical information obtained by FCM measurement of the sample as described above. The second ratio represents a value calculated according to the following Equation (B).

$$\text{(Second ratio)}=[\text{(the number of target mononuclear cells)}/\text{(the number of nucleated cells)}]\times 100 \qquad \text{(B)}$$

The second ratio also includes values based on the value calculated according to Equation (B) as long as tendency of change in the number of the target mononuclear cells is indicated based on the number of nucleated cells in the sample. Examples of the values based on the value calculated according to Equation (B) are similar to the examples of the values based on the value calculated according to Equation (A).

In the second embodiment, whether or not the bone marrow aspirate is abnormal may be determined according to comparison between the second ratio and a threshold value. In a case where the second ratio is the value calculated according to Equation (B), the threshold value for the second ratio can be, for example, any value between 10% and 30%, preferably any value between 15% and 25%, and more preferably 20%. For example, when the second ratio indicates the threshold value or more in comparison between the second ratio and the corresponding predetermined threshold value, the bone marrow aspirate is determined to be abnormal. In this case, it is considered that the number of blast cells or plasmacytes is increased in the bone marrow aspirate of the subject as compared with normal bone marrow aspirate. This means that there is onset or suspicion of acute leukemia or plasmacytoma. Meanwhile, when the second ratio is less than the threshold value, it is determined that the bone marrow aspirate is not abnormal.

In the second embodiment, in a case where the second ratio indicates the threshold value or more, distinguishable display may be provided. The distinguishable display is preferably made on a screen of a display device, similarly to the first ratio. On the screen of the display device, for example, the second ratio itself is displayed as the distinguishable display, and/or distinguishable display indicating that the ratio indicates the threshold value or more is made, in a form that

can distinguish characters and numerical values of the distinguishable display from the other characters and numerical values. The display device and a manner of display on the screen are the same as described for the first embodiment. In a case where the second ratio indicates the threshold value or more, the distinguishable display may indicate that the bone marrow aspirate is a specimen that needs to be preferentially tested for a hematopoietic tumor. The test is as described above. The significance and effect of the distinguishable display are also the same as described for the first embodiment.

(Third embodiment)

As described above, in acute leukemia, cancerated blast cells abnormally proliferate. In another embodiment, the screening index for a hematopoietic tumor (in particular, acute leukemia) may be obtained based on the number of blast cells among the target cells. This embodiment is also referred to as "third embodiment". In the third embodiment, blast cells are counted in the target cell counting step.

In the third embodiment, blast cells may be counted based on the optical information of particles in the sample. Alternatively, after the target cells are counted, blast cells may be counted based on the optical information of the target cells. Blast cells are preferably counted based on the optical information of particles in the sample. In the third embodiment, the fluorescence signal information, the side scattered light information, and the forward scattered light information are used as the optical information. For example, firstly, particles including blast cells are sorted based on the fluorescence signal information and the side scattered light information of particles in the sample. The particles including the blast cells may include erythroblasts in an early differentiation stage and plasmacytes in addition to the blast cells. The blast cells are sorted from the particles including the blast cells based on the forward scattered light information of the particles. This will be described below.

The above-described sorting of particles including blast cells will be described with reference to FIG. 4A. In FIG. 4A, a region (hereinafter, also referred to as "third region") in which particles including blast cells appear is indicated by dashed lines in the scattergram shown in FIG. 1. In FIG. 4A, the transverse line indicates the threshold value corresponding to the fluorescence signal intensity as in FIG. 1. In the third embodiment, particles appearing in the region surrounded by the dashed lines in the scattergram in FIG. 4A can be specified as particles including blast cells. In FIG. 4A, the region surrounded by the dashed lines includes a part of the region below the transverse line. This is because the blast cell population is likely to be distributed also in a region in which the monocyte population appears, in bone marrow aspirate in which blast cells proliferate as in a specimen of acute leukemia. Particles including blast cells can be sorted by, for example, analysis software mounted to the FCM.

The third region may be a preset region or may be set so as to be variable for each specimen. For example, the third region may be empirically set by accumulating data of the scattergrams based on the fluorescence signal information and the side scattered light information that are obtained by FCM measurement of normal bone marrow aspirates and bone marrow aspirates of hematopoietic tumors such as acute leukemia Alternatively, the third region may be determined based on the scattergram in which the fluorescence signal intensity and the side scattered light intensity are each expressed in a range from 0 to 255 channels. Specifically, in the scattergram, the third region can be set as a region surrounded by the following four coordinates of (X:Y)=(80 ch:100 ch), (130 ch:80 ch), (180 ch:255 ch), and (130 ch:255 ch). X represents a side scattered light intensity and Y represents a fluorescence signal intensity.

Alternatively, the four coordinates of the third region may be set based on the analysis results of a plurality of control samples each containing normal blood and the above-described fluorescent dye as follows. The number of the control samples is at least 20, and is preferably 20 or more and 40 or less. For example, in FIG. 4B, a region surrounded by the point B, a point E, a point F, and a point G is set as the third region. FIG. 4B shows a scattergram of normal blood in which the X-axis represents side scattered light intensities and the Y-axis represents fluorescence signal intensities. In FIG. 4B, the straight line extending across the two-dimensional plane of the scattergram is a straight line that indicates the threshold value corresponding to the fluorescence signal intensity. The straight line can be set based on the analysis results of the control samples as described for the first embodiment. The point B in FIG. 4B is the same as the point B in FIG. 3B, and the description in the second embodiment is applied to the coordinates of the point B. An X coordinate of the point E can be, for example, determined as any value between an average value of medians of the monocyte populations of the respective specimens at the X-axis, and an average value of medians of the neutrophil populations at the X-axis. A Y coordinate of the point E is the maximum value of the fluorescence signal intensity in the scattergram as shown in FIG. 4B. An X coordinate of the point F can be, for example, determined as any value between an average value of the medians of the lymphocyte populations of the respective specimens at the X-axis, and an average value of the medians of the monocyte populations at the X-axis. The point F is a point on the straight line that indicates the threshold value corresponding to the fluorescence signal intensity, and thus, a Y coordinate of the point F is equal to the threshold value corresponding to the fluorescence signal intensity. An X coordinate of the point G can be the same as the X coordinate of the point E. A Y coordinate of the point G can be, for example, determined as any value between 0 percentile of the fluorescence signal intensities of the monocyte populations of the respective specimens and the first quartile point (25 percentile). In a case where the third region specified by the four coordinates thus determined is applied to the scattergram generated based on FCM measurement of

the bone marrow aspirate, the third region can include or overlap a region in which the above-described blast cells appear.

Alternatively, the third region may be set based on the analysis results of the control samples each containing normal blood and the above-described fluorescent dye, similarly to the first region. Firstly, as described for the first embodiment, the control samples are measured by the FCM, and the fluorescence signal information and the side scattered light information are obtained. The details of the control sample are as described above. The fluorescence signal information is preferably a fluorescence signal intensity. The side scattered light information is preferably a scattered light intensity. Subsequently, a scattergram is generated based on the fluorescence signal information and the side scattered light information having been obtained. In the generated scattergram, a region in which 95% or more of mature leukocytes are excluded, the fluorescence signal intensity is higher than or equal to a fluorescence signal intensity of the monocyte population, and the side scattered light intensity is higher than or equal to the side scattered light intensity of the lymphocyte population and less than or equal to the side scattered light intensity of the neutrophil population, is specified as the third region. The fluorescence signal intensity and the side scattered light intensity of the cell population on the scattergram can be statistical representative values of the fluorescence signal intensities and the side scattered light intensities of the cells included in the population. The statistical representative value is as described above. In a case where the third region specified based on FCM measurement of peripheral bloods of healthy individuals is applied to the scattergram generated based on FCM measurement of the bone marrow aspirate, the third region can include or overlap a region in which particles including blast cells as described above appear.

As described above, particles including blast cells may include erythroblasts in an early differentiation stage and plasmacytes, in addition to the blast cells. Therefore, in the third embodiment, the blast cells are sorted from the particles including the blast cells based on the forward scattered light information of the particles. The forward scattered light is known to represent a size of a cell in FCM measurement. The size of a blast cell is known to be 10 to 20 $\mu$m. For example, a histogram of the forward scattered light intensities is generated for the particles, including blast cells, which are sorted based on the fluorescence signal information and the side scattered light information, and a population corresponding to the sizes of the blast cells can be sorted based on the forward scattered light intensity. In a case where the digital signal of the forward scattered light is expressed by 8 bit data by the FCM, the range of the forward scattered light intensities is 0 to 255 channels. In this case, in the histogram of the forward scattered light intensities, particles in the range of 50 ch or more and 100 ch or less may be sorted as the blast cells.

In the third embodiment, the screening index for a hematopoietic tumor is obtained based on the number of the sorted blast cells. The hematopoietic tumor and the screening index therefor are as described above. The number of the blast cells may be used, as it is, as the screening index for a hematopoietic tumor. Preferably, a ratio of the number of blast cells to the number of nucleated cells is obtained as the screening index for a hematopoietic tumor. Hereinafter, the ratio is also referred to as "third ratio". The number of nucleated cells represents the number that is counted based on the optical information obtained by FCM measurement of the sample as described above. The third ratio represents a value calculated according to the following Equation (C).

$$\text{(Third ratio)}=[\text{(the number of blast cells)}/\text{(the number of nucleated cells)}]\times 100 \quad \cdots\text{(C)}$$

The third ratio also includes values based on the value calculated according to Equation (C) as long as tendency of change in the number of blast cells is indicated based on the number of nucleated cells in the sample. Examples of the values based on the value calculated according to Equation (C) are similar to the examples of the values based on the value calculated according to Equation (A).

In the third embodiment, whether or not the bone marrow aspirate is abnormal may be determined according to comparison between the third ratio and a threshold value. In a case where the third ratio is a value calculated according to Equation (C), the threshold value for the third ratio can be, for example, any value between 5% and 25% and preferably 10%. For example, when the third ratio indicates the threshold value or more in comparison between the third ratio and the threshold value, the bone marrow aspirate is determined to be abnormal. When the third ratio is 10% or more, it is considered that the number of blast cells is increased in the bone marrow aspirate of the subject as compared with normal bone marrow aspirate. This means that there is a symptom of acute leukemia, or onset or suspicion of acute leukemia. Meanwhile, when the third ratio is less than the threshold value, it is determined that the bone marrow aspirate is not abnormal. This indicates that there is no symptom of acute leukemia and no suspicion of acute leukemia.

The threshold value for the third ratio may be, for example, 20%. When the third ratio is 20% or more, it is considered that the number of blast cells is significantly increased in the bone marrow aspirate of the subject. This means that there is onset or suspicion of acute leukemia. Meanwhile, when the third ratio is less than 20%, significant increase in the number of blast cells in the bone marrow aspirate is not confirmed, and this indicates that there is no suspicion of acute leukemia.

In a case where whether or not the bone marrow aspirate is abnormal is determined based on the third ratio, two

threshold values may be used. In a case where the third ratio is a value calculated according to Equation (C), for example, a first threshold value is set as 10%, and a second threshold value is set as 20%, in the determination. Specifically, firstly, the third ratio and the first threshold value are compared with each other. When the third ratio is less than the first threshold value, it is determined that the bone marrow aspirate is not abnormal. When the third ratio indicates the first threshold value or more, the third ratio and the second threshold value are compared with each other. When the third ratio is less than the second threshold value, it is determined that the bone marrow aspirate is abnormal, and there is suspicion of increase in the number of blast cells. When the third ratio indicates the second threshold value or more, it is determined that the bone marrow aspirate is abnormal, and there is suspicion of acute leukemia.

In the third embodiment, in a case where the third ratio indicates the threshold value or more, distinguishable display may be provided. Similarly to the first ratio, the distinguishable display is preferably made on a screen of a display device. On the screen of the display device, for example, the third ratio itself is displayed as the distinguishable display, and/or distinguishable display indicating that the ratio indicates the threshold value or more is made, in a form that can distinguish characters and numerical values of the distinguishable display from the other characters and numerical values. The display device and a manner for display on the screen are the same as described for the first embodiment. In a case where the third ratio indicates the threshold value or more, the distinguishable display may indicate that the bone marrow aspirate is a specimen that needs to be preferentially tested for a hematopoietic tumor, in particular, acute leukemia. The test is as described above. In the third embodiment, in particular, blast cells are detected. Therefore, in a case where the third ratio indicates the threshold value or more, for example, a remark, such as "Blastosis?", indicating increase in the number of blast cells may be displayed on the screen of the display device. The significance and effect of the distinguishable display are the same as described for the first embodiment.

(Fourth embodiment)

In mature lymphoma, it is known that, in bone marrow aspirate, the number of lymphocytic tumor cells is increased as compared with the number of mononuclear cells in an early differentiation stage. In another embodiment, an index concerning screening for mature lymphoma may be obtained based on the number of the target mononuclear cells, and the numbers of the mononuclear leukocytes and erythroblasts in a late differentiation stage. This embodiment is also referred to as "fourth embodiment". In the fourth embodiment, in the target cell counting step, the target mononuclear cells, mononuclear leukocytes, and erythroblasts in a late differentiation stage are counted. The counting can be performed based on the fluorescence signal information and the side scattered light information. Mononuclear leukocyte and erythroblast in a late differentiation stage are mononuclear cells for which the fluorescence signal information represents a value less than a threshold value. In FIG. 3A, in the scattergram, mononuclear leukocytes and erythroblasts in a late differentiation stage can be sorted as particles included in a region below the transverse line and leftward of the oblique line. By counting the particles included in the region, mononuclear leukocytes and erythroblasts in a late differentiation stage can be counted. The mononuclear leukocytes and erythroblasts in a late differentiation stage may be distinguished from each other and counted. Alternatively, mononuclear leukocytes and erythroblasts in a late differentiation stage may be counted as one cell group including the mononuclear leukocytes and the erythroblasts in a late differentiation stage. The target mononuclear cells can be counted as particles included in the second region, as described above. The counting can be performed by, for example, analysis software mounted to the FCM.

In the scattergram, a region (hereinafter, also referred to as "fourth region") in which mononuclear leukocytes and erythroblasts in a late differentiation stage appear may be empirically set by accumulating data of the scattergrams based on the fluorescence signal information and the side scattered light information that are obtained by FCM measurement of normal bone marrow aspirates and bone marrow aspirates of hematopoietic tumors such as acute leukemia, similarly to the second region. Alternatively, the fourth region may be determined based on the scattergram in which the fluorescence signal intensity and the side scattered light intensity are each expressed in a range from 0 to 255 channels. Specifically, in the scattergram, a region surrounded by the following four coordinates of (X:Y)=(0 ch:0 ch), (0 ch:100 ch), (135 ch:100 ch), and (0 ch:100 ch) can be set as the fourth region. X represents a side scattered light intensity and Y represents a fluorescence signal intensity.

Alternatively, the four coordinates of the fourth region may be set based on the analysis results of a plurality of control samples each containing normal blood and the above-described fluorescent dye. The number of the control samples is at least 20, and is preferably 20 or more and 40 or less. For example, in FIG. 4C, a region surrounded by the point A, the point D, the originating point, and a point H is set as the fourth region. FIG. 4C shows a scattergram of normal blood in which the X-axis represents side scattered light intensities and the Y-axis represents fluorescence signal intensities. In FIG. 4C, the straight line extending across the two-dimensional plane of the scattergram is a straight line that indicates the threshold value corresponding to the fluorescence signal intensity. The straight line can be set based on the analysis results of the control samples as described for the first embodiment. The point A and the point D in FIG. 4C are the same as the point A and the point D in FIG. 3B. Therefore, the coordinates of these points are the same as described for the second embodiment. An X coordinate of the point H can be determined as an intersection of the X-axis and a line

obtained by extending a straight line BA toward the X-axis. A Y coordinate of the point H is 0 as shown in FIG. 4C. In a case where the fourth region specified by the four coordinates thus determined is applied to the scattergram generated based on FCM measurement of the bone marrow aspirate, the fourth region can include or overlap a region in which the above-described mononuclear leukocytes and erythroblasts in a late differentiation stage appear.

Alternatively, the fourth region may be set from the scattergram of the control samples each containing peripheral bloods of healthy individuals and the above-described fluorescent dye, similarly to the second region. In the scattergram based on the fluorescence signal information and the side scattered light information, a region in which 95% or more of mononuclear leukocytes is included, the fluorescence signal intensity is less than or equal to the fluorescence signal intensity of the monocyte population, and the side scattered light intensity is less than or equal to the side scattered light intensity of the neutrophil population, is specified as the fourth region. In a case where the fourth region specified based on FCM measurement of peripheral bloods of healthy individuals is applied to the scattergram generated based on FCM measurement of the bone marrow aspirate, the fourth region can include or overlap a region in which the mononuclear leukocytes and erythroblasts in a late differentiation stage appear.

A ratio of the numbers of mononuclear leukocytes and erythroblasts in a late differentiation stage, to the number of nucleated cells is preferably obtained in order to obtain an index concerning screening for mature lymphoma. Hereinafter, the ratio is also referred to as "fourth ratio". The number of nucleated cells represents the number that is counted based on the optical information obtained by FCM measurement of the sample as described above. The fourth ratio represents a value calculated according to the following Equation (D).

(Fourth ratio)=[(the numbers of mononuclear leukocytes and erythroblasts in a late differentiation stage)/(the number of nucleated cells)]$\times$100 (D)

The fourth ratio also includes values based on the value calculated according to Equation (D) as long as tendency of increase in the number of mononuclear leukocytes and erythroblasts in a late differentiation stage can be indicated based on the number of nucleated cells in the sample. Examples of the values based on the value calculated according to Equation (D) are similar to the examples of the values based on the value calculated according to Equation (A).

In the fourth embodiment, the second ratio and the fourth ratio may be obtained as the index concerning screening for mature lymphoma. Whether the number of the target mononuclear cells tends to increase, or the numbers of mononuclear leukocytes and erythroblasts in a late differentiation stage tend to increase, can be known from these ratios. Preferably, a ratio of the number of the target mononuclear cells to the numbers of mononuclear leukocytes and erythroblasts in a late differentiation stage is obtained as the index concerning screening for mature lymphoma. Hereinafter, the ratio is also referred to as "fifth ratio". The fifth ratio is a ratio of the value of the second ratio to the value of the fourth ratio, and is calculated according to the following Equation (E). The second ratio is a value calculated according to Equation (B).

$$\text{(Fifth ratio)=[(second ratio)/(fourth ratio)]}\times 100 \cdots \text{(E)}$$

The fifth ratio can also be obtained by dividing the number of the target mononuclear cells by the numbers of mononuclear leukocytes and erythroblasts in a late differentiation stage, and calculating a percentage. In a case where Equation (E) is used, the fourth ratio is required. As a result, it is advantageous in that not only the fifth ratio but also the second ratio and the fourth ratio can be obtained.

In the fourth embodiment, whether or not the bone marrow aspirate is abnormal may be determined according to comparison between the fifth ratio and a threshold value. In a case where the fifth ratio is a value calculated according to Equation (E), the threshold value for the fifth ratio can be, for example, any value between 5% and 15% and preferably 10%. For example, when the fifth ratio is less than the threshold value in comparison between the fifth ratio and the threshold value, the bone marrow aspirate is determined to be abnormal. In this case, it is considered that the number of lymphocytic tumor cells is increased in the bone marrow aspirate of the subject. This means that there is onset or suspicion of mature lymphoma. Meanwhile, when the fifth ratio indicates the threshold value or more, it is determined that the bone marrow aspirate is not abnormal.

In the fourth embodiment, in a case where the fifth ratio is less than the threshold value, distinguishable display may be provided. The distinguishable display is preferably made on a screen of a display device, similarly to the first ratio. On the screen of the display device, for example, the fifth ratio itself is displayed as the distinguishable display, and/or distinguishable display indicating that the fifth ratio is less than the threshold value is made, in a form that can distinguish characters and numerical values of the distinguishable display from the other characters and numerical values. The second ratio and the fourth ratio may also be displayed together. The display device and a manner for display on the screen are the same as described for the first embodiment. In a case where the fifth ratio is less than the threshold value, the distinguishable display may indicate that the bone marrow aspirate is a specimen that needs to be preferentially tested for a

hematopoietic tumor, in particular, mature lymphoma. The test is as described above. In the fourth embodiment, focus is particularly placed on mature lymphoma. Therefore, in a case where the fifth ratio is less than the threshold value, for example, a remark, such as "Mature Lymphocytosis?", indicating increase in the number of lymphocytes, may be displayed on the screen of the display device. The significance and effect of the distinguishable display are the same as described for the first embodiment.

(Fifth embodiment)

In plasmacytoma, a ratio of plasmacytes to nucleated cells in bone marrow aspirate is increased. The plasmacyte is an antibody-producing cell, and is thus known to have a greater amount of nucleic acid even as compared with a blast cell and erythroblast in an early differentiation stage. In another embodiment, an index concerning screening for plasmacytoma may be obtained based on the number of plasmacytes. This embodiment is also referred to as "fifth embodiment". In the fifth embodiment, plasmacytes are counted in the target cell counting step.

As described above, plasmacyte contains a greater amount of nucleic acid even as compared with a blast cell or erythroblast in an early differentiation stage. Therefore, the threshold value corresponding to the fluorescence signal information is preferably set to be higher than that in the first embodiment in order to count plasmacytes in the target cell counting step. For example, as the threshold value corresponding to the fluorescence signal information, a fluorescence signal intensity higher than a fluorescence signal intensity of the blast cell population or the population of erythroblasts in an early differentiation stage can be set. The FCM performs A/D conversion of analog signals of fluorescence and scattered light at predetermined sampling rates, and generates digital signals. As described above, in a case where the digital signal is expressed by 8 bit data, the ranges of the fluorescence signal intensity and the side scattered light intensity in the scattergram are each a range from 0 to 255 ch. In a case where the analog signal of fluorescence emitted from the particle is such that an intensity is within a range from 0 to 255 ch after the A/D conversion, the analog signal is converted to a digital signal in the range from 0 to 255 ch. However, in a case where an analog signal of fluorescence emitted from the particle is such that an intensity is higher than 255 ch after the A/D conversion, the analog signal is converted to a 255 ch digital signal regardless of the intensity. In the scattergram, even when a particle has a fluorescence signal intensity higher than 255 ch, the fluorescence signal intensity is plotted at a coordinate of 255 ch. The fluorescence signal intensities of a blast cell and erythroblast in an early differentiation stage are each in a range from 100 to 255 in general. However, the fluorescence signal intensity of plasmacyte is higher than 255 in general. Therefore, in the scattergram represented in a range from 0 to 255 ch, it is difficult to clearly distinguish plasmacytes from blast cells and erythroblasts in an early differentiation stage. Therefore, in the fifth embodiment, a digital signal for fluorescence is, for example, expressed by 10 to 16 bit data, and preferably expressed by 10 bit data. In a case where the digital signal is expressed by 10 bit data, the fluorescence signal intensity is expressed by 1024 gradations from 0 to 1023 ch.

Since plasmacyte is a mononuclear cell, plasmacyte can be distinguished from a megakaryocytic cell or immature granulocyte in an early differentiation stage based on the side scattered light information. Therefore, in the fifth embodiment, plasmacytes can be sorted based on the fluorescence signal information and the side scattered light information of particles in the sample. Alternatively, after the target cells are sorted, plasmacytes may be sorted based on the side scattered light information of the target cells.

Counting of plasmacytes will be described with reference to FIG. 5A. In FIG. 5A, the vertical axis (fluorescence signal intensity) in the scattergram shown in FIG. 1 is expressed by 0 to 1023 ch, and the horizontal axis (side scattered light intensity) is expressed by 0 to 255 ch. A region (hereinafter, also referred to as "fifth region") in which plasmacytes appear is indicated by dashed lines. In the fifth embodiment, particles included in the region surrounded by the dashed lines in the scattergram in FIG. 5A can be specified as plasmacytes. Plasmacytes can be counted by, for example, analysis software mounted to the FCM.

The fifth region may be a preset region, or may be set so as to be variable for each specimen. For example, the fifth region may be empirically set by accumulating data of the scattergrams based on the fluorescence signal information and the side scattered light information that are obtained by FCM measurement of normal bone marrow aspirates and bone marrow aspirates of hematopoietic tumors such as acute leukemia. Alternatively, the fifth region may be determined based on the scattergram in which a fluorescence signal intensity is expressed by 0 to 1023 ch and the side scattered light intensity is expressed by 0 to 255 ch. Specifically, in the scattergram, a region surrounded by the following four coordinates of (X:Y)=(0 ch:1023 ch), (170 ch:1023 ch), (170 ch:296 ch), and (0 ch:296 ch) can be set as the fifth region. X represents a side scattered light intensity and Y represents a fluorescence signal intensity.

Alternatively, the four coordinates of the fifth region may be set based on the analysis results of a plurality of control samples each containing normal blood and the above-described fluorescent dye. The number of the control samples is at least 20, and is preferably 20 or more and 40 or less. For example, in FIG. 5B, a region surrounded by a point I, a point J, a point K, and a point L is set as the fifth region. FIG. 5B shows a scattergram of normal blood in which the X-axis represents side scattered light intensities and the Y-axis represents fluorescence signal intensities. In the scattergram, the fluorescence signal intensity is expressed by 0 to 1023 ch, and the side scattered light intensity is expressed by 0 to 255 ch.

As in FIG. 5B, an X coordinate of the point I is 0, and a Y coordinate thereof is the maximum value of the fluorescence signal intensity in the scattergram. As in FIG. 5B, an X coordinate of the point J is 0. A Y coordinate of the point J can be, for example, determined as a value (that is, 14 to 16×IQR) that is 15+1 times higher than the average of values in the IQR (interquartile range) of the fluorescence signal intensities of nucleated cells in the plurality of control samples. An X coordinate of the point K can be, for example, determined as any value between the average value of the medians of the neutrophil populations of the respective specimens at the X-axis, and the average value of the medians of the eosinophil populations at the X-axis. A Y coordinate of the point K is the same as the Y coordinate of the point J. An X coordinate of the point L is the same as the X coordinate of the point K, and a Y coordinate of the point L is the maximum value of the fluorescence signal intensity in the scattergram. In a case where the fifth region specified by the four coordinates thus determined is applied to the scattergram generated based on FCM measurement of the bone marrow aspirate, the fifth region can include or overlap a region in which plasmacytes appear.

In the fifth embodiment, the screening index for plasmacytoma is obtained based on the number of plasmacytes. The number of plasmacytes may be used, as it is, as the screening index for plasmacytoma. Preferably, a ratio of the number of plasmacytes to the number of nucleated cells is obtained as the screening index for plasmacytoma. Hereinafter, the ratio is also referred to as "sixth ratio". The number of nucleated cells represents the number that is counted based on the optical information obtained by FCM measurement of the sample as described above. The sixth ratio represents a value calculated according to the following Equation (F).

$$\text{(Sixth ratio)}=[\text{(the number of plasmacytes)}/\text{(the number of nucleated cells)}]\times 100 \quad \cdots\text{(F)}$$

The sixth ratio also includes values based on the value calculated according to Equation (F) as long as tendency of change in the number of plasmacytes is indicated based on the number of nucleated cells in the sample. Examples of the values based on the value calculated according to Equation (F) are similar to the examples of the values based on the value calculated according to Equation (A).

In the fifth embodiment, whether or not the bone marrow aspirate is abnormal may be determined according to comparison between the sixth ratio and a threshold value. In a case where the sixth ratio is a value calculated according to Equation (F), the threshold value for the sixth ratio can be, for example, any value between 5% and 15% and preferably 5%. For example, when the sixth ratio indicates the threshold value or more in comparison between the sixth ratio and the threshold value, the bone marrow aspirate is determined to be abnormal. In this case, it is considered that the number of plasmacytes is increased in the bone marrow aspirate of the subject as compared with normal bone marrow aspirate. This means that there is onset or suspicion of plasmacytoma. Meanwhile, when the sixth ratio is less than the threshold value, it is determined that the bone marrow aspirate is not abnormal.

In the fifth embodiment, in a case where the sixth ratio indicates the threshold value or more, distinguishable display may be provided. The distinguishable display is preferably made on a screen of a display device, similarly to the first ratio. On the screen of the display device, for example, the sixth ratio itself is displayed as the distinguishable display, and/or distinguishable display indicating that the ratio indicates the threshold value or more is made, in a form that can distinguish characters and numerical values of the distinguishable display from the other characters and numerical values. The display device and a manner for display on the screen are the same as described for the first embodiment. In a case where the sixth ratio indicates the threshold value or more, the distinguishable display may indicate that the bone marrow aspirate is a specimen that needs to be preferentially tested for a hematopoietic tumor, in particular, plasmacytoma. The test is as described above. In the fifth embodiment, focus is particularly placed on plasmacytoma. Therefore, in a case where the sixth ratio indicates the threshold value or more, for example, a remark, such as "Plasmacytosis?", indicating increase in the number of plasmacytes may be displayed on the screen of the display device. The significance and effect of the distinguishable display are the same as described for the first embodiment.

Thus, in the analysis method of the present embodiment, information for supporting, for example, screening for a hematopoietic tumor and determination as to whether or not bone marrow aspirate is abnormal can be provided to medical professionals such as doctors. The medical professional who has obtained the information is allowed to determine whether or not the analyzed bone marrow aspirate is to be preferentially tested for a hematopoietic tumor. Furthermore, myelogram can be performed in consideration of a suspected hematopoietic tumor and noticeable cells according to the obtained information.

[2. Sample analyzer]

An example of a sample analyzer of the present embodiment will be described below with reference to the drawings.

(Configuration of sample analyzer)

As shown in FIG. 6, a sample analyzer 10 includes a measurement unit 20 and an analysis unit 30. The measurement unit 20 takes in bone marrow aspirate, prepares a sample from the bone marrow aspirate, and performs optical measurement of the sample. The analysis unit 30 processes measurement data obtained through the measurement by the measurement unit 20, and outputs an analysis result of the bone marrow aspirate. However, the present embodiment is not limited to this example. For example, the measurement unit 20 and the analysis unit 30 may be integrated with each other as one apparatus.

The measurement unit 20 includes a suction part 40, a sample preparation part 50, a detector 60, a signal processing circuit 81, a microcomputer 82, and a communication interface 83. The suction part 40 has a suction tube 42. The suction part 40 suctions bone marrow aspirate contained in a test tube 41 by using the suction tube 42.

The sample preparation part 50 has a reaction chamber 54, and is connected to reagent containers 51, 52, and 53. The test tube 41 stores bone marrow aspirate. The reagent container 51 stores diluent. The diluent stored in the reagent container 51 is used as a sheath liquid in measurement by flow cytometry. The reagent container 52 stores a hemolytic reagent containing a hemolytic agent. The reagent container 53 stores a staining reagent containing a fluorescent dye. The suction part 40 moves the suction tube 42 upward of the reaction chamber 54, and discharges the bone marrow aspirate suctioned from the test tube 41, into the reaction chamber 54. The bone marrow aspirate, the hemolytic reagent, and the staining reagent are mixed in the reaction chamber 54, and the sample is thus prepared. Instead of the staining reagent and the hemolytic reagent being used, one reagent containing both the fluorescent dye and the cationic surfactant may be used. The sample is subjected to optical measurement by flow cytometry.

The detector 60 is used for optical measurement of a particle by flow cytometry. The detector 60 includes a flow cell 61, a light source part 62, and light receivers 63 and 64. To the flow cell 61, the diluent stored in the reagent container 51 and the sample prepared by the sample preparation part 50 are supplied. Hereinafter, a method in which the detector 60 obtains the optical information including the fluorescence signal information for a particle in the sample will be described. However, the method is not limited to one described below.

The flow cell 61 is made of a material, such as quartz, glass, or synthetic resin, having translucency, and is formed into a tubular shape. Inside the flow cell 61, a flow path in which the sample and the sheath liquid flow is formed. In FIG. 7, the flow cell 61 has an orifice 61a at which an internal space is made narrower than the other portions. A double-tube structure is formed near the inlet of the orifice 61a, the inner tube portion serves as a sample nozzle 61b, and the sample prepared by the sample preparation part 50 is supplied through the sample nozzle 61b. A space outside the sample nozzle 61b serves as a flow path 61c through which the sheath liquid flows. The sheath liquid is introduced into the orifice 61a through the flow path 61c. Thus, the sheath liquid supplied to the flow cell 61 flows so as to envelop the sample discharged from the sample nozzle 61b. The flow of the sample is narrowed by the orifice 61a, and particles, in the sample, which are enveloped by the sheath liquid pass through the orifice 61a one by one.

The light source part 62 is a semiconductor laser light source, and, for example, applies red laser light having a wavelength of 633 nm, to the orifice 61a of the flow cell 61. The wavelength of the laser is not particularly limited, and a laser light source in which the wavelength is appropriate for excitation of the fluorescent dye can be selected as appropriate. When light is applied to the flow of the sample in the flow cell 61, the light receivers 63, 64, and 65 detect light emitted from an individual particle in the sample. An avalanche photodiode, a photodiode, or a photomultiplier can be adopted as the light receivers 63, 64, and 65. Hereinafter, the direction connecting between the light source part 62 and the flow cell 61 is referred to as "X direction", and the direction orthogonal to the X direction is referred to as "Y direction". A dichroic mirror 66 is disposed on the Y direction side with respect to the flow cell 61. The dichroic mirror 66 transmits therethrough fluorescence emitted from an individual particle, and reflects side scattered light emitted from the individual particle. The light receiver 63 is disposed on the Y direction side with respect to the flow cell 61, and can detect fluorescence transmitted through the dichroic mirror 66. The light receiver 64 is disposed on the X direction side with respect to the flow cell 61. More specifically, the light receiver 64 is disposed on the side opposite to the light source part 62 across the flow cell 61. The light receiver 64 can detect forward scattered light emitted from an individual particle. The light receiver 65 can detect side scattered light reflected by the dichroic mirror 66.

The side scattered light is not limited to light scattered in the 90° direction (Y direction) relative to the optical axis direction (X direction) of the light source part 62. The side scattered light may be, for example, light scattered in the direction of 80° or more and 100° or less relative to the X direction. The forward scattered light is not limited to light scattered in the optical axis direction (X direction) of the light source part 62. The forward scattered light may be, for example, light scattered in the direction of -10° or more and 10° or less relative to the X direction.

In the present embodiment, a light application lens system including a plurality of lenses (not shown) may be disposed between the light source part 62 and the flow cell 61. The light application lens system allows collimated beams emitted from the semiconductor laser light source to be condensed at a beam spot.

The light receivers 63, 64, and 65 receive fluorescence, forward scattered light, and side scattered light, respectively, and output, to the signal processing circuit 81, electric analog signals having different voltages according

to an amount of received light. That is, the light receivers 63, 64, and 65 each output an analog signal indicating an intensity of the received light. Hereinafter, the analog signal outputted from the light receiver 63 is referred to as "fluorescence signal", the analog signal outputted from the light receiver 64 is referred to as "forward scattered light signal", and the analog signal outputted from the light receiver 65 is referred to as "side scattered light signal". These analog signals are inputted to the signal processing circuit 81 as waveform signals indicating voltages that change so as to form waveforms according to passage of a particle in the flow cell 61.

The signal processing circuit 81 performs A/D conversion of the analog signals outputted by the light receivers 63, 64, 65 at predetermined sampling rates, and thus generates a fluorescence signal, a forward scattered light signal, and a side scattered light signal which are digital signals. The signal processing circuit 81 extracts a peak value of the fluorescence signal corresponding to a waveform of one particle, as a "fluorescence signal intensity" of the particle. Similarly, the signal processing circuit 81 extracts a peak value of the forward scattered light signal as a "forward scattered light intensity", and extracts a peak value of the side scattered light signal as a "side scattered light intensity".

In a case where the digital signal generated by the signal processing circuit 81 is, for example, expressed by 8 bit data, the range of the fluorescence signal intensity is, for example, 0 to 255 ch. In a case where the digital signal is, for example, expressed by 10 bit data, the range of the fluorescence signal intensity is, for example, 0 to 1023 ch. In a case where the digital signal is, for example, expressed by 16 bit data, the fluorescence signal intensity is, for example, expressed in a range from 0 to 65535. In a case where the particles detected by FCM measurement are plotted in a scattergram in which the vertical axis represents 256 channel fluorescence signal intensities, particles having the fluorescence intensities of 0 to 255 are plotted at coordinates of the fluorescence intensities of 0 to 255 channels as they are. Meanwhile, a particle having the fluorescence signal intensity of 256 or more (for example, 500) is plotted at a coordinate of 255 channel. However, in a case where particles are plotted in a scattergram in which the vertical axis represents 1023 channel fluorescence signal intensities, a particle having the fluorescence signal intensity of 500 is plotted at a coordinate of 500 channel.

The microcomputer 82 controls the suction part 40, the sample preparation part 50, the detector 60, the signal processing circuit 81, and the communication interface 83. The communication interface 83 is connected to the analysis unit 30 by a communication cable. The measurement unit 20 performs data communication with the analysis unit 30 via the communication interface 83. The communication interface 83 allows measurement data including feature parameters to be transmitted to the analysis unit 30.

A configuration of the analysis unit 30 will be described with reference to FIG. 8. The analysis unit 30 includes a body 300, an input part 309, and a display part 310. The body 300 has a CPU 301, a ROM 302, a RAM 303, a solid-state drive (SSD) 304, a readout device 305, an input/output interface 306, an image-output interface 307, and a communication interface 308. The body 300 may include a hard disk instead of the SSD. In FIG. 8, a display for displaying an image is used as the display part 310.

The CPU 301 executes a computer program 322 stored in the ROM 302, and a computer program loaded to the RAM 303. The RAM 303 is used for reading out each of computer programs stored in the ROM 302 and the SSD 304. The RAM 303 is used also as a work area for the CPU 301 when each computer program is executed. An application program 320 as a computer program for analyzing measurement data provided from the measurement unit 20 and outputting the analysis result is installed in the SSD 304. The computer program 322 includes a basic input/output system (BIOS). The application program 320 includes an OS, a bone marrow aspirate analyzing program, and a program for determining whether or not bone marrow aspirate is abnormal. The bone marrow aspirate analyzing program is a program for obtaining the number of target cells based on the optical information, and obtaining the screening index for a hematopoietic tumor based on the number of target cells. The program for determining whether or not bone marrow aspirate is abnormal is a program for determining whether or not bone marrow aspirate is abnormal, based on the screening index for a hematopoietic tumor.

The readout device 305 is implemented by a USB port, an SD card reader, a CF card reader, a memory stick reader, a CD-ROM drive, a DVD-ROM drive, a flexible disk drive, or the like, and can read out a computer program or data stored in a portable storage medium 321. The computer program 320 for causing a computer to function as the analysis unit 30 is stored in the portable storage medium 321. The computer program 320 which is read out from the portable storage medium 321 is installed in the SSD 304.

The input part 309 is connected to the input/output interface 306. The display part 310 is connected to the image-output interface 307. The communication interface 308 is connected to the communication interface 83 of the measurement unit 20. Instead of the input part 309 and the display part 310, a touch panel having an input part disposed on the surface of the display part may be provided as a display input part. A known type of a touch panel such as a capacitance type touch panel may be used as a touch panel.

(Operation of sample analyzer)

An operation of the sample analyzer 10 will be described with reference to FIG. 9. Firstly, an instruction for

performing measurement is received from a user through the input part 309 by the CPU 301 of the analysis unit 30 (step S101).

When the analysis unit 30 receives the instruction for performing the measurement, the CPU 301 operates to transmit instruction data for starting the measurement to the measurement unit 20 (step S102), and the measurement unit 20 receives the instruction data (step S103). The microcomputer 82 of the measurement unit 20 operates to perform a sample preparation process (step S104), and perform a measurement process (step S105).

The sample preparation process will be described with reference to FIGS. 6 and 10. The microcomputer 82 causes the suction part 40 to supply a predetermined amount of bone marrow aspirate to the reaction chamber 54 (step S201). Subsequently, the microcomputer 82 controls the sample preparation part 50 so as to supply a predetermined amount of hemolytic reagent from the reagent container 52 to the reaction chamber 54, and supply a predetermined amount of staining reagent from the reagent container 53 to the reaction chamber 54 (step S202). The reaction chamber 54 is heated by a heater and has a predetermined temperature (for example, 30 to 45°C). In the heated state, a mixture in the reaction chamber 54 is stirred (step S203). Through the operations of step S201 to S203, the sample is prepared in the reaction chamber 54. The microcomputer 82 causes the sample preparation part 50 to introduce the sample from the reaction chamber 54 to the detector 60 (step S204). When the process step of step S204 has ended, the microcomputer 82 returns the process to a main routine.

When the sample passes through the flow cell 61 of the detector 60, laser light is applied to a particle in the sample by the light source part 62. Fluorescence emitted from the particle is detected by the light receiver 63. Forward scattered light emitted from the particle is detected by the light receiver 64. Side scattered light emitted from the particle is detected by the light receiver 65. The light receivers 63, 64, and 65 output electric signals according to light reception levels, as a fluorescence signal, a forward scattered light signal, and a side scattered light signal, respectively. The signal processing circuit 81 extracts a fluorescence intensity from the fluorescence signal, extracts a forward scattered light intensity from the forward scattered light signal, and extracts a side scattered light intensity from the side scattered light signal. In FIG. 9, after the measurement process, the microcomputer 82 operates to transmit measurement data including feature parameters to the analysis unit 30 (step S106), and ends the process. The analysis unit 30 receives the measurement data (step S107). Thereafter, the CPU 301 operates to perform a measurement data analyzing process, generate the analysis result of the bone marrow aspirate, and store the analysis result in the SSD 304 (step S108).

[3. Computer program]

An example of the measurement data analyzing process will be described below. In FIG. 11A, in the measurement data analyzing process of the first embodiment, when the measurement data analyzing process is started, the CPU 301 of the analysis unit 30 operates to sort target cells and nucleated cells based on the fluorescence intensity included in the measurement data (step S301). The CPU 301 operates to exclude cells and particles having no nucleuses from the particles in the sample based on the fluorescence signal intensity, and thus sort nucleated cells. Furthermore, the CPU 301 operates to extract particles for which the fluorescence signal intensity indicates a threshold value or more, from the particles in the sample, and thus sort the target cells. The CPU 301 may operate to generate a scattergram by using data of the fluorescence signal intensities and the side scattered light intensities.

In step S302, the CPU 301 operates to count the target cells and the nucleated cells which are sorted in step S301, and stores the numbers thereof in the SSD 304. In step S303, the CPU 301 operates to obtain a screening index for a hematopoietic tumor based on the number of the target cells and the number of the nucleated cells. In a case where the first ratio is obtained as the index, the CPU 301 performs the calculation represented by the above-described Equation (A), and thus obtains the first ratio. The CPU 301 operates to store the obtained screening index for a hematopoietic tumor in the SSD 304. In step S304, the CPU 301 determines whether or not the bone marrow aspirate is abnormal based on the screening index for a hematopoietic tumor.

The measurement data analyzing process of the second embodiment will be described with reference to FIG. 11B. When the measurement data analyzing process is started, the CPU 301 of the analysis unit 30 operates to sort target mononuclear cells and nucleated cells based on the fluorescence intensity and the side scattered light intensity which are included in the measurement data (step S311). The nucleated cells are sorted in the same manner as in the analyzing process of the first embodiment. The CPU 301 operates to extract particles included in the second region, from particles in the sample, based on the fluorescence signal intensity and the side scattered light intensity, and thus sort the target mononuclear cells. The CPU 301 may operate to generate a scattergram by using data of the fluorescence signal intensities and the side scattered light intensities.

In step S312, the CPU 301 operates to count the target mononuclear cells and the nucleated cells which are sorted in step S311, and store the numbers thereof in the SSD 304. In step S313, the CPU 301 operates to obtain a screening index for a hematopoietic tumor based on the number of the target mononuclear cells and the number of the nucleated cells. In a case where the second ratio is obtained as the index, the CPU 301 performs the calculation represented by the above-described Equation (B), and thus obtains the second ratio. The CPU 301 operates to store the

obtained screening index for a hematopoietic tumor in the SSD 304. Step S314 is the same as step S304. That is, the CPU 301 determines whether or not the bone marrow aspirate is abnormal, in step S314.

The measurement data analyzing process of the third embodiment will be described with reference to FIG. 11C. In this analyzing process, a screening index for acute leukemia is obtained as a screening index for a hematopoietic tumor. When the measurement data analyzing process is started, the CPU 301 of the analysis unit 30 operates to sort blast cells and nucleated cells based on the fluorescence intensity, the side scattered light intensity, and the forward scattered light intensity which are included in the measurement data (step S321). The nucleated cells are sorted in the same manner as in the analyzing process of the first embodiment. The CPU 301 operates to extract particles included in the third region, from the particles in the sample, based on the fluorescence signal intensity and the side scattered light intensity. The CPU 301 may operate to generate a scattergram by using data of the fluorescence signal intensities and the side scattered light intensities. The blast cells are sorted by extracting particles each corresponding to the size of a blast cell, based on the forward scattered light intensity of the extracted particle. The CPU 301 may operate to generate a histogram by using data of the forward scattered light intensities.

In step S322, the CPU 301 operates to count the blast cells and the nucleated cells which are sorted in step S321, and store the numbers thereof in the SSD 304. In step S323, the CPU 301 operates to obtain a screening index for acute leukemia based on the number of the blast cells and the number of the nucleated cells. In a case where the third ratio is obtained as the index, the CPU 301 performs the calculation represented by the above-described Equation (C), and thus obtains the third ratio. The CPU 301 operates to store the obtained screening index for acute leukemia in the SSD 304. Step S324 is the same as step S304. That is, the CPU 301 determines whether or not the bone marrow aspirate is abnormal, in step S324.

The measurement data analyzing process of the fourth embodiment will be described with reference to FIG. 11D. In this analyzing process, a screening index for mature lymphoma is obtained as a screening index for a hematopoietic tumor. When the measurement data analyzing process is started, the CPU 301 of the analysis unit 30 operates to sort the target mononuclear cells, the mononuclear leukocytes, the erythroblasts in a late differentiation stage, and nucleated cells based on the fluorescence intensity, the side scattered light intensity, and the forward scattered light intensity which are included in the measurement data (step S331). The nucleated cells are sorted in the same manner as in the analyzing process of the first embodiment. The CPU 301 operates to sort the target mononuclear cells by extracting particles included in the second region, from the particles in the sample, based on the fluorescence signal intensity and the side scattered light intensity. Furthermore, mononuclear leukocytes and erythroblasts in a late differentiation stage are sorted by extracting particles included in the fourth region. The CPU 301 may operate to generate a scattergram by using data of the fluorescence signal intensities and the side scattered light intensities.

In step S332, the CPU 301 operates to count the target mononuclear cells, the mononuclear leukocytes, the erythroblasts in a late differentiation stage, and the nucleated cells which are sorted in step S331, and store the numbers thereof in the SSD 304. In step S333, the CPU 301 operates to obtain a screening index for mature lymphoma based on the number of the target mononuclear cells, the numbers of the mononuclear leukocytes and the erythroblasts in a late differentiation stage, and the number of the nucleated cells. In a case where the fifth ratio is obtained as the index, the CPU 301 performs the calculations represented by the above-described Equations (B) and (D), and thus obtains the second ratio and the fourth ratio. Furthermore, the CPU 301 performs the calculation represented by the above-described Equation (E), and thus obtains the fifth ratio. The CPU 301 operates to store the obtained screening index for mature lymphoma in the SSD 304. Step S334 is the same as step S304. That is, the CPU 301 determines whether or not the bone marrow aspirate is abnormal, in step S334.

The measurement data analyzing process of the fifth embodiment will be described with reference to FIG. 11E. In this analyzing process, a screening index for plasmacytoma is obtained as a screening index for a hematopoietic tumor. When the measurement data analyzing process is started, the CPU 301 of the analysis unit 30 operates to sort plasmacytes and nucleated cells based on the fluorescence intensity and the side scattered light intensity which are included in the measurement data (step S341). In this analyzing process, the digital signal for fluorescence is expressed by 10 bit data, and the digital signal for the side scattered light is expressed by 8 bit data. Therefore, the range of the fluorescence signal intensity is 0 to 1023 ch, and the range of the side scattered light intensity is 0 to 255 ch. The nucleated cells are sorted in the same manner as in the analyzing process of the first embodiment. The CPU 301 operates to sort plasmacytes by extracting particles included in the fifth region, from the particles in the sample, based on the fluorescence signal intensity and the side scattered light intensity. The CPU 301 may operate to generate a scattergram by using data of the fluorescence signal intensities and the side scattered light intensities.

In step S342, the CPU 301 operates to count the plasmacytes and the nucleated cells which are sorted in step S341, and stores the numbers thereof in the SSD 304. In step S343, the CPU 301 operates to obtain a screening index for plasmacytoma, based on the number of plasmacytes and the number of the nucleated cells. In a case where the sixth ratio is obtained as the index, the CPU 301 performs the calculation represented by the above-described Equation (F), and thus obtains the sixth ratio. The CPU 301 operates to store the obtained screening index for plasmacytoma in the SSD 304. Step S344 is the same as step S304. That is, the CPU 301 determines whether or not the bone marrow aspirate is abnormal, in

step S344.

In FIG. 9, when the above-described measurement data analyzing process is ended, the CPU 301 operates to output the analysis result to the display part 310 in step S109, and end the process.

The measurement data analyzing processes shown in FIGS. 11A to 11E, respectively, have been separately described above. However, the measurement data analyzing processes (step S108) shown in FIG. 9 may be partially performed, or the entirety of the analyzing processes may be performed. For example, in FIG. 11A, the target mononuclear cells, blast cells, mononuclear leukocytes, erythroblasts in a late differentiation stage, and plasmacytes as well as target cells and nucleated cells may be sorted in step S301 and counted in step S302. Furthermore, in step S303, the screening indexes for acute leukemia, mature lymphoma, and plasmacytoma may be obtained as the screening index for hematopoietic tumors.

A case in which whether or not a subject is suspected of having a hematopoietic tumor is determined, will be described below as an example of the determination process for determining whether or not bone marrow aspirate is abnormal. In FIG. 12A, in the determination process of the first embodiment, the first ratio calculated according to Equation (A) is obtained as the screening index for a hematopoietic tumor, and determination is performed based on the ratio. However, the present disclosure is not limited thereto. In step S401, the CPU 301 operates to compare the first ratio and a threshold value (for example, 30%) for the first ratio with each other. The threshold value is stored in the SSD 304. When the first ratio is less than the predetermined threshold value, the process proceeds to step S402. The CPU 301 obtains a determination result that there is no suspicion of a hematopoietic tumor, and stores the determination result in the SSD 304. When the first ratio indicates the predetermined threshold value or more, the process proceeds to step S403. The CPU 301 obtains a determination result that there is suspicion of a hematopoietic tumor, and stores the determination result in the SSD 304. Thus, the CPU 301 ends the determination process, and returns the process to the main routine.

In FIG. 12B, in the determination process of the second embodiment, the second ratio calculated according to Equation (B) is obtained as the screening index for a hematopoietic tumor, and the determination is performed based on the ratio. However, the present disclosure is not limited thereto. In step S411, the CPU 301 operates to compare the second ratio and a threshold value (for example, 20%) for the second ratio with each other. The threshold value is stored in the SSD 304. When the second ratio is less than the predetermined threshold value, the process proceeds to step S412. The CPU 301 obtains a determination result that there is no suspicion of a hematopoietic tumor, and stores the determination result in the SSD 304. When the second ratio indicates the predetermined threshold value or more, the process proceeds to step S413. The CPU 301 obtains a determination result that there is suspicion of a hematopoietic tumor, and stores the determination result in the SSD 304. Thus, the CPU 301 ends the determination process, and returns the process to the main routine.

In FIG. 12C, in the determination process of the third embodiment, the third ratio calculated according to Equation (C) is obtained as the screening index for acute leukemia, and the determination is performed based on the ratio. In the determination, the first threshold value, and the second threshold value that is higher than the first threshold value are used as predetermined threshold values for the third ratio. However, the present disclosure is not limited thereto. In step S421, the CPU 301 operates to compare the third ratio and the first threshold value (for example, 10%) with each other. The first threshold value is stored in the SSD 304. When the third ratio is less than the first threshold value, the process proceeds to step S422. The CPU 301 obtains a determination result that there is no suspicion of acute leukemia, and stores the determination result in the SSD 304. When the third ratio indicates the first threshold value or more, the process proceeds to step S423. In step S423, the CPU 301 operates to compare the third ratio and the second threshold value (for example, 20%) with each other. The second threshold value is stored in the SSD 304. When the third ratio is less than the second threshold value, the process proceeds to step S424. The CPU 301 obtains a determination result that there is suspicion of increase in the number of blast cells, and stores the determination result in the SSD 304. When the third ratio indicates the second threshold value or more, the process proceeds to step S425. The CPU 301 obtains a determination result that there is suspicion of acute leukemia, and stores the determination result in the SSD 304. Thus, the CPU 301 ends the determination process, and returns the process to the main routine.

In FIG. 12D, in the determination process of the fourth embodiment, the fifth ratio calculated according to Equation (E) is obtained as the screening index for mature lymphoma, and the determination is performed based on the ratio. However, the present disclosure is not limited thereto. In step S431, the CPU 301 operates to compare the fifth ratio and a threshold value (for example, 10%) for the fifth ratio with each other. The threshold value is stored in the SSD 304. When the fifth ratio is less than the predetermined threshold value, the process proceeds to step S432. The CPU 301 obtains a determination result that there is suspicion of mature lymphoma, and stores the determination result in the SSD 304. When the fifth ratio indicates the predetermined threshold value or more, the process proceeds to step S433. The CPU 301 obtains a determination result that there is no suspicion of mature lymphoma, and stores the determination result in the SSD 304. Thus, the CPU 301 ends the determination process, and returns the process to the main routine.

In FIG. 12E, in the determination process of the fifth embodiment, the sixth ratio calculated according to Equation (F) is obtained as the screening index for plasmacytoma, and the determination is performed based on the ratio. However, the present disclosure is not limited thereto. In step S441, the CPU 301 operates to compare the sixth ratio and a threshold

value (for example, 5%) for the sixth ratio with each other. The threshold value is stored in the SSD 304. When the sixth ratio is less than the predetermined threshold value, the process proceeds to step S442. The CPU 301 obtains a determination result that there is no suspicion of plasmacytoma, and stores the determination result in the SSD 304. When the sixth ratio indicates the predetermined threshold value or more, the process proceeds to step S443. The CPU 301 obtains a determination result that there is suspicion of plasmacytoma, and stores the determination result in the SSD 304. Thus, the CPU 301 ends the determination process, and returns the process to the main routine.

In FIG. 9, when the above-described determination process has ended, the CPU 301 operates to output the determination result as the analysis result to the display part 310 in step S109, and end the process. "The determination process as to whether or not there is suspicion of a hematopoietic tumor", "the determination process as to whether or not there is suspicion of acute leukemia", "the determination process as to whether or not there is suspicion of mature lymphoma", and "the determination process as to whether or not there is suspicion of plasmacytoma" are separately described above with reference to FIGS. 12A to 12E. However, the determination processes for determining whether or not bone marrow aspirate is abnormal may be partially performed, or the entirety of the determination processes may be performed.

For example, the number of nucleated cells (TNC), and a ratio (RATIO) as the screening index for a hematopoietic tumor may be included in the analysis result screen outputted to the display part 310. In addition to such information, a scattergram generated based on the optical information may be included in the screen. Furthermore, a result of determining whether or not bone marrow aspirate is abnormal based on the screening index for a hematopoietic tumor may be included in the screen.

As described above, the sample analyzer and the computer program according to the present embodiment can provide medical professionals such as doctors with information for supporting, for example, screening for hematopoietic tumors and determination as to whether or not bone marrow aspirate is abnormal. The medical professional who has obtained the information is allowed to determine whether or not the analyzed bone marrow aspirate is to be preferentially tested for a hematopoietic tumor. Furthermore, myelogram can be performed in consideration of a suspected hematopoietic tumor and noticeable cells according to the obtained information.

The present disclosure will be described below in more detail by means of examples. However, the present disclosure is not limited to the examples.

[Examples]

Example 1: Screening based on the number of cells for which a fluorescence signal intensity indicated a predetermined threshold value or more

Acute leukemia has such a symptom that, for example, the number of immature cells in an early differentiation stage is increased in bone marrow. Furthermore, plasmacytoma (myeloma) has such a symptom that the number of plasmacytes is increased in bone marrow. An amount of nucleic acid in each of an immature cell in an early differentiation stage and plasmacyte is greater than that of each of a mature cell and a cell in a late differentiation stage. Therefore, a cell in bone marrow aspirate was analyzed by an FCM, and examination as to whether or not screening for a hematopoietic tumor was possible based on an amount of nucleic acid in the cell, was performed.

(1) Specimen

Bone marrow aspirates (38 cases in total), in the following cases, which were checked by myelogram (microscopic examination) in a hospital were used as specimens.

- acute leukemia (a ratio of the number of blast cells to the number of nucleated cells was 20% or more): 2 cases
- plasmacytoma (a ratio of the number of plasmacytes to the number of nucleated cells was 10% or more): one case
- moderate level increase of blast cells (a ratio of the number of blast cells to the number of nucleated cells was 10% or more and less than 20%): one case
- mild level increase of blast cells (a ratio of the number of blast cells to the number of nucleated cells was 5% or more and less than 10%): three cases
- cases in which increase in the numbers of blast cells and plasmacytes was not found: 31 cases

(2) Reagent and Analyzer

Lysercell WDFII (SYSMEX CORPORATION) was used as the hemolytic reagent. Fluorocell WDF (SYSMEX CORPORATION) was used as the staining reagent. An XR-1000 (SYSMEX CORPORATION) automated hematology analyzer having an FCM was used as an analyzer.

(3) Measurement

(3. 1) Preparation and microscopic examination of smear

Microscopic examination of the above-described bone marrow aspirates was performed for comparison with the analysis result from the analyzer. A part of each specimen was taken out and stained by a general method, to prepare a smear. The smear was observed by a microscope, and nucleated cells were counted. Furthermore, presence or absence of the following target cells was confirmed. In a case where the target cells were detected, the number of the cells was counted. The target cells in the microscopic examination were blast cells, proerythroblasts, polychromatic erythroblasts, plasmacytes, promonocytes, immature neutrophils (promyelocytes and myelocytes), and megakaryocytic cells. The number of the cells was the total number of these target cells.

(3. 2) Preparation and measurement of sample by analyzer

Each specimen was filtered through a nylon mesh having a pore diameter of 40 μm, to remove spicules, and set in the automated hematology analyzer XR-1000. The sample was automatically prepared and measured by the analyzer. Specifically, the hemolytic reagent (50 μL) and the staining reagent (1 μL) were added to the bone marrow aspirate (1 μL), and the obtained product was incubated at 40°C for 20 seconds, to prepare the sample. Light was applied to the sample, and the optical information was obtained from an individual particle in the sample. The fluorescence signal intensity and the side scattered light intensity were obtained as the optical information. Furthermore, a scattergram which had the horizontal axis representing side scattered light intensities and the vertical axis representing fluorescence signal intensities was generated based on the obtained optical information. In the scattergram, the minimum value of each of the side scattered light intensity and the fluorescence signal intensity was 0 ch, and the maximum value thereof was 255 ch. A time required from setting of the specimen in the automatic hematology analyzer to generation of the scattergram was three minutes or shorter in each case.

(4) Analysis and Result

FIG. 13 shows an example of the generated scattergram. Nucleated cells were counted for each of the samples measured by the analyzer, based on the optical information. The predetermined threshold value corresponding to the fluorescence signal intensity was set as 100 ch, and particles for which the fluorescence signal intensity was 100 ch or more were counted. In FIG. 13, the particles for which the fluorescence signal intensity was 100 ch or more appeared in a region surrounded by the dashed lines. The region was a region (hereinafter, also referred to as "region in which the fluorescence signal intensity was 100 ch or more") on the upper side of the two-dimensional plane divided by the fluorescence signal intensity of 100 ch in the scattergram.

The fluorescence signal intensity of 100 ch was a value which was previously determined as the predetermined threshold value. Specifically, the predetermined threshold value was determined as follows. Firstly, 20 control samples were prepared from peripheral bloods of healthy individuals (20 specimens) by using the above-described staining reagent and hemolytic reagent, and were subjected to FCM measurement by the above-described analyzer. The nucleated cells were sorted and counted based on the fluorescence signal intensity and the side scattered light intensity. Subsequently, the fluorescence signal intensity of 100 ch was set as a provisional threshold value, and cells for which the fluorescence signal intensity was 100 ch or more were counted. A ratio of the number of cells for which the fluorescence signal intensity was 100 ch or more, to the number of nucleated cells, was calculated. The median, the 10th percentile, and the 90th percentile of the ratios of the 20 control samples were calculated. As a result, the median was 3.89%, the 10th percentile was 2.52%, and the 90th percentile was 5.07%. This result indicates that the fluorescence intensity of 100 ch was a value that preferably distinguished mature leukocytes and cells in a late differentiation stage, in which an amount of nucleic acid was relatively small, from the target cells in which an amount of nucleic acid was relatively great. Therefore, the fluorescence intensity of 100 ch was determined as the predetermined threshold value. Also in FIG. 13, mature leukocytes (lymphocytes, monocytes, neutrophils, and eosinophils) in the bone marrow aspirate actually appeared in a region (hereinafter, also referred to as "region in which the fluorescence signal intensity was less than 100 ch") on the lower side of the two-dimensional plane divided by the fluorescence signal intensity of 100 ch in the scattergram. In FIG. 13, the oblique line represents a boundary between a region in which mononuclear cells appeared and a region in which polymorphonuclear cells appeared.

For each specimen, a ratio of the number of particles for which the fluorescence signal intensity was 100 ch or more to the number of nucleated cells was calculated as the first ratio. Also for the nucleated cells and the target cells counted by the microscopic examination, a ratio of the number of the target cells to the number of nucleated cells was calculated. For each specimen, the first ratio obtained by the analyzer and the ratio obtained by the microscopic examination were plotted, and a regression line ($y=0.62x+8.69$) was obtained. A correlation coefficient ($r$) was 0.88.

FIG. 14 shows the graph. As shown in FIG. 14, the first ratio obtained by the analyzer and the ratio obtained by the microscopic examination preferably correlated with each other. The specimen in which increase in the number of blast cells or plasmacytes was found tended to have a high first ratio. Particularly, it is found that, when the threshold value for the first ratio was set as 30%, specimens of acute leukemia and plasmacytoma were able to be distinguished from the other specimens. Therefore, it is indicated that, when the analyzer having the FCM counted, in bone marrow aspirate, nucleated cells and particles for which the fluorescence signal intensity indicated a predetermined threshold value or more, and obtained the first ratio, screening for acute leukemia and plasmacytoma was able to be quickly performed.

Example 2: Screening based on the number of mononuclear cells for which a fluorescence signal intensity indicated a predetermined threshold value or more

Blast cells and plasmacytes are mononuclear cells. Therefore, mononuclear cells were further sorted from the particles sorted in Example 1. Examination as to whether or not screening for a hematopoietic tumor was possible by analyzing the sorted mononuclear cells, was performed.

(1) Specimen, reagent, and analyzer

The specimens, the reagents, and the analyzer were the same as those in Example 1.

(2) Measurement data and analysis

For the number of cells in the smear, the counting result in Example 1 was used. In Example 2, the target cells in the microscopic examination were blast cells, proerythroblasts, polychromatic erythroblasts, plasmacytes, and promonocytes, which were mononuclear cells. The total number of these cells was obtained from the counting result in Example 1, and was used as the number of the target cells. As the measurement data obtained by the analyzer for each specimen, the optical information obtained in Example 1 for each specimen, was used. In FIG. 15, a region (hereinafter, also referred to as "region CDFE") surrounded by points C, D, F, and E was specified in the region in which the fluorescence signal intensity was 100 ch or more, in order to sort mononuclear cells from particles for which the fluorescence signal intensity was 100 ch or more. In FIG. 15, the region CDFE was indicated by dashed lines. The coordinates of the points C, D, E, and F were as follows when the horizontal axis representing side scattered light intensities was called X-axis and the vertical axis representing the fluorescence signal intensities was called Y-axis. The point C was (X:Y=0 ch:100 ch), the point D was (X:Y=135 ch:100 ch), the point E was (X:Y=0 ch:255 ch), and the point F was (X:Y=180 ch:255 ch). In FIG. 15, the oblique line passing through the point F and the point D represents a boundary between a region in which mononuclear cells appeared, and a region in which polymorphonuclear cells appeared. In FIG. 15, the point A represents the originating point (X:Y=0 ch:0 ch), and the point B represents an intersection (X:Y=100 ch:0 ch) of the above-described oblique line and the X-axis.

As shown in FIG. 15, the region CDFE was a region leftward of the oblique line in the region in which the fluorescence signal intensity was 100 ch or more. For each specimen, the particles appearing in the region CDFE were counted, and the number of mononuclear cells for which the fluorescence signal intensity was 100 ch or more was obtained. A ratio of the number of the mononuclear cells for which the fluorescence signal intensity was 100 ch or more to the number of the nucleated cells was calculated as the second ratio. Also for the nucleated cells and the target cells which were counted by the microscopic examination, the ratio of the number of the target cells to the number of the nucleated cells was calculated.

For each specimen, the second ratio obtained by the analyzer and the ratio obtained by the microscopic examination were plotted, and a regression line (y=0.64x+5.07) was obtained. A correlation coefficient (r) was 0.95. FIG. 16 shows the graph. As shown in FIG. 16, the second ratio obtained by the analyzer and the ratio obtained by the microscopic examination preferably correlated with each other. The specimen in which increase in the number of blast cells or plasmacytes was found tended to have a high second ratio. Particularly, it is found that, when the threshold value for the second ratio was set as 20%, specimens of acute leukemia and plasmacytoma were able to be distinguished from the other specimens. Therefore, it is indicated that, when the analyzer having the FCM counted, in bone marrow aspirate, nucleated cells and mononuclear cells for which the fluorescence signal intensity indicated a predetermined threshold value or more, and obtained the second ratio, screening for acute leukemia and plasmacytoma was able to be quickly performed.

Example 3: Screening based on the number of blast cells

In acute leukemia, neoplastic blast cells having no differentiation potential proliferate. Therefore, blast cells were further sorted from the particles sorted in Example 1. Examination as to whether or not screening for acute leukemia was

possible by analyzing the sorted blast cells, was performed.

(1) Specimen, reagent, and analyzer

The specimens, the reagents, and the analyzer were the same as those in Example 1.

(2) Measurement data and analysis

For the number of cells in the smear, the counting result in Example 1 was used. In Example 3, the target cells in the microscopic examination were blast cells. However, benign blast cells and malignant blast cells were not distinguished from each other. According to the counting result in Example 1, the number of blast cells was used as the number of the target cells. As the measurement data obtained by the analyzer for each specimen, the optical information obtained in Example 1 for each specimen was used. In FIG. 17, a region surrounded by points H, D, F, and I was specified in the region in which the fluorescence signal intensity was 100 ch or more, in order to sort blast cells from particles for which the fluorescence signal intensity was 100 ch or more. A region surrounded by points D, G, and H was specified in the scattergram in FIG. 17 in order to more accurately count blast cells. Therefore, a region (hereinafter, also referred to as "region HGFI") surrounded by the points H, G, F, and I was specified as a region in which blast cells appeared, in the scattergram. The coordinates of the points D, F, G, H, and I were as follows. The point D was (X:Y=135 ch:100 ch), the point F was (X:Y=180 ch:255 ch), the point G was (X:Y=130 ch:80 ch), the point H was (X:Y=80 ch:100 ch), and the point I was (X:Y=130 ch:255 ch).

As shown in FIG. 17, the region HGFI was a region in which blast cells appeared in the scattergram based on the fluorescence signal intensity and the side scattered light intensity. For each specimen, particles appearing in the region HGFI were counted. In FIG. 18, particles for which the forward scattered light intensity was 50 ch or more and 100 ch or less were sorted in particles appearing in the region HGFI in order to more accurately count the blast cells. The number of the sorted particles was obtained as the number of blast cells. A ratio of the number of the blast cells to the number of the nucleated cells was calculated as the third ratio. Also for the nucleated cells and the blast cells which were counted by the microscopic examination, a ratio of the number of the blast cells to the number of the nucleated cells was calculated.

For each specimen, the third ratio obtained by the analyzer, and the ratio obtained by the microscopic examination were plotted, and a regression line (y=0.60x+5.26) was obtained. A correlation coefficient (r) was 0.921. FIG. 19 shows the graph. As shown in FIG. 19, the third ratio obtained by the analyzer and the ratio obtained by the microscopic examination preferably correlated with each other. A specimen in which the ratio of the number of the blast cells to the number of the nucleated cells was 10% or more tended to have a high third ratio. Particularly, it is found that, when the threshold value for the third ratio was set as 20%, specimens of acute leukemia were able to be distinguished from the other specimens. Therefore, it is indicated that, when the analyzer having the FCM counted nucleated cells and blast cells in bone marrow aspirate, and obtained the third ratio, screening for acute leukemia was able to be quickly performed.

Example 4: Indication of appearance of neoplastic cells

Performance of the analyzer for determining a specimen suspected of a hematopoietic tumor was examined by comparison with determination of the specimen by microscopic examination.

(1) Specimen

Bone marrow aspirates (38 cases in total), in the following cases, which were checked by microscopic examination in a hospital were used as specimens.

- acute leukemia (a ratio of the number of blast cells to the number of nucleated cells was 20% or more): 2 cases
- plasmacytoma (a ratio of the number of plasmacytes to the number of nucleated cells was 10% or more): one case
- moderate level increase of blast cells (a ratio of the number of blast cells to the number of nucleated cells was 10% or more and less than 20%): one case
- mild level increase of blast cells (a ratio of the number of blast cells to the number of nucleated cells was 5% or more and less than 10%): three cases
- metastasis of mature lymphoma to bone marrow (a ratio of the number of mature lymphoma cells to the number of nucleated cells was 5% or more): five cases
- cases in which increase in the numbers of blast cells and plasmacytes was not found: 26 cases

(2) Reagent, analyzer, and measurement

The reagents and the analyzer were the same as those in Example 1. By using the reagents and the analyzer, measurement by the analyzer and microscopic examination were performed for the above-described specimens in the same manner as in Example 1.

(3) Analysis and result

(3.1) Determination of specimen in which the number of blast cells was increased

The smear of each specimen was observed by a microscope, and the number of nucleated cells and the number of blast cells were obtained. A ratio of the number of the blast cells to the number of the nucleated cells was calculated. A specimen in which the calculated ratio was 10% or more was determined as a specimen suspected of having the increased number of blast cells. The number of nucleated cells and the number of blast cells were obtained through the same analysis as in Example 3 based on the optical information obtained by the analyzer for each specimen. The third ratio was calculated. A specimen in which the third ratio was 10% or more was determined as a specimen suspected of having the increased number of blast cells. Table 1 indicates the result of comparison between the determination by the analyzer and the determination by the microscopic examination. In Table 1, "Positive" represents a specimen suspected of having the increased number of blast cells, and "Negative" represents a specimen in which the increase in the number of blast cells was not found.

[Table 1]

| Concordance rate: 84.2% | | Microscopic examination | |
|---|---|---|---|
| | | Positive | Negative |
| Analyzer | Positive | 3 | 6 |
| | Negative | 0 | 29 |

As indicated in Table 1, the concordance rate between the result of the determination by the analyzer and the result of the determination by the microscopic examination was 84.2%. In the determination by the analyzer, the sensitivity was 100% and the specificity was 82.9%. Thus, the concordance rate between the result of the determination by the analyzer and the result of the determination by the microscopic examination was high, and this indicates that performance of the analyzer for determining a specimen in which the number of blast cells was increased was preferable. When the determination result indicates that the third ratio of the specimen is 10% or more, the analyzer may output a remark such as "Blastosis?" onto the screen for displaying an analysis result of the specimen. Thus, the screening index for acute leukemia can be provided to the user.

(3.2) Determination of mature lymphoma specimen

The smear of each specimen was observed by a microscope, and the number of nucleated cells and the number of mature lymphoma cells were obtained. A ratio of the number of the mature lymphoma cells to the number of the nucleated cells was calculated. A specimen in which the calculated ratio was 10% or more was determined as a specimen suspected of mature lymphoma. The number of nucleated cells and the number of mononuclear cells for which the fluorescence signal intensity was 100 ch or more were obtained through the same analysis as in Example 2 based on the optical information obtained by the analyzer for each specimen. The second ratio was calculated. Furthermore, the number of mononuclear cells for which the fluorescence signal intensity was less than 100 ch was obtained based on the optical information of each specimen. The mononuclear cells for which the fluorescence signal intensity was less than a predetermined threshold value appeared in a region (hereinafter, also referred to as "region ABDC") surrounded by points A, B, D, and C in a scattergram of each specimen in FIG. 15. A ratio of the number of the mononuclear cells for which the fluorescence signal intensity was less than 100 ch, to the number of the nucleated cells, was calculated as the fourth ratio. The fifth ratio was calculated according to the above-described Equation (E).

A specimen in which the fifth ratio was less than 10% was determined as a specimen suspected of mature lymphoma. Table 2 indicates a result of comparison between the determination by the analyzer and the determination by the microscopic examination. In Table 2, "Positive" represents a specimen suspected of mature lymphoma, and "Negative" represents a specimen determined to have no mature lymphoma.

[Table 2]

| Concordance rate: 92.1% | | Microscopic examination | |
|---|---|---|---|
| | | Positive | Negative |
| Analyzer | Positive | 3 | 2 |
| | Negative | 1 | 32 |

As indicated in Table 2, the concordance rate between the result of the determination by the analyzer and the result of the determination by the microscopic examination was 92.1%. In the determination by the analyzer, the sensitivity was 75.0% and the specificity was 94.1%. Thus, the concordance rate between the result of the determination by the analyzer and the result of the determination by the microscopic examination was high, and this indicates that performance of the analyzer for determining a mature lymphoma specimen was preferable. When the determination result indicates that the fifth ratio of the specimen is less than 10%, the analyzer may output a remark such as "Mature Lymphocytosis?" onto the screen for displaying an analysis result of the specimen. Thus, the screening index for mature lymphoma can be provided to the user.

(3.3) Determination of plasmacytoma specimen

The smear of each specimen was observed by a microscope, and the number of nucleated cells and the number of plasmacytes were obtained. A ratio of the number of the plasmacytes to the number of the nucleated cells was calculated. A specimen in which the calculated ratio was 10% or more was determined as a specimen suspected of plasmacytoma. A scattergram which had the horizontal axis representing side scattered light intensities, and the vertical axis representing fluorescence signal intensities was generated based on the optical information obtained by the analyzer for each specimen. In the scattergram, the minimum value of the fluorescence signal intensity was 0 ch, and the maximum value thereof was 1023 ch. The minimum value of the side scattered light intensity was 0 ch, and the maximum value thereof was 255 ch. FIG. 20 shows an example of the generated scattergram. In FIG. 20, plasmacytes appeared in a region surrounded by an ellipse. A region (hereinafter, also referred to as "region JKLM") surrounded by points J, K, L, and M was specified in order to specify plasmacytes in FIG. 20. In FIG. 20, the region JKLM was indicated by dashed lines. The coordinates of the points J, K, L, and M were as follows when the horizontal axis representing side scattered light intensities was called X-axis and the vertical axis representing fluorescence signal intensities was called Y-axis. The point J was (X:Y=0 ch: 1023 ch), the point K was (X:Y=170 ch:1023 ch), the point L was (X:Y=170 ch:296 ch), and the point M was (X:Y=0 ch:296 ch).

In FIG. 20, the region JKLM was a region including plasmacytes in the scattergram based on the fluorescence signal intensity and the side scattered light intensity. For each specimen, particles appearing in the region JKLM were counted as the number of plasmacytes. A ratio of the number of plasmacytes to the number of nucleated cells was calculated as the sixth ratio. A specimen in which the sixth ratio was 5% or more was determined as a specimen suspected of plasmacytoma. Table 3 indicates a result of comparison between the determination by the analyzer and the determination by the microscopic examination. In Table 3, "Positive" represents a specimen suspected of plasmacytoma, and "Negative" represents a specimen determined to have no plasmacytoma.

[Table 3]

| Concordance rate: 100% | | Microscopic examination | |
|---|---|---|---|
| | | Positive | Negative |
| Analyzer | Positive | 1 | 0 |
| | Negative | 0 | 37 |

As indicated in Table 3, the concordance rate between the result of the determination by the analyzer and the result of the determination by the microscopic examination was 100%. Both the sensitivity and the specificity in the determination by the analyzer were each 100%. Thus, the concordance rate between the result of the determination by the analyzer and the result of the determination by the microscopic examination was high, and this indicates that performance of the analyzer for determining a plasmacytoma specimen was preferable. When the determination result indicates that the sixth ratio of the specimen is 10% or more, the analyzer may output a remark such as "Plasmacytosis?" onto the screen for displaying an analysis result of the specimen. Thus, the screening index for plasmacytoma can be provided to the user.

According to the examples, a determination result that highly correlates with that of visual examination can be

obtained within three minutes after a specimen is set in the device. Therefore, screening for a hematopoietic tumor, which has been conventionally performed by visual examination, can be automatically and quickly performed. For example, an acute myeloid leukemia patient who is to be treated early can be specified, and work-up can be performed quickly thereafter.

DESCRIPTION OF THE REFERENCE CHARACTERS

| | |
|---|---|
| 10 | sample analyzer |
| 20 | measurement unit |
| 30 | analysis unit |
| 40 | suction part |
| 50 | sample preparation part |
| 51, 52, and 53 | reagent container |
| 54 | reaction chamber |
| 60 | detector |
| 61 | flow cell |
| 62 | light source part |
| 63, 64, and 65 | light receiver |
| 66 | dichroic mirror |
| 300 | body |
| 309 | input part |
| 310 | display part |
| 321 | portable storage medium |

**Claims**

1. A bone marrow aspirate analysis method comprising:

   measuring a sample containing bone marrow aspirate and a fluorescent dye that can stain nucleic acid, by flow cytometry, and obtaining optical information including fluorescence signal information for particles in the sample and **characterized by**:

      counting particles for which the fluorescence signal information indicates a threshold value or more, as target cells; and
      obtaining a screening index for a hematopoietic tumor based on a number of the target cells.

2. The analysis method of claim 1, wherein the optical information further includes at least one of side scattered light information and forward scattered light information.

3. The analysis method of claim 1 or 2, wherein the obtaining the number of target cells further comprises obtaining a number of nucleated cells based on the optical information.

4. The analysis method according to any one of claims 1 to 3, wherein the obtaining the index comprises obtaining a first ratio as a ratio of the number of the target cells to the number of the nucleated cells.

5. The analysis method according to any one of claims 1 to 4, further comprising providing distinguishable display when the first ratio indicates a threshold value for the first ratio or more.

6. The analysis method according to any one of claims 1 to 5, wherein the threshold value for the first ratio is any value between 20 and 40%, optionally, wherein the threshold value for the first ratio is 30%.

7. The analysis method according to any one of claims 1 to 6, wherein the threshold value corresponding to the fluorescence signal information is a value that allows 95% or more of mature leukocytes to be excluded.

8. The analysis method according to any one of claims 2 to 7, wherein the threshold value corresponding to the fluorescence signal information is represented by a straight line that intersects a monocyte population in the sample

and does not intersect a lymphocyte population in the sample when a distribution of particles in the sample is rendered on a two-dimensional plane based on the fluorescence signal information and the side scattered light information.

**9.** The analysis method according to any one of claims 1 to 8, wherein the threshold value corresponding to the fluorescence signal information represents a value satisfying the following condition:

when a plurality of control samples prepared from the fluorescent dye and peripheral bloods, of healthy individuals, obtained from at least 20 specimens are measured by flow cytometry, and the fluorescence signal information and side scattered light information for the plurality of control samples are obtained,
a number of nucleated cells in each of the plurality of control samples is obtained based on the fluorescence signal information and the side scattered light information,
any fluorescence signal intensity is set as a provisional threshold value, and a number of cells for which a fluorescence signal intensity indicates the provisional threshold value or more is obtained,
a ratio of the number of the cells for which the fluorescence signal intensity indicates the provisional threshold value or more, to a number of nucleated cells, is calculated, and
a median of the ratios of the plurality of control samples is calculated,
the median is 2.5% or more and 5% or less.

**10.** The analysis method according to any one of claims 3-9, wherein

the optical information includes the fluorescence signal information and the side scattered light information,
the counting the target cells comprises counting mononuclear cells for which the fluorescence signal information indicates the threshold value or more, and
the obtaining the index comprises obtaining a second ratio as a ratio of a number of the mononuclear cells to the number of nucleated cells.

**11.** The analysis method of claim 10, further comprising providing distinguishable display when the second ratio indicates a threshold value for the second ratio or more.

**12.** The analysis method of claim 11, wherein the threshold value for the second ratio is any value between 10% and 30, optionally, wherein the threshold value for the second ratio is 20%.

**13.** The analysis method according to any one of claims 3-12, wherein

the optical information includes the fluorescence signal information, the side scattered light information, and the forward scattered light information,
the counting the target cells comprises counting blast cells, and
the obtaining the index comprises obtaining a third ratio as a ratio of a number of the blast cells to the number of nucleated cells.

**14.** The analysis method of claim 13, further comprising providing distinguishable display when the third ratio indicates a threshold value for the third ratio or more.

**15.** The analysis method of claim 14, wherein the threshold value for the third ratio is any value between 5% and 25%, optionally, wherein the threshold value for the third ratio is 10% or 20%.

**16.** The analysis method according to any one of claims 10 to 15, wherein

the obtaining the number of target cells comprises counting mononuclear cells for which the fluorescence signal information indicates a value less than the threshold value, and
the obtaining the index comprises obtaining a fourth ratio as a ratio of a number of the mononuclear cells to the number of nucleated cells, and obtaining a fifth ratio as a ratio of a value of the second ratio to a value of the fourth ratio.

**17.** The analysis method of claim 16, further comprising providing distinguishable display when the fifth ratio indicates a threshold value for the fifth ratio or less.

**18.** The analysis method of claim 17, wherein the threshold value for the fifth ratio is any value between 5% and 15%, optionally, wherein the threshold value for the fifth ratio is 10%.

**19.** The analysis method according to any one of claims 3 to 18, wherein

the optical information includes the fluorescence signal information and the side scattered light information,
the counting the target cells comprises counting plasmacytes, and
the obtaining the index comprises obtaining a sixth ratio as a ratio of a number of the plasmacytes to the number of nucleated cells.

**20.** The analysis method of claim 19, further comprising providing distinguishable display when the sixth ratio indicates a threshold value for the sixth ratio or more.

**21.** The analysis method of claim 20, wherein the threshold value for the sixth ratio is any value between 5% and 15%, optionally, wherein the threshold value for the sixth ratio is 5%.

**22.** The analysis method according to any one of claims 5 to 21, wherein the distinguishable display indicates that the bone marrow aspirate is a specimen that needs to be preferentially tested for a hematopoietic tumor.

**23.** The analysis method according to any one of claims 1 to 22, wherein the sample further contains a cationic surfactant, optionally, wherein the sample is prepared by mixing bone marrow aspirate, a staining reagent containing the fluorescent dye, and a hemolytic reagent containing a cationic surfactant.

**24.** A sample analyzer (10) comprising;

a sample preparation unit (50) configured to prepare a sample containing bone marrow aspirate, and a fluorescent dye that can stain nucleic acid;
a detector (60) configured to obtain optical information including fluorescence signal information for particles in the sample;
**characterized in that** it further includes:

a controller configured to count particles for which the fluorescence signal information indicates a threshold value or more, as target cells, wherein
the controller obtains a screening index for a hematopoietic tumor based on a number of the target cells.

**25.** The sample analyzer of claim 24, wherein

the detector comprises
a flow cell (61) through which the sample prepared by the sample preparation unit flows,
a light source (62) configured to apply light to a particle in the sample flowing in the flow cell, and
a light receiver (63,64,65) configured to obtain the optical information when light is applied to the particle.

**26.** A computer program for analyzing bone marrow aspirate, the computer program causing a computer to execute:

obtaining optical information including fluorescence signal information, obtained by flow cytometry, for particles in a sample containing the bone marrow aspirate and a fluorescent dye that can stain nucleic acid;
and **characterized in that** it further causes the computer to execute:

counting particles for which the fluorescence signal information indicates a threshold value or more, as target cells, based on the optical information; and
obtaining a screening index for a hematopoietic tumor based on a number of the target cells.

## Patentansprüche

**1.** Verfahren zur Analyse von Knochenmarkaspirat, umfassend:
Messen einer Probe, die Knochenmarkaspirat und einen fluoreszierenden Farbstoff beinhaltet, der Nukleinsäure färben kann, mittels Durchflusszytometrie, und Erhalten optischer Informationen, die Fluoreszenzsignalinformationen für Partikel in der Probe einschließen, und **gekennzeichnet durch**:

Zählen von Partikeln, für die die Fluoreszenzsignalinformationen einen Schwellenwert oder mehr anzeigen, als Zielzellen; und
Erhalten eines Screening-Index für einen hämatopoetischen Tumor basierend auf einer Anzahl der Zielzellen.

**2.** Analyseverfahren nach Anspruch 1, wobei die optischen Informationen weiter mindestens eines von seitlich gestreuten Lichtinformationen und vorwärts gestreuten Lichtinformationen einschließen.

**3.** Analyseverfahren nach Anspruch 1 oder 2, wobei das Erhalten der Anzahl der Zielzellen weiter das Erhalten einer Anzahl nukleierter Zellen, basierend auf den optischen Informationen, umfasst.

**4.** Analyseverfahren nach einem der Ansprüche 1 bis 3, wobei das Erhalten des Index das Erhalten eines ersten Verhältnisses als Verhältnis der Anzahl der Zielzellen zu der Anzahl der nukleierten Zellen umfasst.

**5.** Analyseverfahren nach einem der Ansprüche 1 bis 4, weiter umfassend das Bereitstellen einer unterscheidbaren Anzeige, wenn das erste Verhältnis einen Schwellenwert für das erste Verhältnis oder mehr anzeigt.

**6.** Analyseverfahren nach einem der Ansprüche 1 bis 5, wobei der Schwellenwert für das erste Verhältnis jeder Wert zwischen 20 und 40 % ist, wobei der Schwellenwert für das erste Verhältnis optional 30 % beträgt.

**7.** Analyseverfahren nach einem der Ansprüche 1 bis 6, wobei der Schwellenwert, der den Fluoreszenzsignalinformationen entspricht, ein Wert ist, der es ermöglicht, 95 % oder mehr der reifen Leukozyten auszuschließen.

**8.** Analyseverfahren nach einem der Ansprüche 2 bis 7, wobei der Schwellenwert, der den Fluoreszenzsignalinformationen entspricht, durch eine gerade Linie dargestellt wird, die eine Monozytenpopulation in der Probe schneidet und eine Lymphozytenpopulation in der Probe nicht schneidet, wenn eine Verteilung von Partikeln in der Probe auf einer zweidimensionalen Ebene basierend auf den Fluoreszenzsignalinformationen und den seitlich gestreuten Lichtinformationen dargestellt wird.

**9.** Analyseverfahren nach einem der Ansprüche 1 bis 8, wobei
der Schwellenwert, der den Fluoreszenzsignalinformationen entspricht, einen Wert darstellt, der die folgende Bedingung erfüllt:

wenn eine Vielzahl von Kontrollproben, die aus dem fluoreszierenden Farbstoff und peripherem Blut gesunder Personen vorbereitet und aus mindestens 20 Proben gewonnen wurden, mittels Durchflusszytometrie gemessen werden und die Fluoreszenzsignalinformationen und die seitlich gestreuten Lichtinformationen für die Vielzahl der Kontrollproben erhalten werden,
wird eine Anzahl von nukleierten Zellen in jeder der Vielzahl von Kontrollproben basierend auf den Fluoreszenzsignalinformationen und den seitlich gestreuten Lichtinformationen erhalten,
wird eine beliebige Fluoreszenzsignalintensität als vorläufiger Schwellenwert festgelegt und wird eine Anzahl von Zellen, für die eine Fluoreszenzsignalintensität den vorläufigen Schwellenwert oder mehr anzeigt, erhalten,
wird ein Verhältnis der Anzahl der Zellen, für die die Fluoreszenzsignalintensität den vorläufigen Schwellenwert oder mehr anzeigt, zu einer Anzahl nukleierter Zellen berechnet, und
wird ein Mittelwert der Verhältnisse der Vielzahl von Kontrollproben berechnet,
beträgt der Mittelwert 2,5 % oder mehr und 5 % oder weniger.

**10.** Analyseverfahren nach einem der Ansprüche 3-9, wobei

die optischen Informationen die Fluoreszenzsignalinformationen und die seitlich gestreuten Lichtinformationen einschließen,
das Zählen der Zielzellen das Zählen mononukleärer Zellen umfasst, für die die Fluoreszenzsignalinformationen den Schwellenwert oder mehr anzeigen, und
das Erhalten des Index das Erhalten eines zweiten Verhältnisses als Verhältnis einer Anzahl der mononukleären Zellen zu der Anzahl der nukleierten Zellen umfasst.

**11.** Analyseverfahren nach Anspruch 10, weiter umfassend das Bereitstellen einer unterscheidbaren Anzeige, wenn das zweite Verhältnis einen Schwellenwert für das zweite Verhältnis oder mehr anzeigt.

**12.** Analyseverfahren nach Anspruch 11, wobei der Schwellenwert für das zweite Verhältnis jeder Wert zwischen 10 % und 30 ist, wobei der Schwellenwert für das zweite Verhältnis optional 20 % beträgt.

**13.** Analyseverfahren nach einem der Ansprüche 3-12, wobei

die optischen Informationen die Fluoreszenzsignalinformationen, die seitlich gestreuten Lichtinformationen und die vorwärts gestreuten Lichtinformationen einschließen,
das Zählen der Zielzellen das Zählen von Blastenzellen umfasst, und
das Erhalten des Index das Erhalten eines dritten Verhältnisses als Verhältnis einer Anzahl der Blastenzellen zu der Anzahl der nukleierten Zellen umfasst.

**14.** Analyseverfahren nach Anspruch 13, weiter umfassend das Bereitstellen einer unterscheidbaren Anzeige, wenn das dritte Verhältnis einen Schwellenwert für das dritte Verhältnis oder mehr anzeigt.

**15.** Analyseverfahren nach Anspruch 14, wobei der Schwellenwert für das dritte Verhältnis jeder Wert zwischen 5 % und 25 % ist, wobei der Schwellenwert für das dritte Verhältnis optional 10 % oder 20 % beträgt.

**16.** Analyseverfahren nach einem der Ansprüche 10 bis 15, wobei

das Erhalten der Anzahl der Zielzellen das Zählen mononukleärer Zellen umfasst, für die die Fluoreszenzsignalinformation einen Wert anzeigen, der kleiner als der Schwellenwert ist, und
das Erhalten des Index das Erhalten eines vierten Verhältnisses als Verhältnis einer Anzahl der mononukleären Zellen zu der Anzahl der nukleierten Zellen und das Erhalten eines fünften Verhältnisses als Verhältnis eines Wertes des zweiten Verhältnisses zu einem Wert des vierten Verhältnisses umfasst.

**17.** Analyseverfahren nach Anspruch 16, weiter umfassend das Bereitstellen einer unterscheidbaren Anzeige, wenn das fünfte Verhältnis einen Schwellenwert für das fünfte Verhältnis oder weniger anzeigt.

**18.** Analyseverfahren nach Anspruch 17, wobei der Schwellenwert für das fünfte Verhältnis jeder Wert zwischen 5 % und 15 % ist, wobei der Schwellenwert für das fünfte Verhältnis optional 10 % beträgt.

**19.** Analyseverfahren nach einem der Ansprüche 3 bis 18, wobei

die optischen Informationen die Fluoreszenzsignalinformationen und die seitlich gestreuten Lichtinformationen einschließen,
das Zählen der Zielzellen das Zählen von Plasmazyten umfasst, und
das Erhalten des Index das Erhalten eines sechsten Verhältnisses als Verhältnis einer Anzahl der Plasmazellen zu der Anzahl der nukleierten Zellen umfasst.

**20.** Analyseverfahren nach Anspruch 19, weiter umfassend das Bereitstellen einer unterscheidbaren Anzeige, wenn das sechste Verhältnis einen Schwellenwert für das sechste Verhältnis oder mehr anzeigt.

**21.** Analyseverfahren nach Anspruch 20, wobei der Schwellenwert für das sechste Verhältnis jeder Wert zwischen 5 % und 15 % ist, wobei der Schwellenwert für das sechste Verhältnis optional 5 % beträgt.

**22.** Analyseverfahren nach einem der Ansprüche 5 bis 21, wobei die unterscheidbare Anzeige anzeigt, dass das Knochenmarkaspirat eine Probe ist, die vorzugsweise auf einen hämatopoetischen Tumor getestet werden muss.

**23.** Analyseverfahren nach einem der Ansprüche 1 bis 22, wobei die Probe weiter ein kationisches Tensid beinhaltet, wobei die Probe optional durch Mischen von Knochenmarkaspirat, einem Färbereagenz, das den fluoreszierenden Farbstoff beinhaltet, und einem hämolytischen Reagenz, das ein kationisches Tensid beinhaltet, vorbereitet wird.

**24.** Probenanalysator (10), umfassend:

eine Probenvorbereitungseinheit (50), die so eingerichtet ist, dass sie eine Probe vorbereitet, die Knochenmarkaspirat und einen fluoreszierenden Farbstoff beinhaltet, der Nukleinsäure färben kann;
einen Detektor (60), der so eingerichtet ist, dass er optische Informationen einschließlich Fluoreszenzsignalinformationen für Partikel in der Probe erhält;

**dadurch gekennzeichnet, dass** er weiter Folgendes einschließt:

eine Steuerung, die so eingerichtet ist, dass sie Partikel, für die die Fluoreszenzsignalinformationen einen Schwellenwert oder mehr anzeigen, als Zielzellen zählt, wobei
die Steuerung einen Screening-Index für einen hämatopoetischen Tumor basierend auf einer Anzahl der Zielzellen erhält.

**25.** Probenanalysator nach Anspruch 24, wobei

der Detektor eine Durchflusszelle (61) umfasst, durch die die von der Probenvorbereitungseinheit vorbereitete Probe fließt,
eine Lichtquelle (62), die so eingerichtet ist, dass sie Licht auf ein Partikel in der Probe aufbringt, das in der Durchflusszelle fließt, und
einen Lichtempfänger (63, 64, 65), der so eingerichtet ist, dass er die optischen Informationen erhält, wenn Licht auf das Partikel aufgebracht wird.

**26.** Computerprogramm zum Analysieren von Knochenmarkaspiraten, wobei das Computerprogramm einen Computer dazu veranlasst, Folgendes auszuführen:

Erhalten optischer Informationen einschließlich Fluoreszenzsignalinformationen, die durch Durchflusszytometrie für Partikel in einer Probe erhalten werden, die das Knochenmarkaspirat und einen fluoreszierenden Farbstoff beinhaltet, der Nukleinsäure färben kann;
und **dadurch gekennzeichnet, dass** es den Computer weiter dazu veranlasst, Folgendes auszuführen:

Zählen von Partikeln, für die die Fluoreszenzsignalinformationen einen Schwellenwert oder mehr anzeigen, als Zielzellen basierend auf den optischen Informationen; und
Erhalten eines Screening-Index für einen hämatopoetischen Tumor basierend auf einer Anzahl der Zielzellen.

## Revendications

**1.** Procédé d'analyse d'aspirat de moelle osseuse comprenant :
la mesure d'un échantillon contenant de l'aspirat de moelle osseuse et un colorant fluorescent qui peut marquer un acide nucléique, par cytométrie en flux, et l'obtention d'informations optiques incluant des informations de signal de fluorescence pour des particules dans l'échantillon et **caractérisé par** :

le comptage de particules pour lesquelles les informations de signal de fluorescence indiquent une valeur seuil ou plus, en tant que cellules cibles ; et
l'obtention d'un indice de criblage d'une tumeur hématopoïétique sur la base d'un nombre des cellules cibles.

**2.** Procédé d'analyse selon la revendication 1, dans lequel les informations optiques incluent en outre au moins l'une parmi des informations de lumière diffusée latéralement et des informations de lumière diffusée vers l'avant.

**3.** Procédé d'analyse selon la revendication 1 ou la revendication 2, dans lequel l'obtention du nombre de cellules cibles comprend en outre l'obtention d'un nombre de cellules nucléées sur la base des informations optiques.

**4.** Procédé d'analyse selon l'une quelconque des revendications 1 à 3, dans lequel l'obtention de l'indice comprend l'obtention d'un premier rapport en tant que rapport entre le nombre des cellules cibles et le nombre des cellules nucléées.

**5.** Procédé d'analyse selon l'une quelconque des revendications 1 à 4, comprenant en outre
la fourniture d'un affichage distinctif lorsque le premier rapport indique une valeur seuil pour le premier rapport ou plus.

**6.** Procédé d'analyse selon l'une quelconque des revendications 1 à 5, dans lequel la valeur seuil pour le premier rapport est toute valeur comprise entre 20 et 40 %, facultativement, dans lequel la valeur seuil pour le premier rapport est 30 %.

7. Procédé d'analyse selon l'une quelconque des revendications 1 à 6, dans lequel la valeur seuil correspondant aux informations de signal de fluorescence est une valeur qui permet d'exclure 95 % ou plus de leucocytes matures.

8. Procédé d'analyse selon l'une quelconque des revendications 2 à 7, dans lequel la valeur seuil correspondant aux informations de signal de fluorescence est représentée par une ligne droite qui croise une population de monocytes dans l'échantillon et ne croise pas une population de lymphocytes dans l'échantillon lorsqu'une distribution de particules dans l'échantillon est restituée sur un plan bidimensionnel sur la base des informations de signal de fluorescence et des informations de lumière diffusée latéralement.

9. Procédé d'analyse selon l'une quelconque des revendications 1 à 8, dans lequel
la valeur seuil correspondant aux informations de signal de fluorescence représente une valeur satisfaisant à la condition suivante :

lorsqu'une pluralité d'échantillons témoins préparés à partir du colorant fluorescent et
de sangs périphériques, d'individus sains, obtenus auprès d'au moins 20 spécimens sont mesurés par cytométrie en flux, et les informations de signal de fluorescence et les informations de lumière diffusée latéralement pour la pluralité d'échantillons témoins sont obtenues,
un nombre de cellules nucléées dans chacun de la pluralité d'échantillons témoins est obtenu sur la base des informations de signal de fluorescence et des informations de lumière diffusée latéralement,
toute intensité de signal de fluorescence est établie comme valeur seuil provisoire, et un nombre de cellules pour lesquelles une intensité de signal de fluorescence indique la valeur seuil provisoire ou plus est obtenu,
un rapport entre le nombre de cellules pour lesquelles l'intensité du signal de fluorescence indique la valeur seuil provisoire ou plus, et un nombre de cellules nucléées, est calculé, et
une médiane des rapports de la pluralité d'échantillons témoins est calculée,
la médiane est de 2,5 % ou plus et de 5 % ou moins.

10. Procédé d'analyse selon l'une quelconque des revendications 3 à 9, dans lequel

les informations optiques incluent les informations de signal de fluorescence et les informations de lumière diffusée latéralement,
le comptage des cellules cibles comprend le comptage de cellules mononucléaires pour lesquelles les informations de signal de fluorescence indiquent la valeur seuil ou plus, et
l'obtention de l'indice comprend l'obtention d'un deuxième rapport en tant que rapport entre un nombre des cellules mononucléaires et le nombre de cellules nucléées.

11. Procédé d'analyse selon la revendication 10, comprenant en outre la fourniture d'un affichage distinctif lorsque le deuxième rapport indique une valeur seuil pour le deuxième rapport ou plus.

12. Procédé d'analyse selon la revendication 11, dans lequel la valeur seuil pour le deuxième rapport est toute valeur comprise entre 10 % et 30 %, facultativement, dans lequel la valeur seuil pour le deuxième rapport est 20 %.

13. Procédé d'analyse selon l'une quelconque des revendications 3 à 12, dans lequel

les informations optiques incluent les informations de signal de fluorescence, les informations de lumière diffusée latéralement et les informations de lumière diffusée vers l'avant,
le comptage des cellules cibles comprend le comptage de cellules blastiques, et
l'obtention de l'indice comprend l'obtention d'un troisième rapport en tant que rapport entre un nombre des cellules blastiques et le nombre de cellules nucléées.

14. Procédé d'analyse selon la revendication 13, comprenant en outre la fourniture d'un affichage distinctif lorsque le troisième rapport indique une valeur seuil pour le troisième rapport ou plus.

15. Procédé d'analyse selon la revendication 14, dans lequel la valeur seuil pour le troisième rapport est toute valeur comprise entre 5 % et 25 %, facultativement, dans lequel la valeur seuil pour le troisième rapport est 10 % ou 20 %.

16. Procédé d'analyse selon l'une quelconque des revendications 10 à 15, dans lequel

l'obtention du nombre de cellules cibles comprend le comptage de cellules mononucléaires pour lesquelles les

informations de signal de fluorescence indiquent une valeur inférieure à la valeur seuil, et
l'obtention de l'indice comprend l'obtention d'un quatrième rapport en tant que rapport entre un nombre des cellules mononucléaires et le nombre de cellules nucléées, et l'obtention d'un cinquième rapport en tant que rapport entre une valeur du deuxième rapport et une valeur du quatrième rapport.

**17.** Procédé d'analyse selon la revendication 16, comprenant en outre la fourniture d'un affichage distinctif lorsque le cinquième rapport indique une valeur seuil pour le cinquième rapport ou moins.

**18.** Procédé d'analyse selon la revendication 17, dans lequel la valeur seuil pour le cinquième rapport est toute valeur comprise entre 5 % et 15 %, facultativement, dans lequel la valeur seuil pour le cinquième rapport est 10 %.

**19.** Procédé d'analyse selon l'une quelconque des revendications 3 à 18, dans lequel

les informations optiques incluent les informations de signal de fluorescence et les informations de lumière diffusée latéralement,
le comptage des cellules cibles comprend le comptage de plasmocytes, et
l'obtention de l'indice comprend l'obtention d'un sixième rapport en tant que rapport entre un nombre des plasmocytes et le nombre de cellules nucléées.

**20.** Procédé d'analyse selon la revendication 19, comprenant en outre la fourniture d'un affichage distinctif lorsque le sixième rapport indique une valeur seuil pour le sixième rapport ou plus.

**21.** Procédé d'analyse selon la revendication 20, dans lequel la valeur seuil pour le sixième rapport est toute valeur comprise entre 5 % et 15 %, facultativement, dans lequel la valeur seuil pour le sixième rapport est 5 %.

**22.** Procédé d'analyse selon l'une quelconque des revendications 5 à 21, dans lequel l'affichage distinctif indique que l'aspirat de moelle osseuse est un spécimen qui doit être testé de préférence quant à une tumeur hématopoïétique.

**23.** Procédé d'analyse selon l'une quelconque des revendications 1 à 22, dans lequel l'échantillon contient en outre un tensioactif cationique, facultativement, dans lequel l'échantillon est préparé en mélangeant de l'aspirat de moelle osseuse, un réactif de coloration contenant le colorant fluorescent et un réactif hémolytique contenant un tensioactif cationique.

**24.** Analyseur (10) d'échantillon comprenant ;

une unité (50) de préparation d'échantillon configurée pour préparer un échantillon contenant de l'aspirat de moelle osseuse, et un colorant fluorescent qui peut marquer un acide nucléique ;
un détecteur (60) configuré pour obtenir des informations optiques incluant des informations de signal de fluorescence pour des particules dans l'échantillon ; **caractérisé en ce qu'**il inclut en outre :
un dispositif de commande configuré pour compter des particules pour lesquelles les informations de signal de fluorescence indiquent une valeur seuil ou plus, en tant que cellules cibles, dans lequel
le dispositif de commande obtient un indice de criblage d'une tumeur hématopoïétique sur la base d'un nombre des cellules cibles.

**25.** Analyseur d'échantillon selon la revendication 24, dans lequel

le détecteur comprend une cellule d'écoulement (61) à travers laquelle s'écoule l'échantillon préparé par l'unité de préparation d'échantillon,
une source de lumière (62) configurée pour appliquer de la lumière à une particule dans l'échantillon s'écoulant dans la cellule d'écoulement, et
un récepteur de lumière (63, 64, 65) configuré pour obtenir les informations optiques lorsque de la lumière est appliquée à la particule.

**26.** Programme informatique pour l'analyse d'aspirat de moelle osseuse, le programme informatique amenant un ordinateur à exécuter :

l'obtention d'informations optiques incluant des informations de signal de fluorescence, obtenues par cytométrie en flux, pour des particules dans un échantillon contenant l'aspirat de moelle osseuse et un colorant fluorescent

pouvant marquer un acide nucléique ;
et **caractérisé en ce qu'**il amène en outre l'ordinateur à exécuter :

le comptage de particules pour lesquelles les informations de signal de fluorescence indiquent une valeur seuil ou plus, en tant que cellules cibles, sur la base des informations optiques ; et
l'obtention d'un indice de criblage d'une tumeur hématopoïétique sur la base d'un nombre des cellules cibles.

FIG. 1

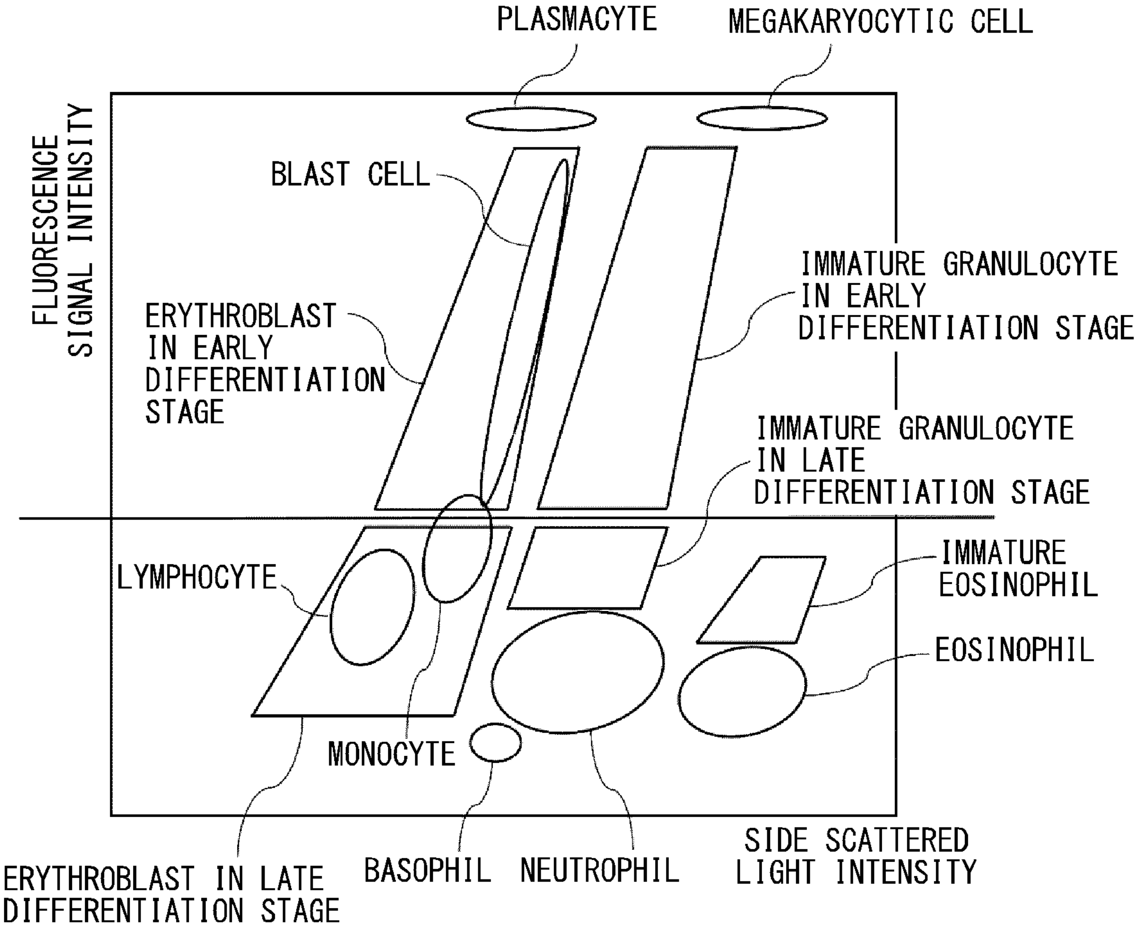
PLASMACYTE
MEGAKARYOCYTIC CELL
FLUORESCENCE SIGNAL INTENSITY
BLAST CELL
ERYTHROBLAST IN EARLY DIFFERENTIATION STAGE
IMMATURE GRANULOCYTE IN EARLY DIFFERENTIATION STAGE
IMMATURE GRANULOCYTE IN LATE DIFFERENTIATION STAGE
LYMPHOCYTE
IMMATURE EOSINOPHIL
EOSINOPHIL
MONOCYTE
ERYTHROBLAST IN LATE DIFFERENTIATION STAGE
BASOPHIL
NEUTROPHIL
SIDE SCATTERED LIGHT INTENSITY

FIG. 2

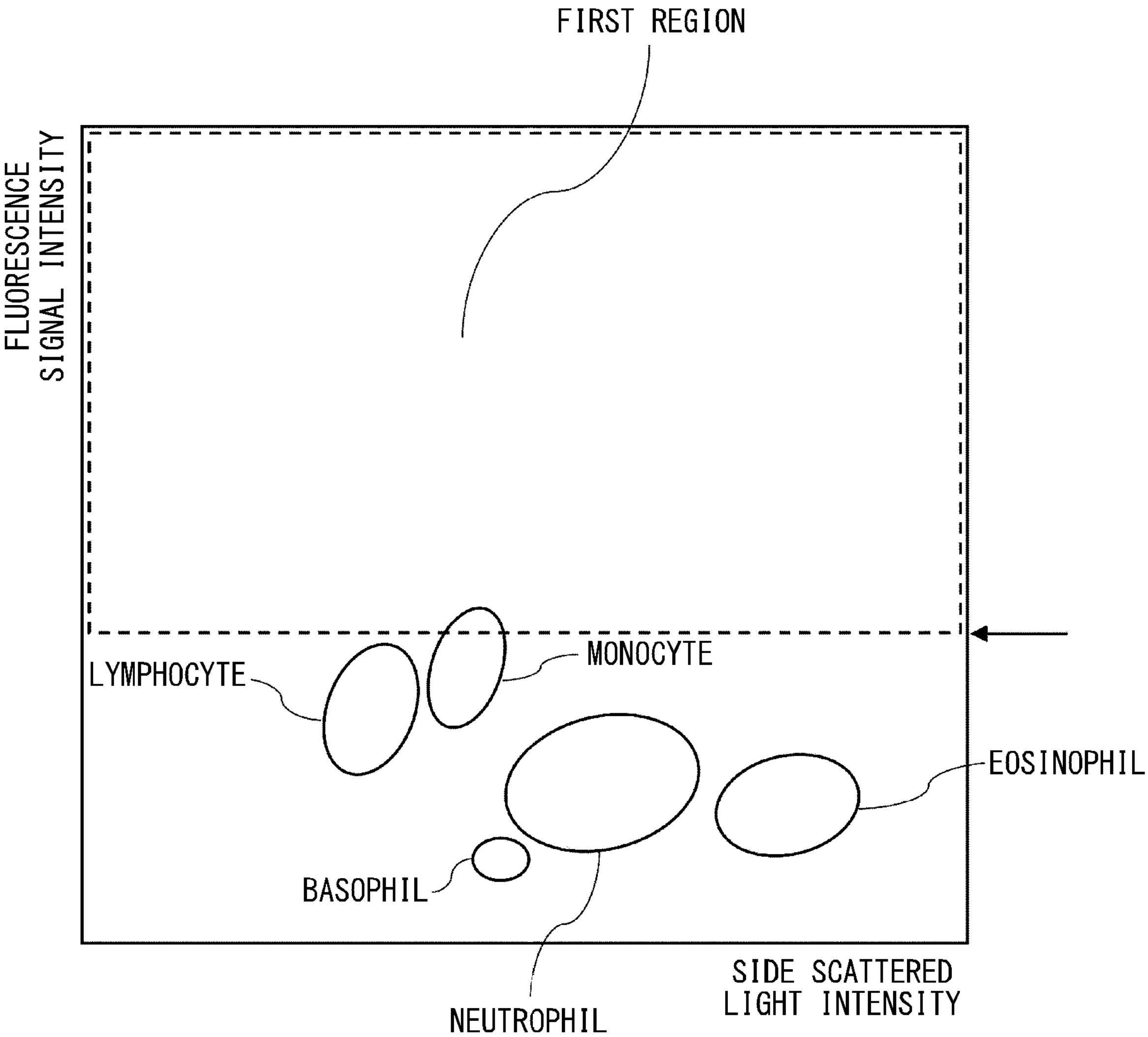
FIRST REGION
FLUORESCENCE SIGNAL INTENSITY
LYMPHOCYTE
MONOCYTE
EOSINOPHIL
BASOPHIL
NEUTROPHIL
SIDE SCATTERED LIGHT INTENSITY

FIG. 3A

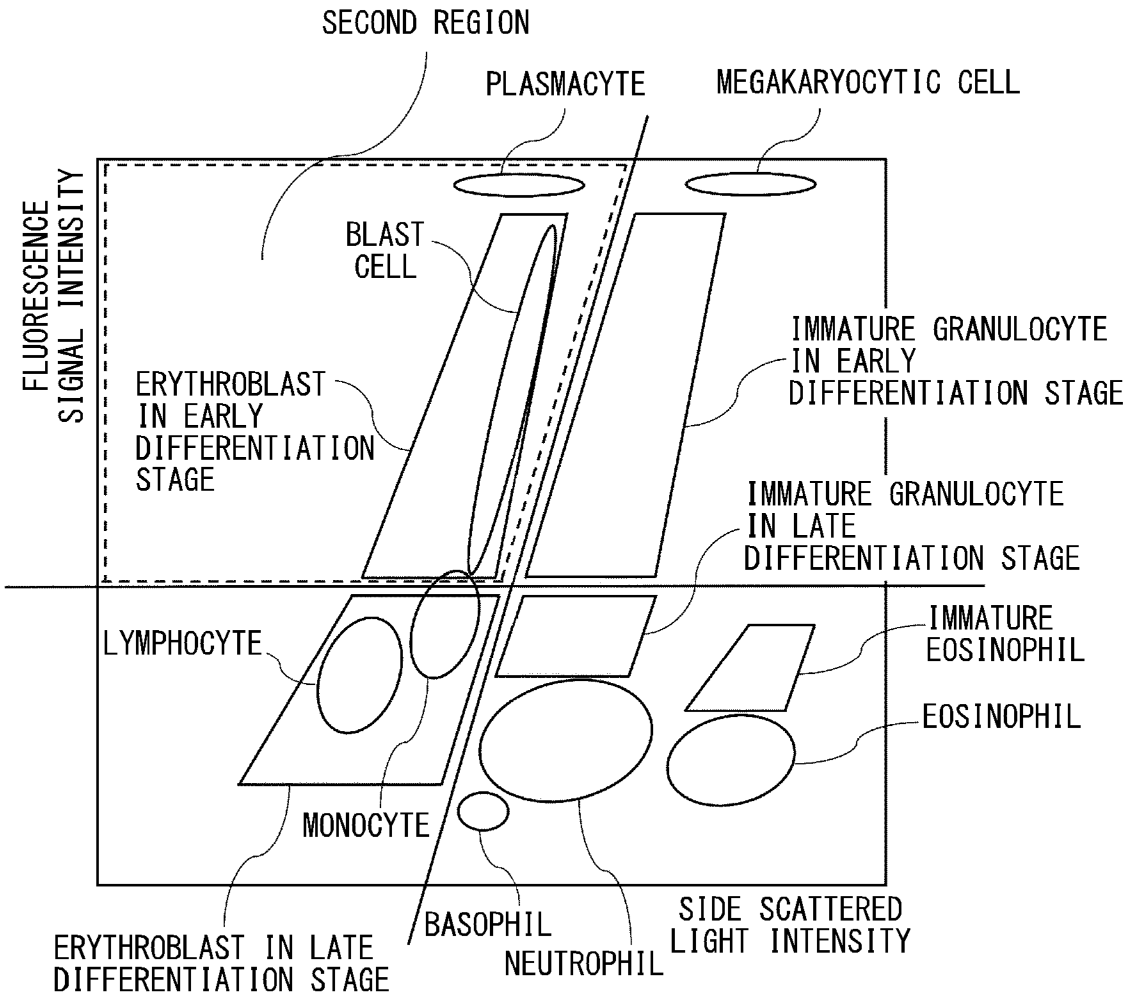
SECOND REGION
PLASMACYTE
MEGAKARYOCYTIC CELL
FLUORESCENCE SIGNAL INTENSITY
BLAST CELL
ERYTHROBLAST IN EARLY DIFFERENTIATION STAGE
IMMATURE GRANULOCYTE IN EARLY DIFFERENTIATION STAGE
IMMATURE GRANULOCYTE IN LATE DIFFERENTIATION STAGE
LYMPHOCYTE
IMMATURE EOSINOPHIL
EOSINOPHIL
MONOCYTE
ERYTHROBLAST IN LATE DIFFERENTIATION STAGE
BASOPHIL
NEUTROPHIL
SIDE SCATTERED LIGHT INTENSITY

FIG. 3B

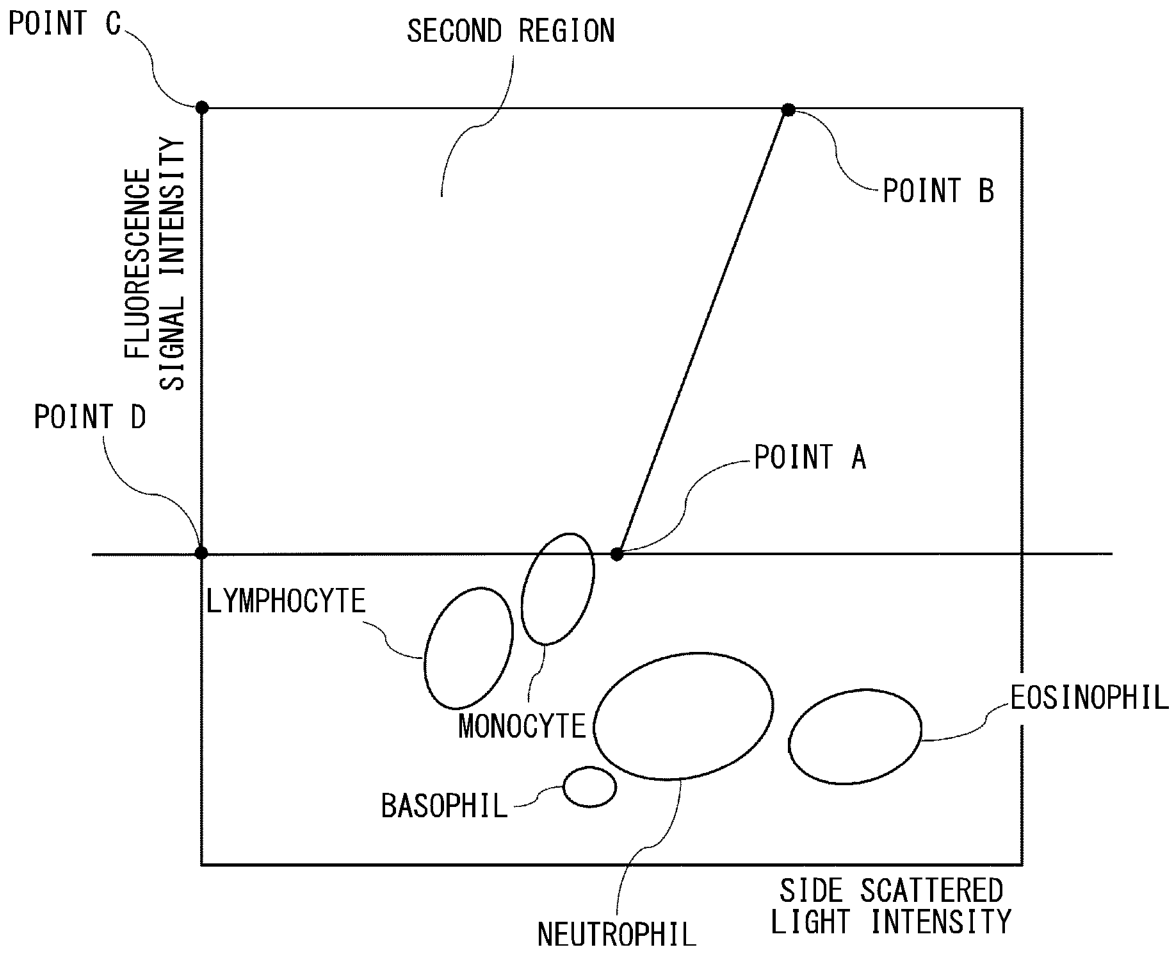
POINT C
SECOND REGION
POINT B
FLUORESCENCE SIGNAL INTENSITY
POINT D
POINT A
LYMPHOCYTE
MONOCYTE
EOSINOPHIL
BASOPHIL
NEUTROPHIL
SIDE SCATTERED LIGHT INTENSITY

FIG. 4A

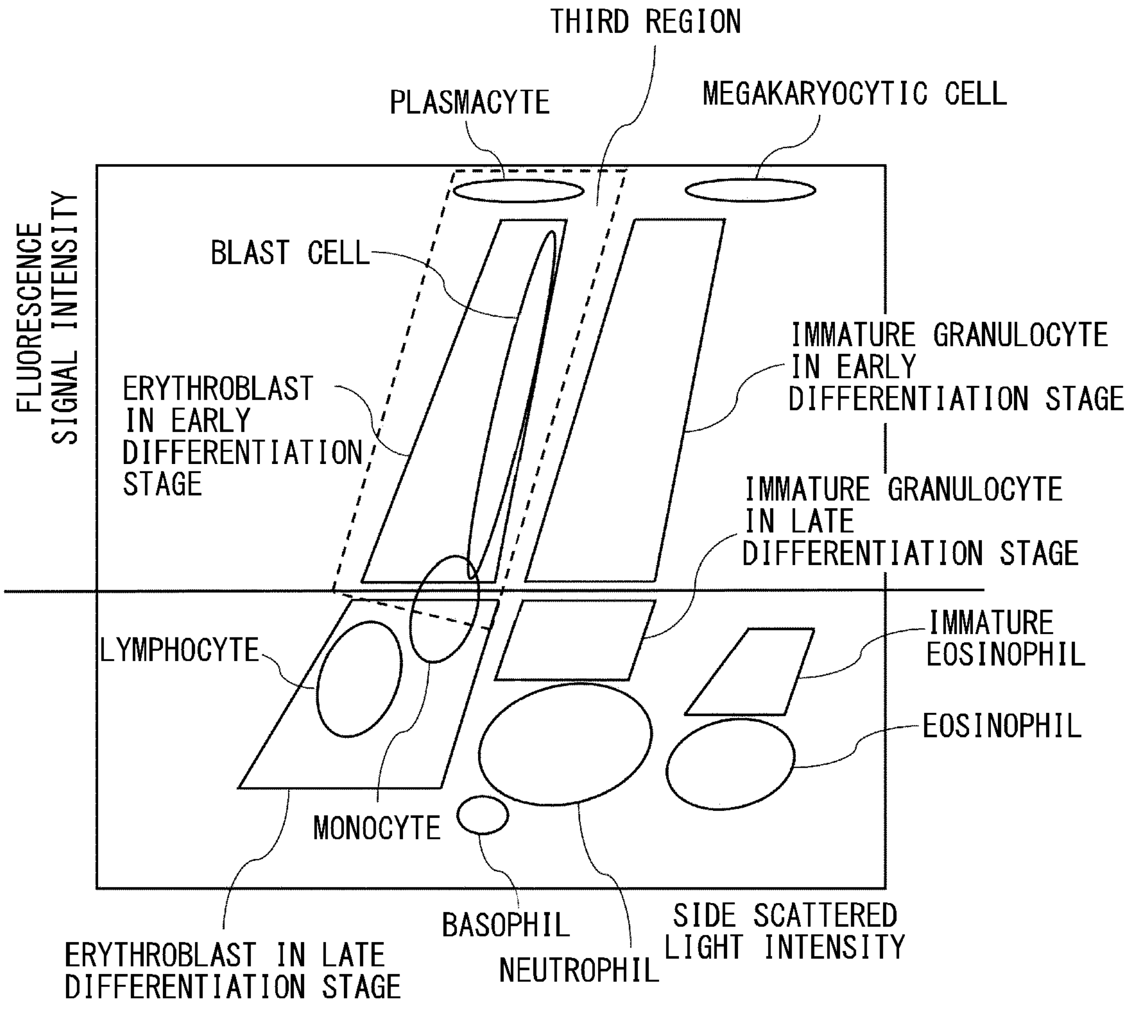
THIRD REGION
PLASMACYTE
MEGAKARYOCYTIC CELL
FLUORESCENCE SIGNAL INTENSITY
BLAST CELL
IMMATURE GRANULOCYTE IN EARLY DIFFERENTIATION STAGE
ERYTHROBLAST IN EARLY DIFFERENTIATION STAGE
IMMATURE GRANULOCYTE IN LATE DIFFERENTIATION STAGE
LYMPHOCYTE
IMMATURE EOSINOPHIL
EOSINOPHIL
MONOCYTE
BASOPHIL
SIDE SCATTERED LIGHT INTENSITY
ERYTHROBLAST IN LATE DIFFERENTIATION STAGE
NEUTROPHIL

FIG. 4B

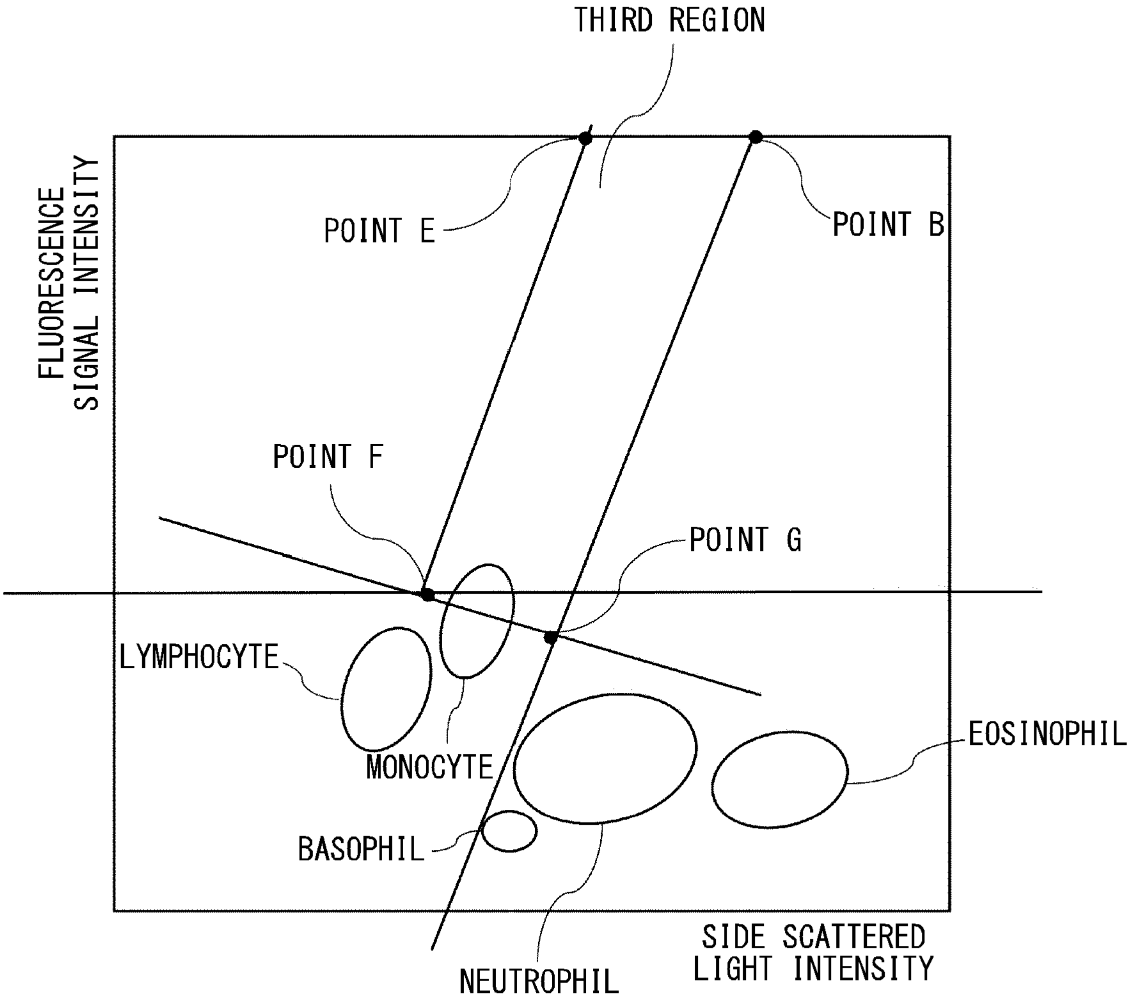
THIRD REGION
POINT E
POINT B
FLUORESCENCE SIGNAL INTENSITY
POINT F
POINT G
LYMPHOCYTE
MONOCYTE
EOSINOPHIL
BASOPHIL
NEUTROPHIL
SIDE SCATTERED LIGHT INTENSITY

FIG. 4C

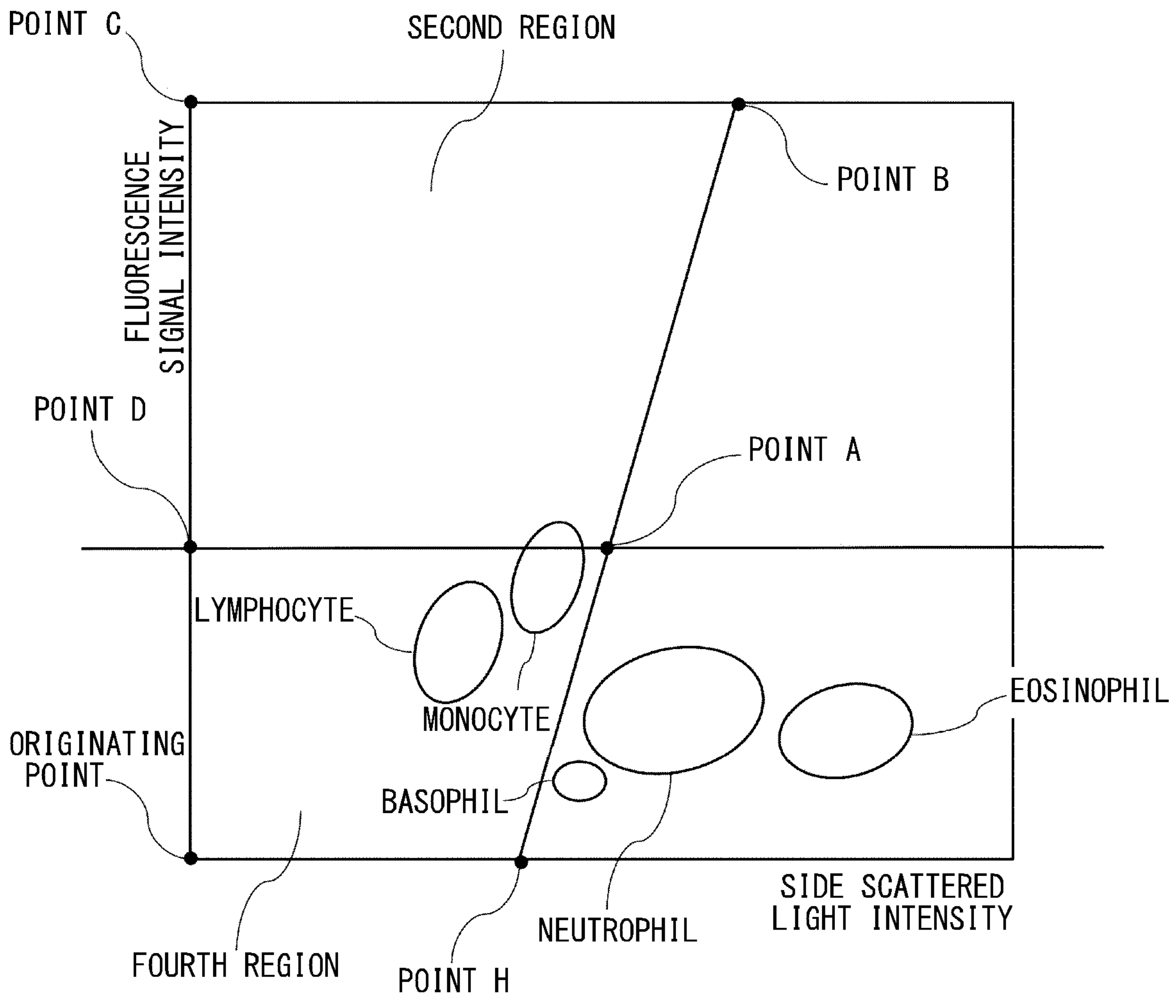
POINT C
SECOND REGION
POINT B
FLUORESCENCE SIGNAL INTENSITY
POINT D
POINT A
LYMPHOCYTE
MONOCYTE
EOSINOPHIL
ORIGINATING POINT
BASOPHIL
SIDE SCATTERED LIGHT INTENSITY
NEUTROPHIL
FOURTH REGION
POINT H

FIG. 5A

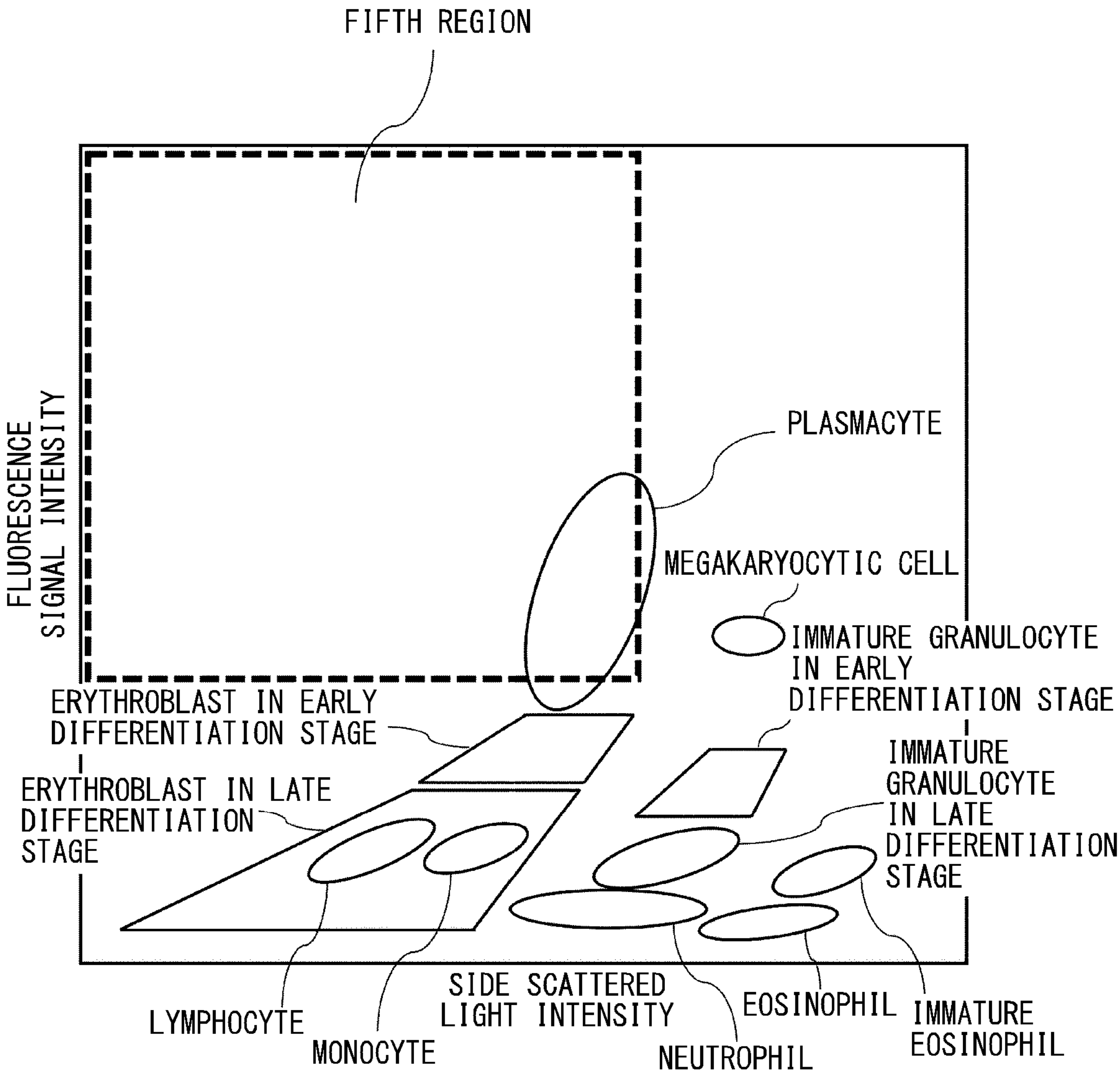
FIFTH REGION
PLASMACYTE
MEGAKARYOCYTIC CELL
IMMATURE GRANULOCYTE IN EARLY DIFFERENTIATION STAGE
ERYTHROBLAST IN EARLY DIFFERENTIATION STAGE
ERYTHROBLAST IN LATE DIFFERENTIATION STAGE
IMMATURE GRANULOCYTE IN LATE DIFFERENTIATION STAGE
FLUORESCENCE SIGNAL INTENSITY
SIDE SCATTERED LIGHT INTENSITY
LYMPHOCYTE
MONOCYTE
NEUTROPHIL
EOSINOPHIL
IMMATURE EOSINOPHIL

FIG. 5B

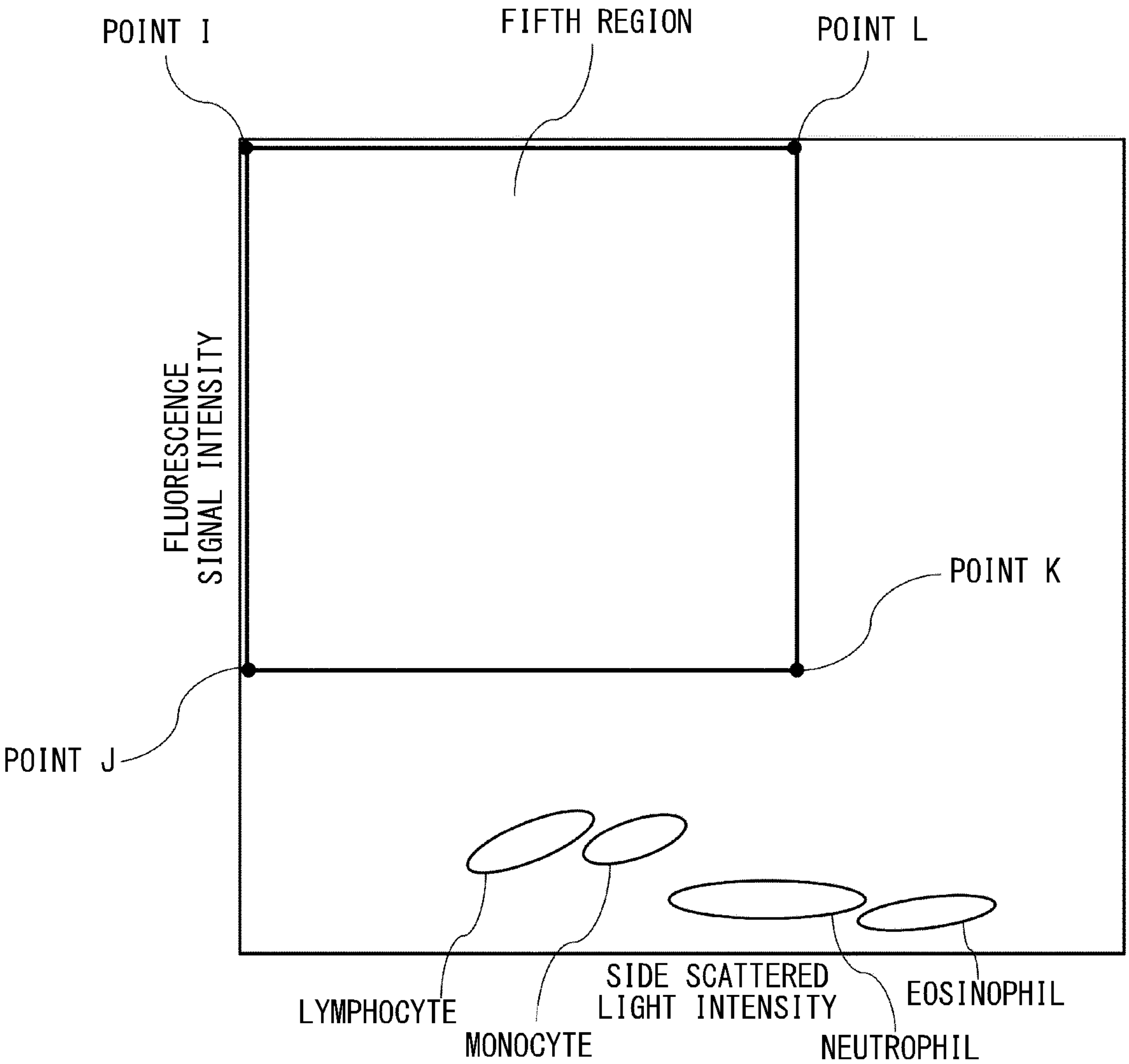
POINT I
FIFTH REGION
POINT L
FLUORESCENCE SIGNAL INTENSITY
POINT K
POINT J
LYMPHOCYTE
MONOCYTE
SIDE SCATTERED LIGHT INTENSITY
EOSINOPHIL
NEUTROPHIL

FIG. 6

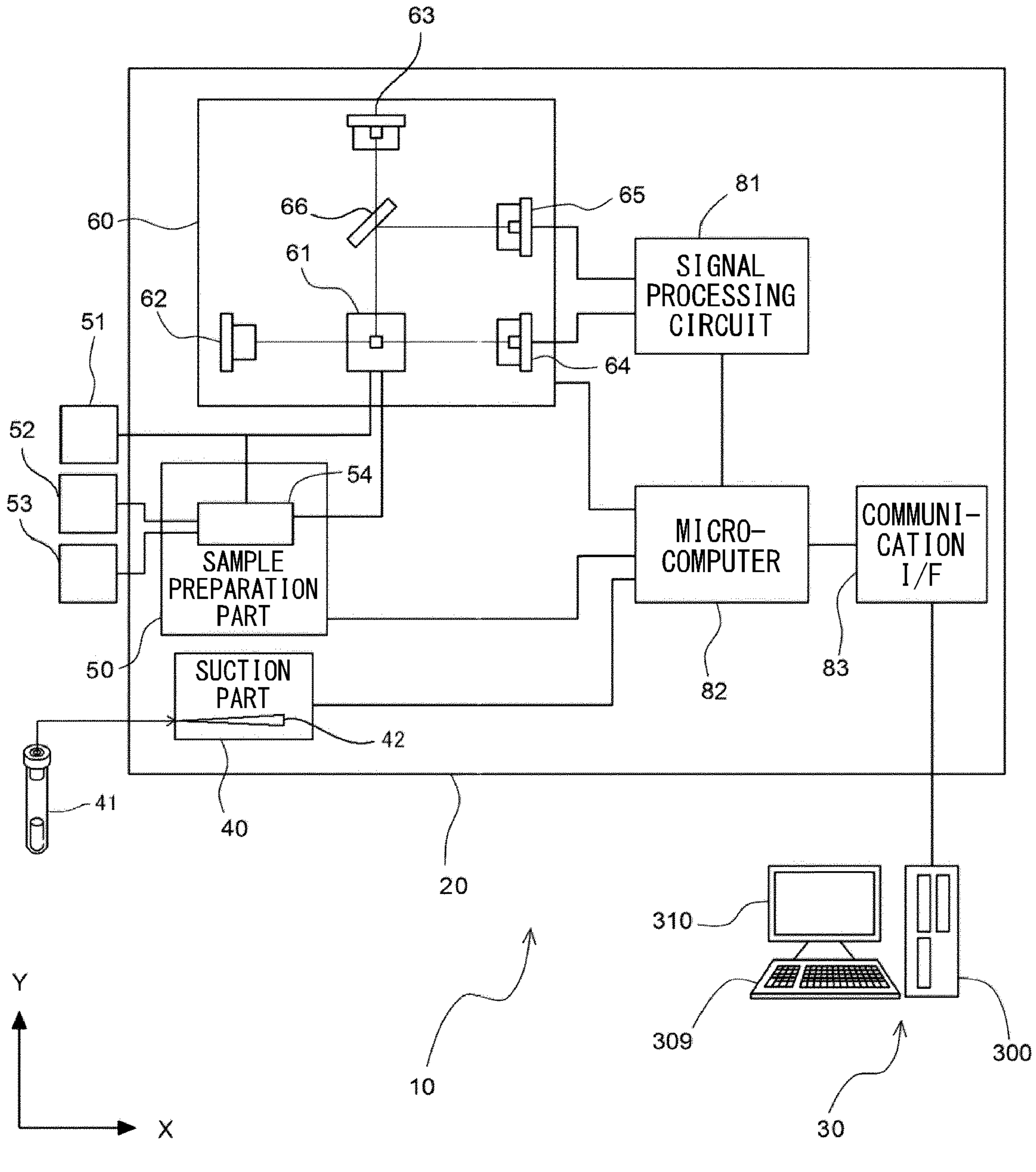
63
65
81
60
66
61
SIGNAL
PROCESSING
CIRCUIT
62
51
64
52
54
53
MICRO-
COMPUTER
COMMUNI-
CATION
I/F
SAMPLE
PREPARATION
PART
50
SUCTION
PART
82
83
42
41
40
20
310
Y
309
300
10
30
X

FIG. 7

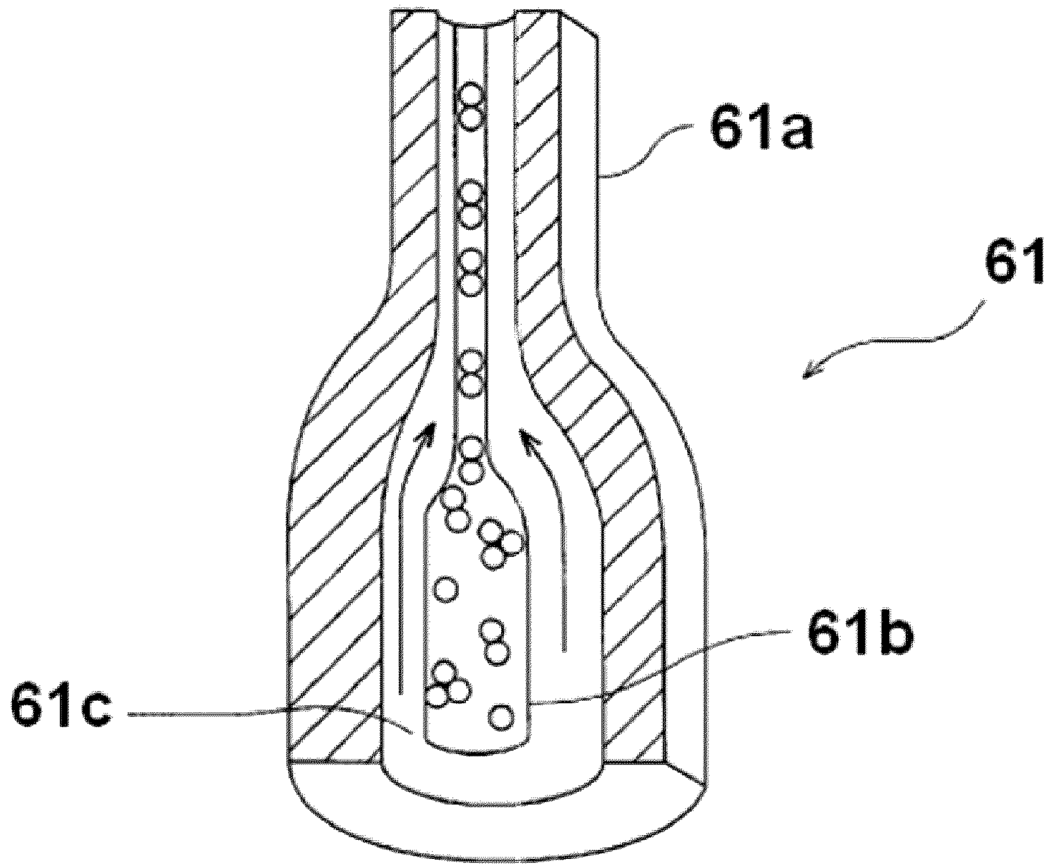

FIG. 8

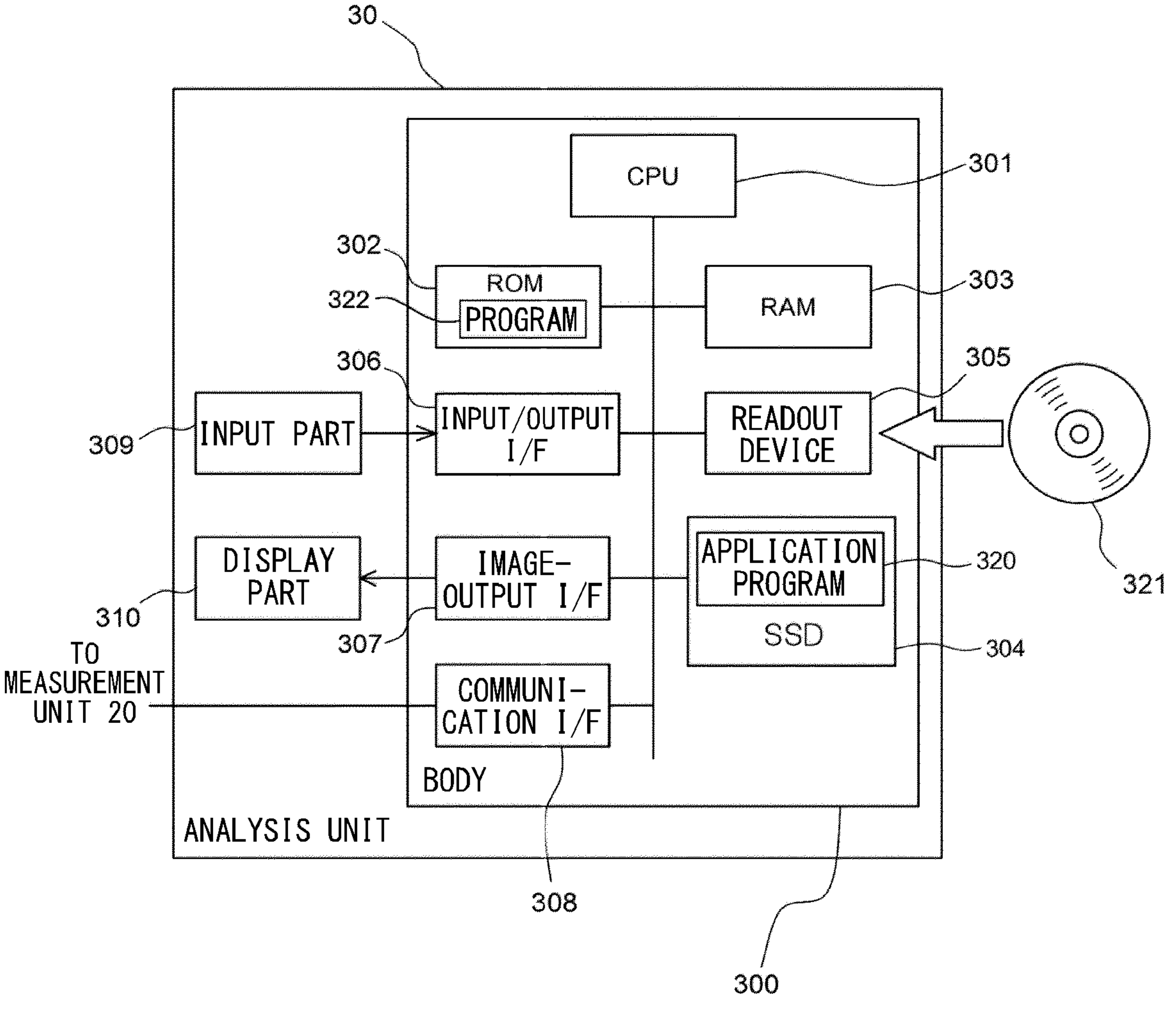
30
CPU
301
302
ROM
322
PROGRAM
RAM
303
306
305
INPUT PART
INPUT/OUTPUT I/F
READOUT DEVICE
309
DISPLAY PART
IMAGE-OUTPUT I/F
APPLICATION PROGRAM
320
321
SSD
310
307
304
TO MEASUREMENT UNIT 20
COMMUNI-CATION I/F
BODY
ANALYSIS UNIT
308
300

FIG. 9

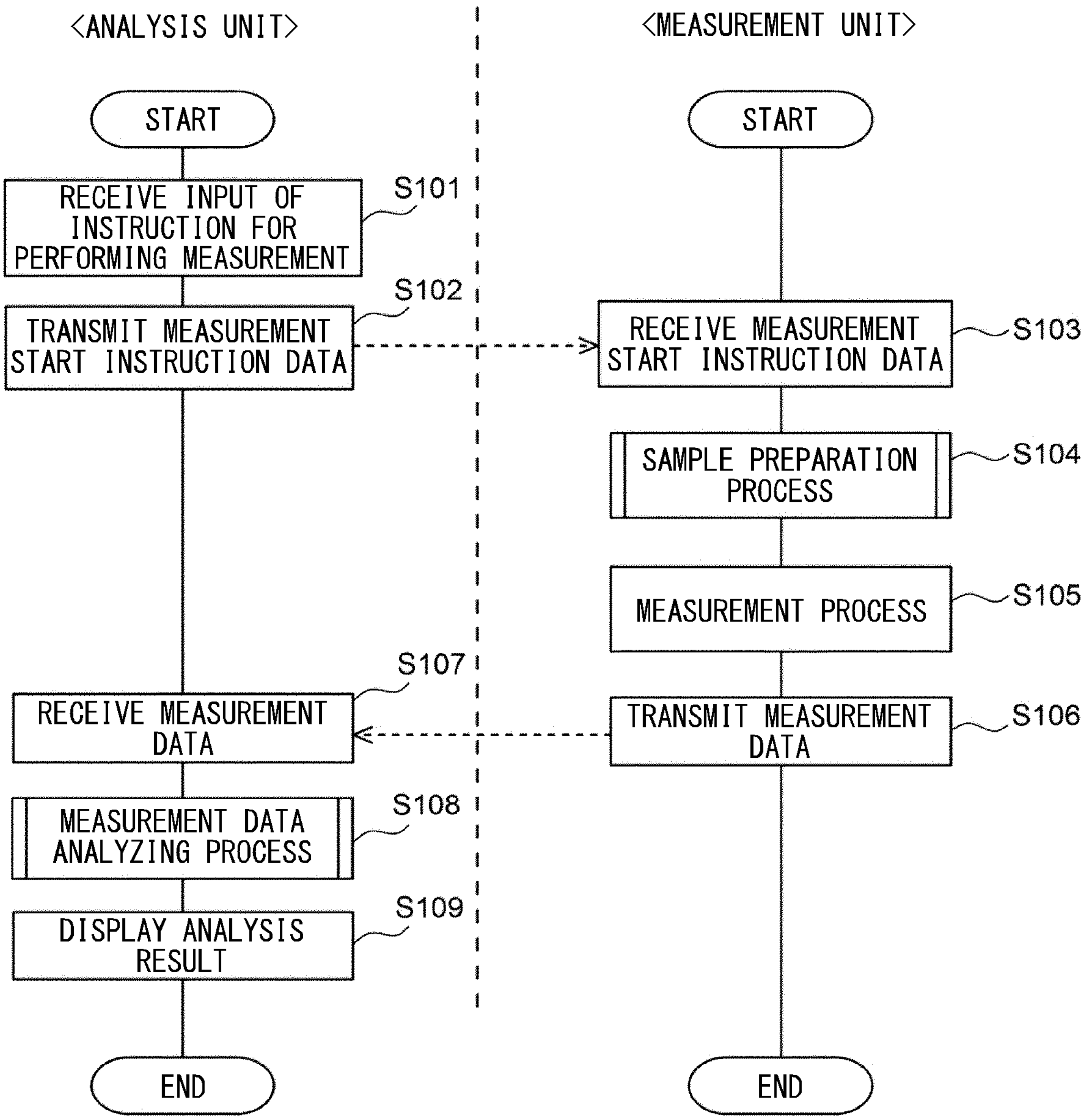

<ANALYSIS UNIT>
<MEASUREMENT UNIT>
START
START
RECEIVE INPUT OF INSTRUCTION FOR PERFORMING MEASUREMENT
S101
TRANSMIT MEASUREMENT START INSTRUCTION DATA
S102
RECEIVE MEASUREMENT START INSTRUCTION DATA
S103
SAMPLE PREPARATION PROCESS
S104
MEASUREMENT PROCESS
S105
TRANSMIT MEASUREMENT DATA
S106
RECEIVE MEASUREMENT DATA
S107
MEASUREMENT DATA ANALYZING PROCESS
S108
DISPLAY ANALYSIS RESULT
S109
END
END

FIG. 10

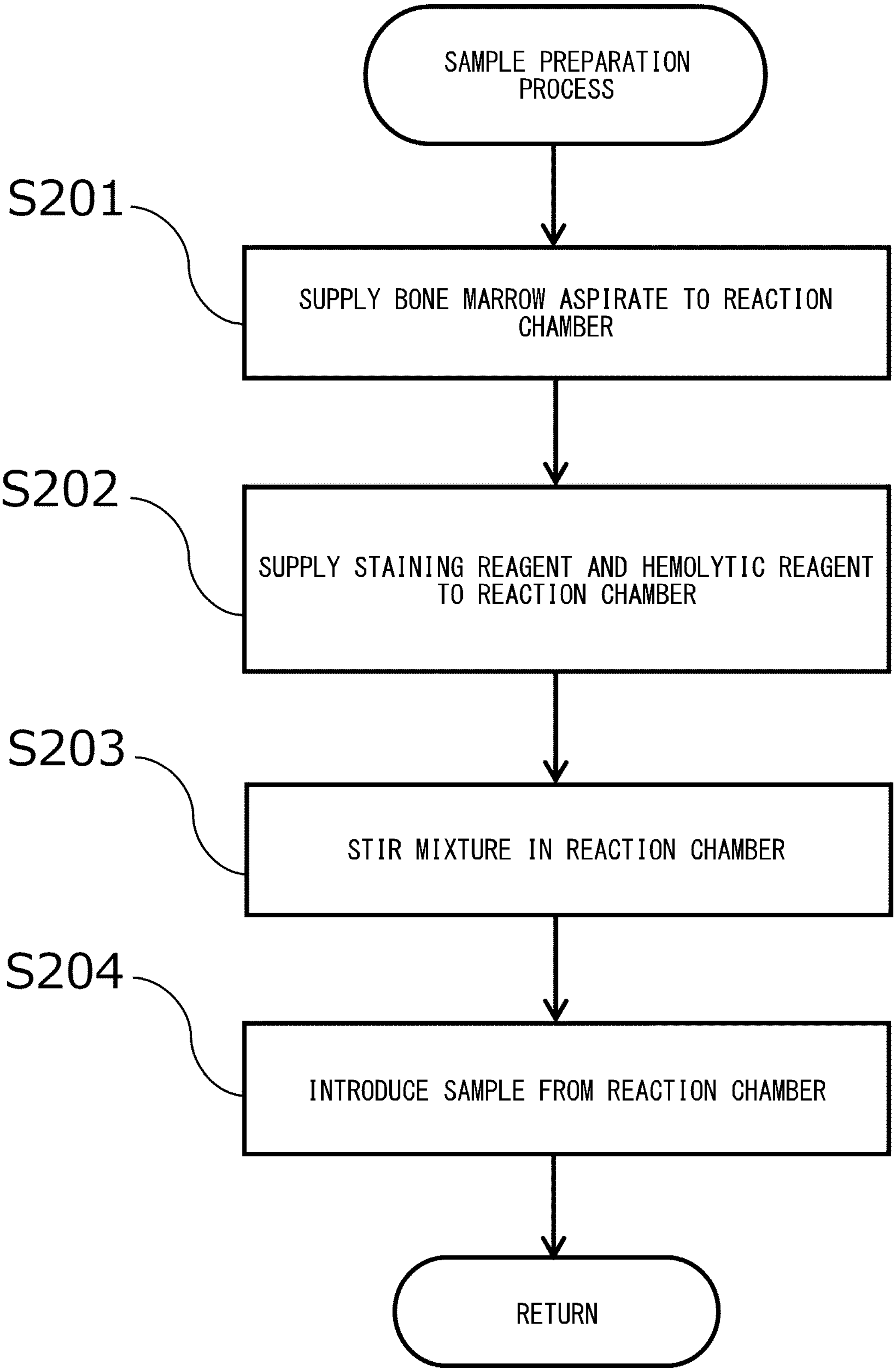
SAMPLE PREPARATION PROCESS
S201
SUPPLY BONE MARROW ASPIRATE TO REACTION CHAMBER
S202
SUPPLY STAINING REAGENT AND HEMOLYTIC REAGENT TO REACTION CHAMBER
S203
STIR MIXTURE IN REACTION CHAMBER
S204
INTRODUCE SAMPLE FROM REACTION CHAMBER
RETURN

FIG. 11A

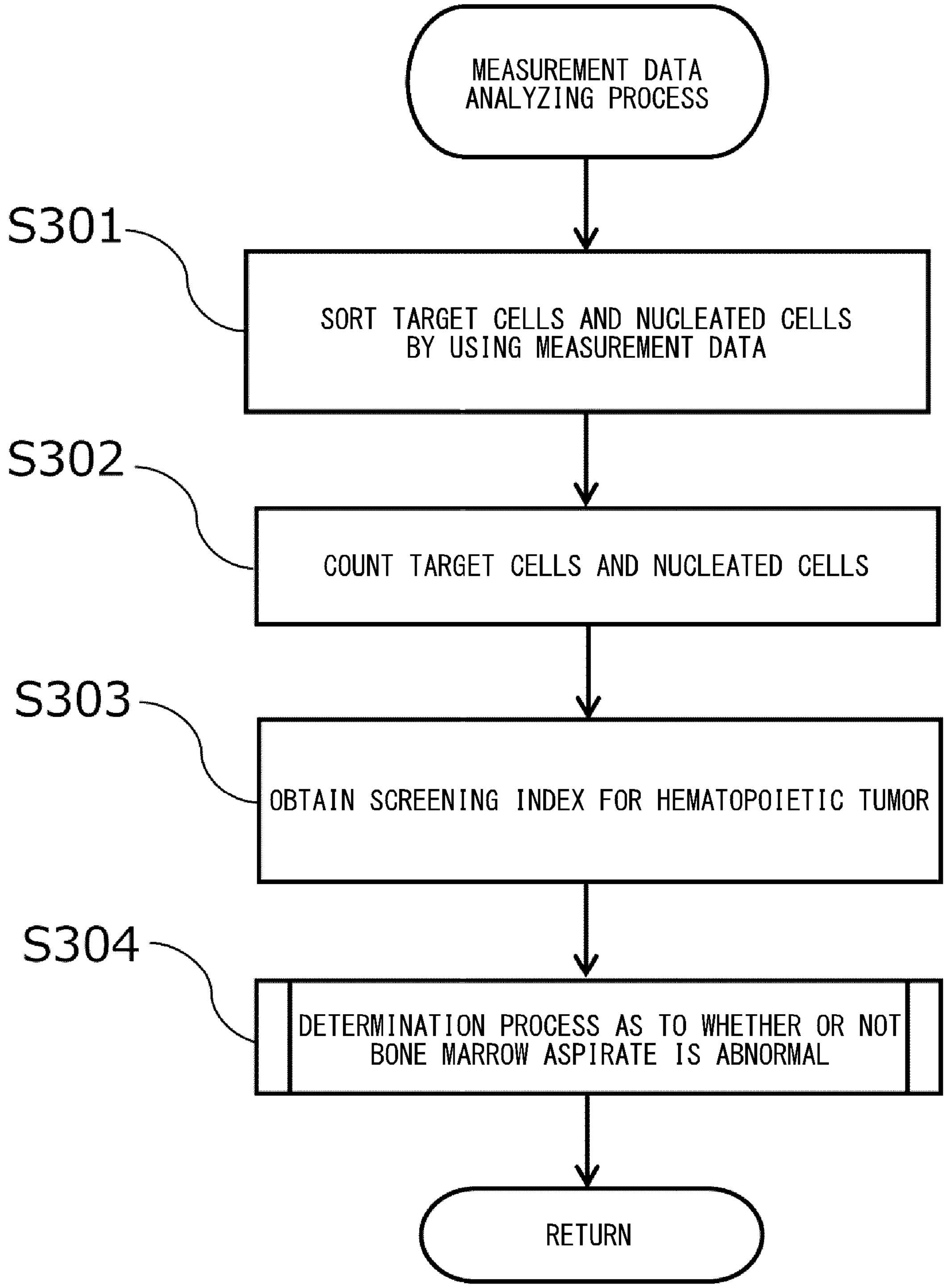
MEASUREMENT DATA ANALYZING PROCESS
S301
SORT TARGET CELLS AND NUCLEATED CELLS BY USING MEASUREMENT DATA
S302
COUNT TARGET CELLS AND NUCLEATED CELLS
S303
OBTAIN SCREENING INDEX FOR HEMATOPOIETIC TUMOR
S304
DETERMINATION PROCESS AS TO WHETHER OR NOT BONE MARROW ASPIRATE IS ABNORMAL
RETURN

FIG. 11B

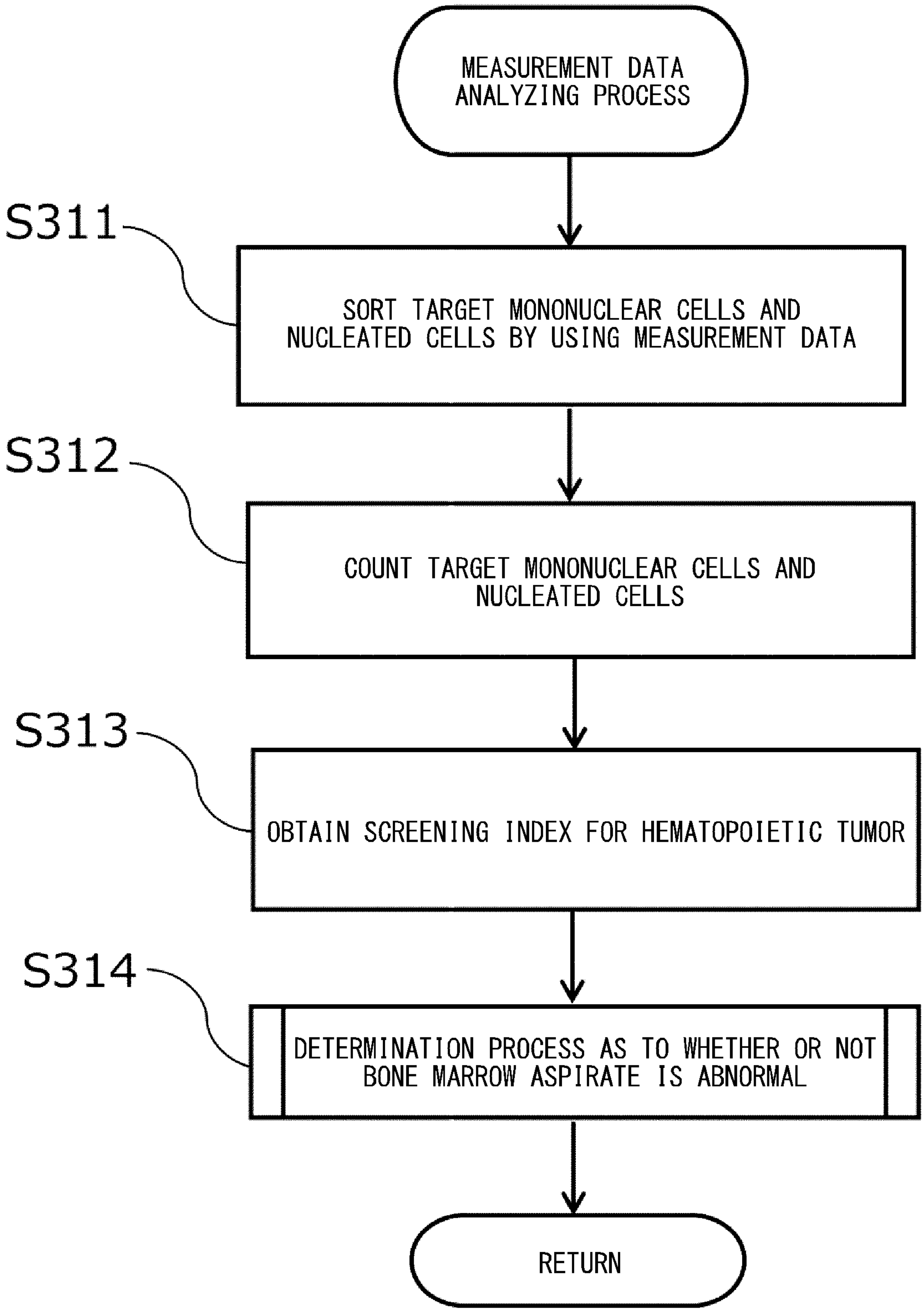
MEASUREMENT DATA ANALYZING PROCESS
S311
SORT TARGET MONONUCLEAR CELLS AND NUCLEATED CELLS BY USING MEASUREMENT DATA
S312
COUNT TARGET MONONUCLEAR CELLS AND NUCLEATED CELLS
S313
OBTAIN SCREENING INDEX FOR HEMATOPOIETIC TUMOR
S314
DETERMINATION PROCESS AS TO WHETHER OR NOT BONE MARROW ASPIRATE IS ABNORMAL
RETURN

FIG. 11C

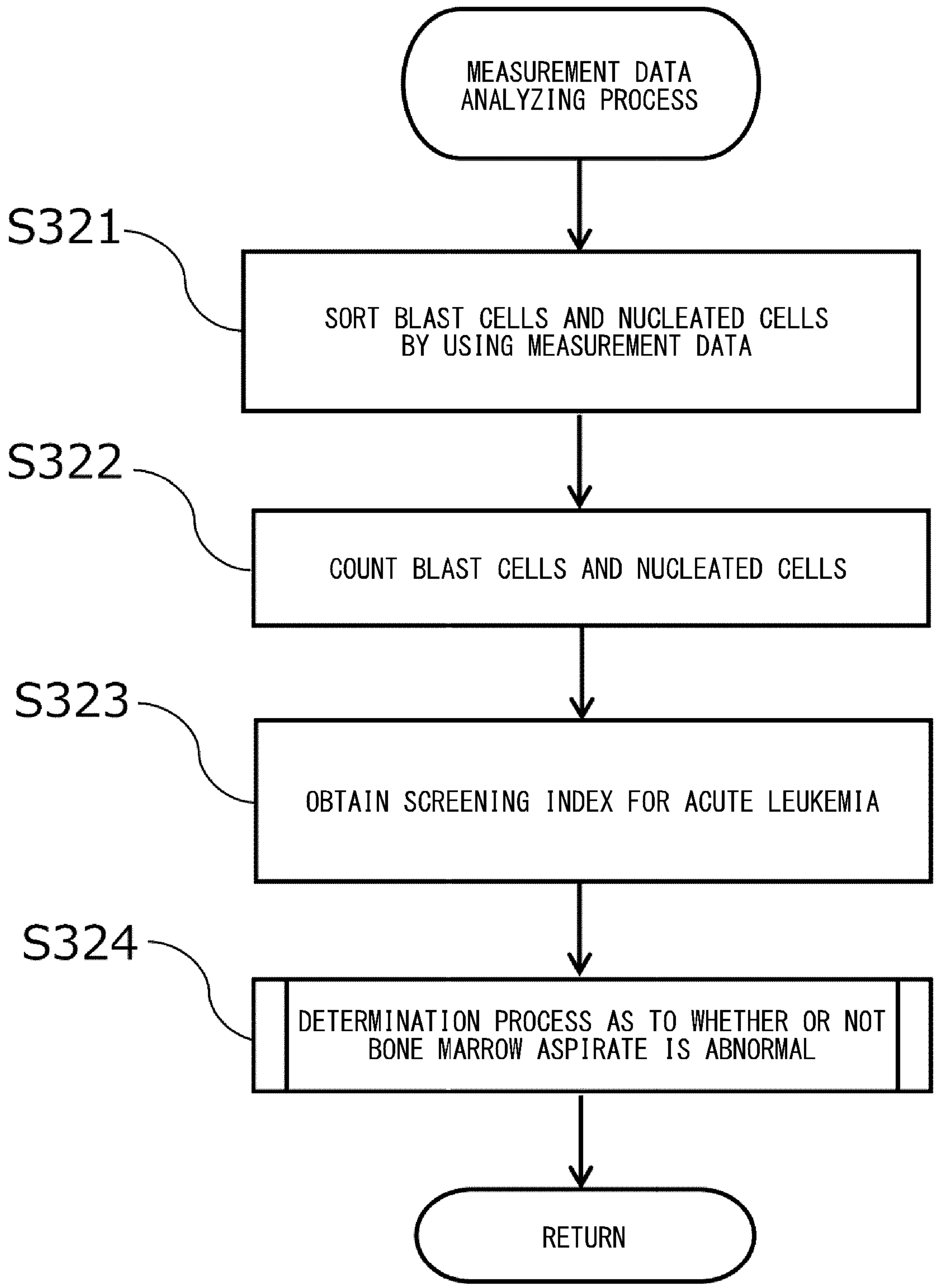
MEASUREMENT DATA ANALYZING PROCESS
S321
SORT BLAST CELLS AND NUCLEATED CELLS BY USING MEASUREMENT DATA
S322
COUNT BLAST CELLS AND NUCLEATED CELLS
S323
OBTAIN SCREENING INDEX FOR ACUTE LEUKEMIA
S324
DETERMINATION PROCESS AS TO WHETHER OR NOT BONE MARROW ASPIRATE IS ABNORMAL
RETURN

FIG. 11D

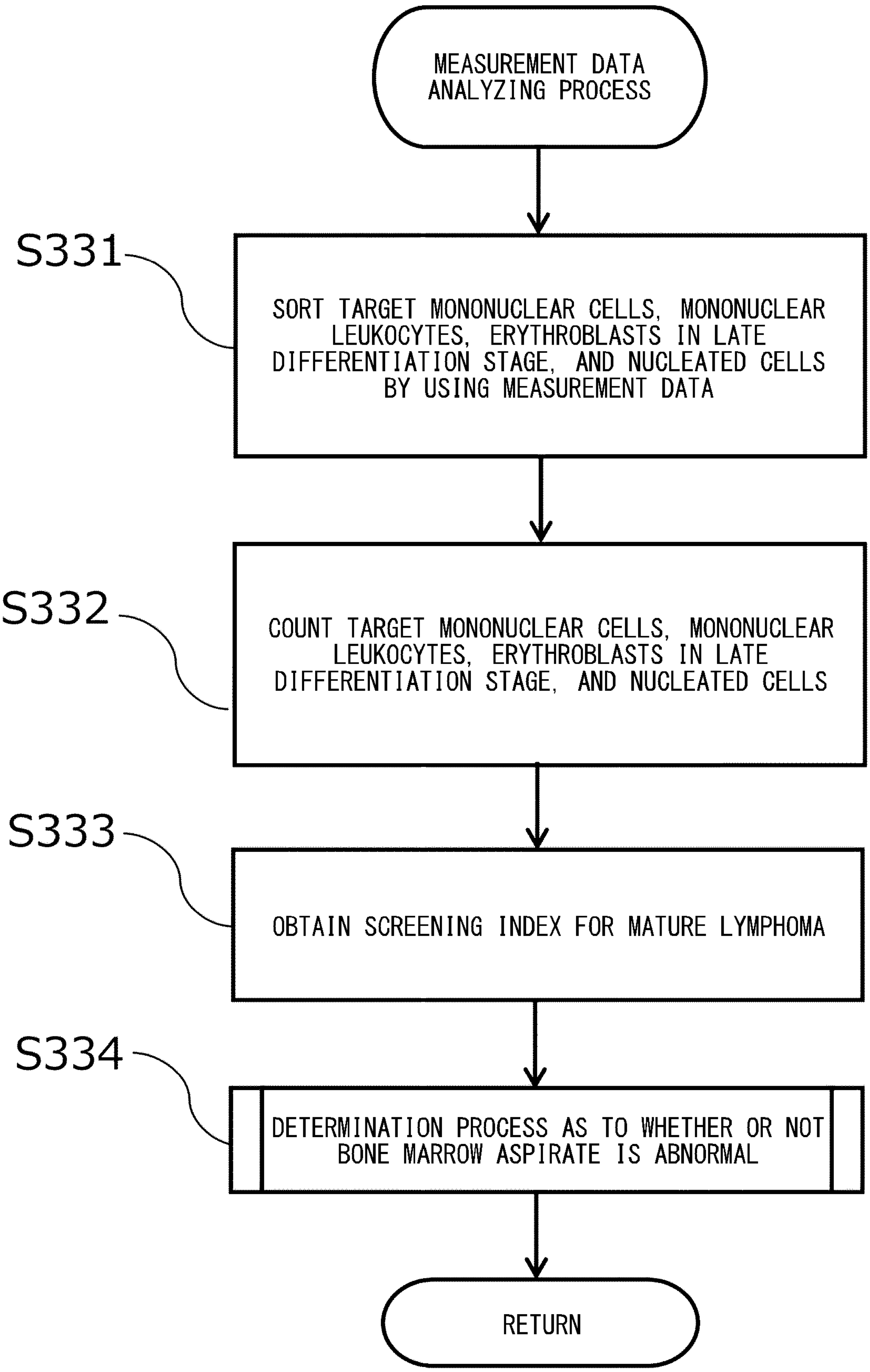
MEASUREMENT DATA ANALYZING PROCESS
S331
SORT TARGET MONONUCLEAR CELLS, MONONUCLEAR LEUKOCYTES, ERYTHROBLASTS IN LATE DIFFERENTIATION STAGE, AND NUCLEATED CELLS BY USING MEASUREMENT DATA
S332
COUNT TARGET MONONUCLEAR CELLS, MONONUCLEAR LEUKOCYTES, ERYTHROBLASTS IN LATE DIFFERENTIATION STAGE, AND NUCLEATED CELLS
S333
OBTAIN SCREENING INDEX FOR MATURE LYMPHOMA
S334
DETERMINATION PROCESS AS TO WHETHER OR NOT BONE MARROW ASPIRATE IS ABNORMAL
RETURN

FIG. 11E

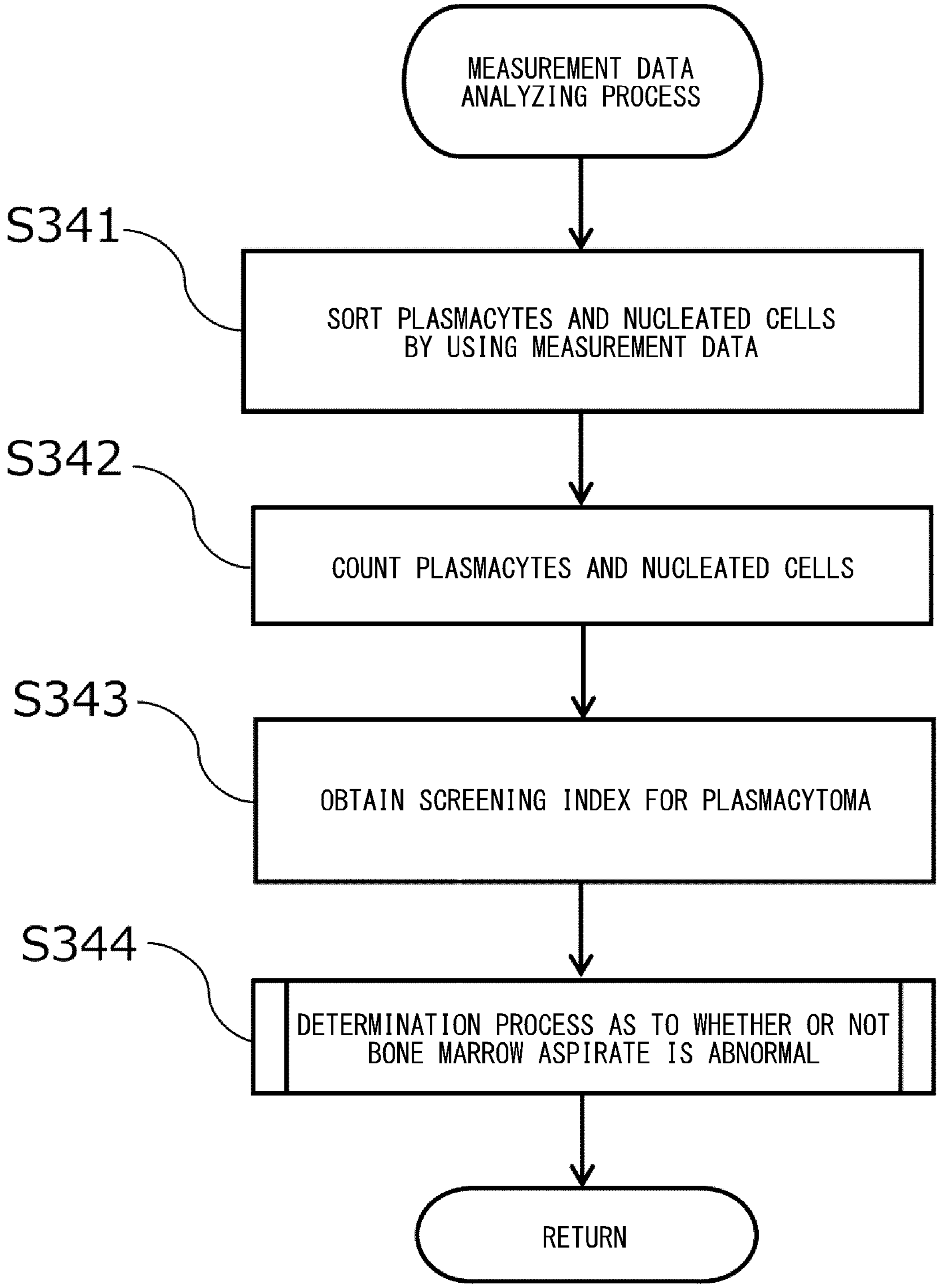
MEASUREMENT DATA ANALYZING PROCESS
S341
SORT PLASMACYTES AND NUCLEATED CELLS BY USING MEASUREMENT DATA
S342
COUNT PLASMACYTES AND NUCLEATED CELLS
S343
OBTAIN SCREENING INDEX FOR PLASMACYTOMA
S344
DETERMINATION PROCESS AS TO WHETHER OR NOT BONE MARROW ASPIRATE IS ABNORMAL
RETURN

FIG. 12A

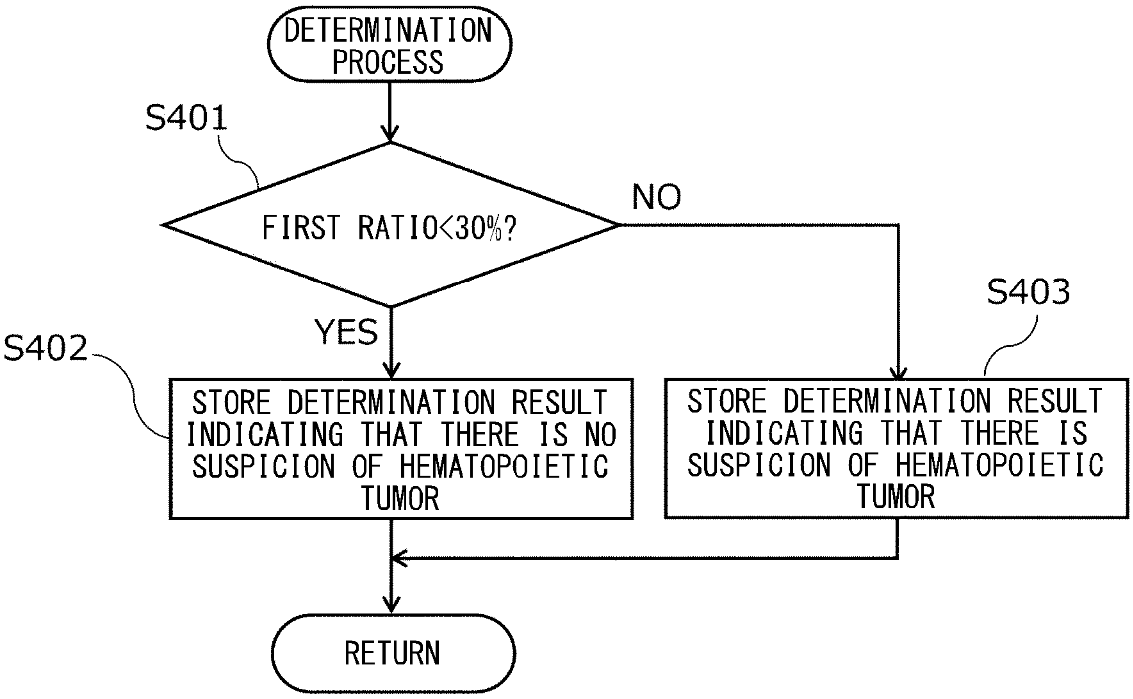


FIG. 12B

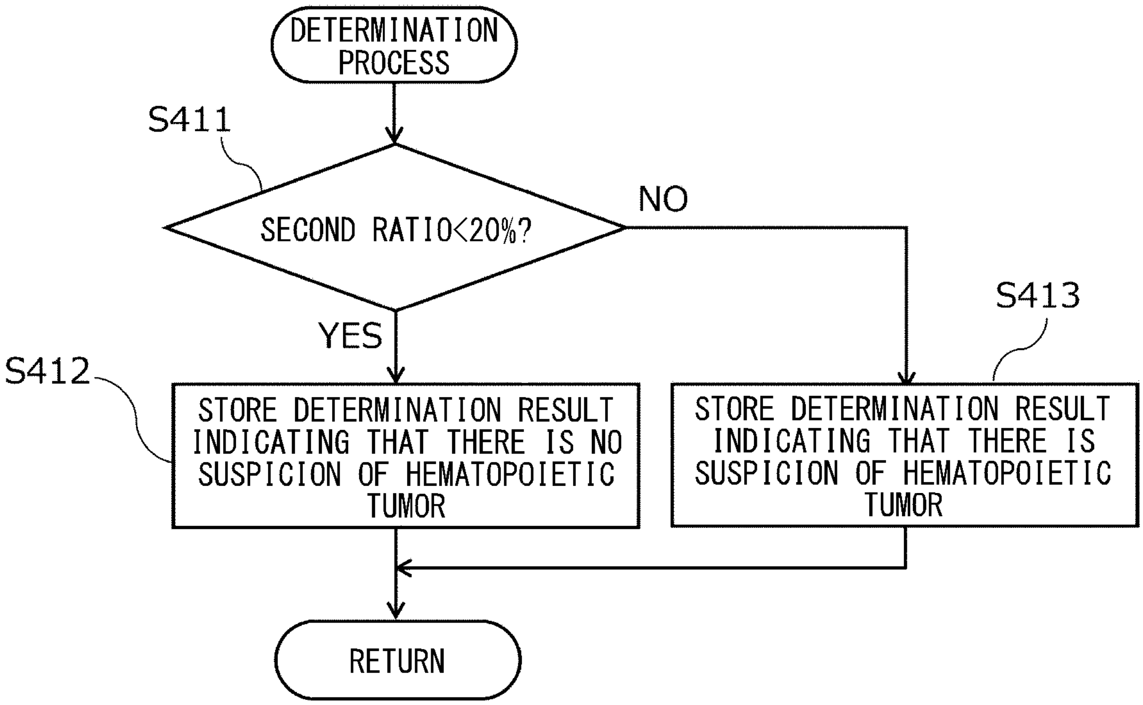

FIG. 12C

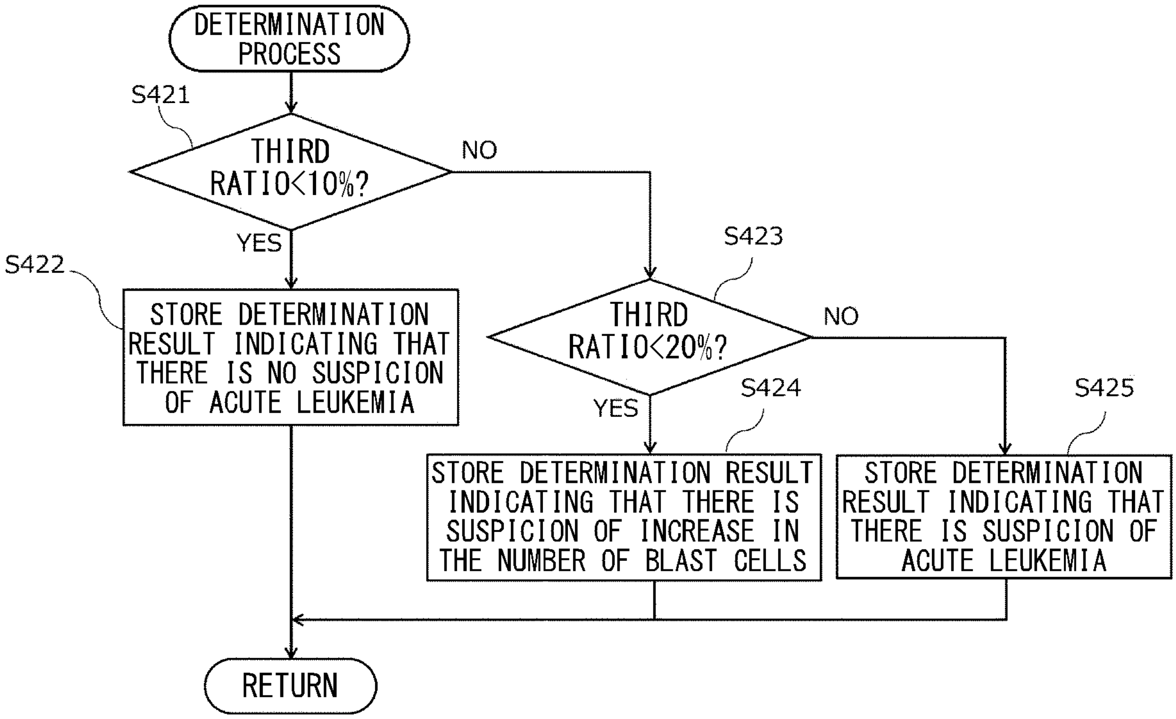


FIG. 12D

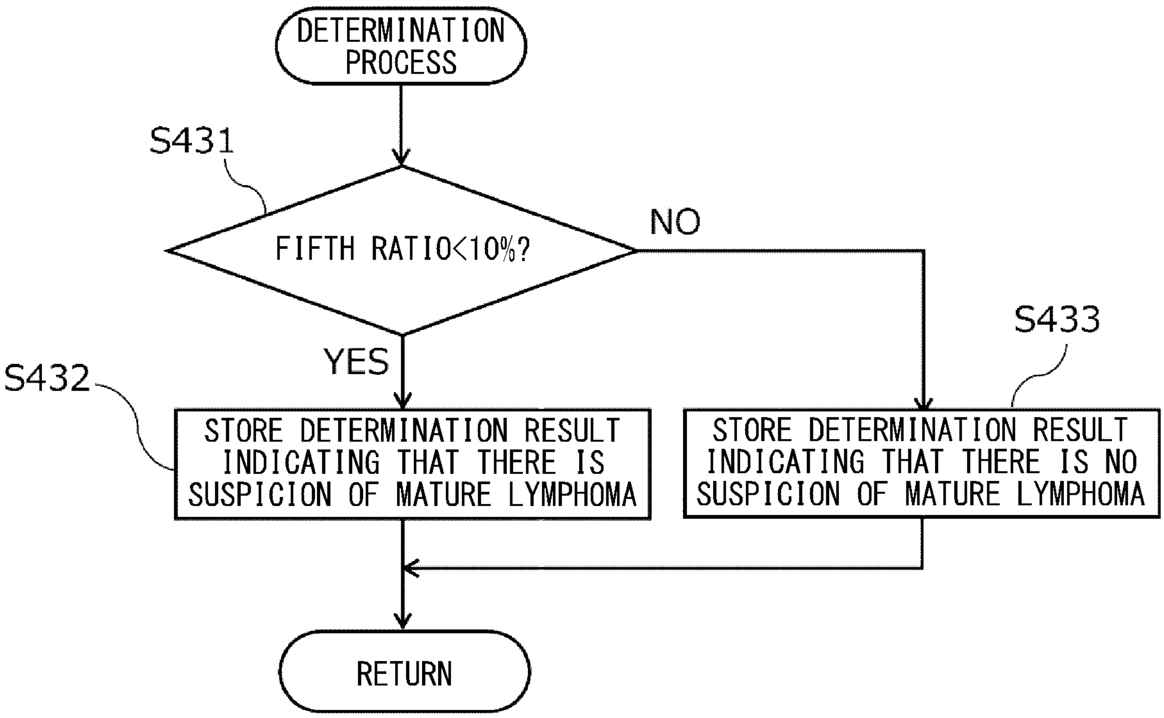

FIG. 12E

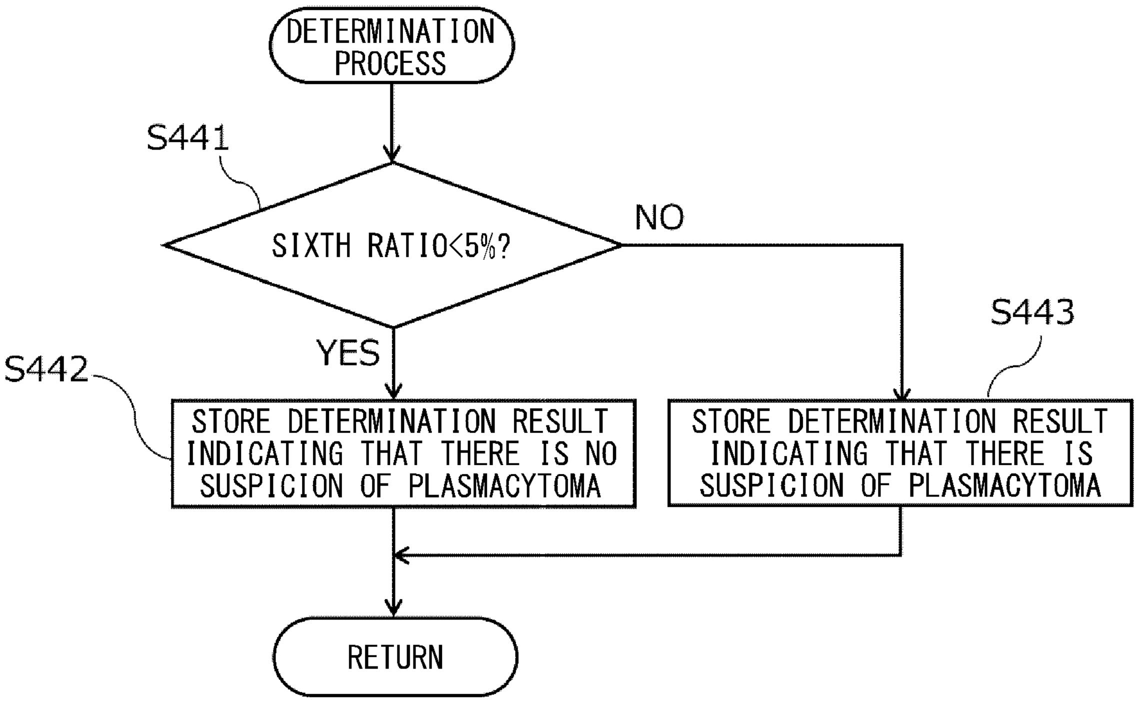
DETERMINATION PROCESS
S441
SIXTH RATIO<5%?
NO
YES
S442
STORE DETERMINATION RESULT INDICATING THAT THERE IS NO SUSPICION OF PLASMACYTOMA
S443
STORE DETERMINATION RESULT INDICATING THAT THERE IS SUSPICION OF PLASMACYTOMA
RETURN

FIG. 13

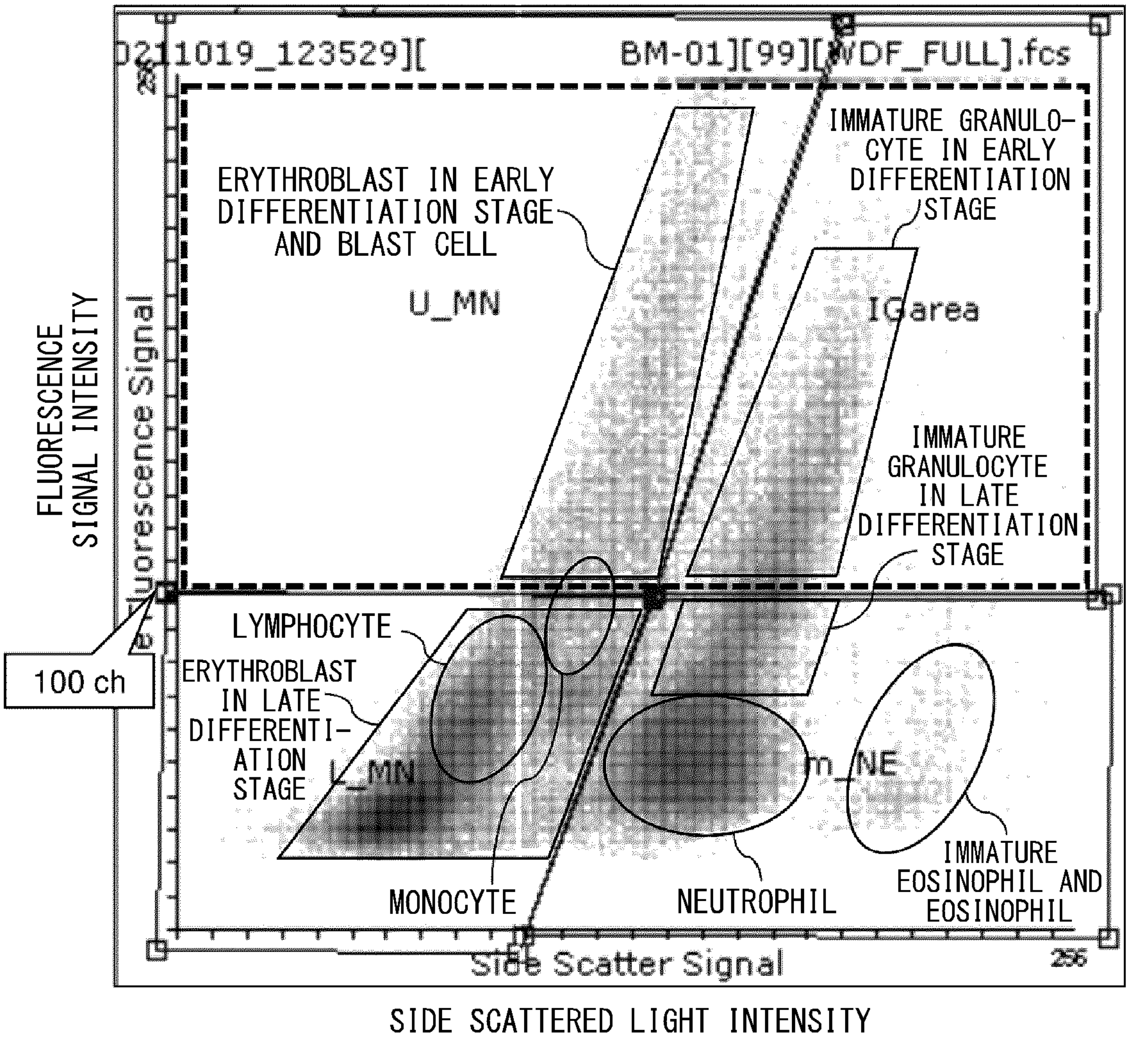
D211019_123529][
BM-01][99][WDF_FULL].fcs
256
ERYTHROBLAST IN EARLY DIFFERENTIATION STAGE AND BLAST CELL
U_MN
IMMATURE GRANULOCYTE IN EARLY DIFFERENTIATION STAGE
IGarea
IMMATURE GRANULOCYTE IN LATE DIFFERENTIATION STAGE
FLUORESCENCE SIGNAL INTENSITY
Fluorescence Signal
LYMPHOCYTE
100 ch
ERYTHROBLAST IN LATE DIFFERENTIATION STAGE
L_MN
m_NE
MONOCYTE
NEUTROPHIL
IMMATURE EOSINOPHIL AND EOSINOPHIL
Side Scatter Signal
256
SIDE SCATTERED LIGHT INTENSITY

FIG. 14

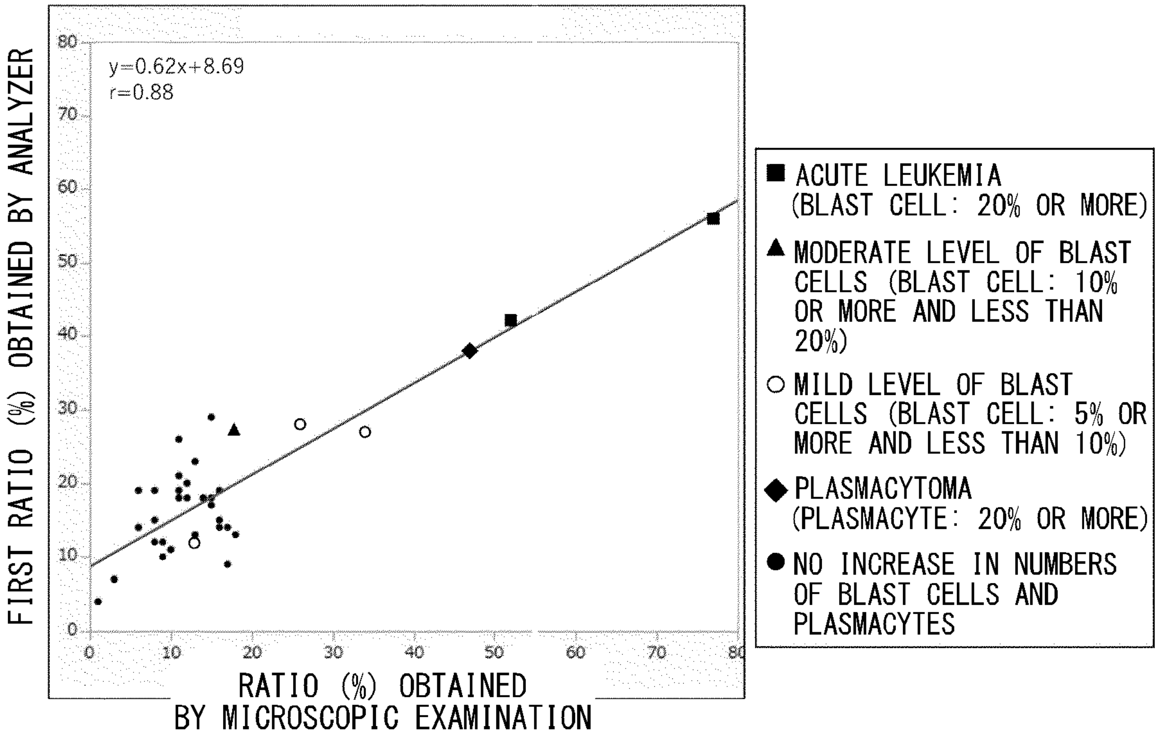
y=0.62x+8.69
r=0.88
FIRST RATIO (%) OBTAINED BY ANALYZER
RATIO (%) OBTAINED BY MICROSCOPIC EXAMINATION
■ ACUTE LEUKEMIA (BLAST CELL: 20% OR MORE)
▲ MODERATE LEVEL OF BLAST CELLS (BLAST CELL: 10% OR MORE AND LESS THAN 20%)
○ MILD LEVEL OF BLAST CELLS (BLAST CELL: 5% OR MORE AND LESS THAN 10%)
◆ PLASMACYTOMA (PLASMACYTE: 20% OR MORE)
● NO INCREASE IN NUMBERS OF BLAST CELLS AND PLASMACYTES

FIG. 15

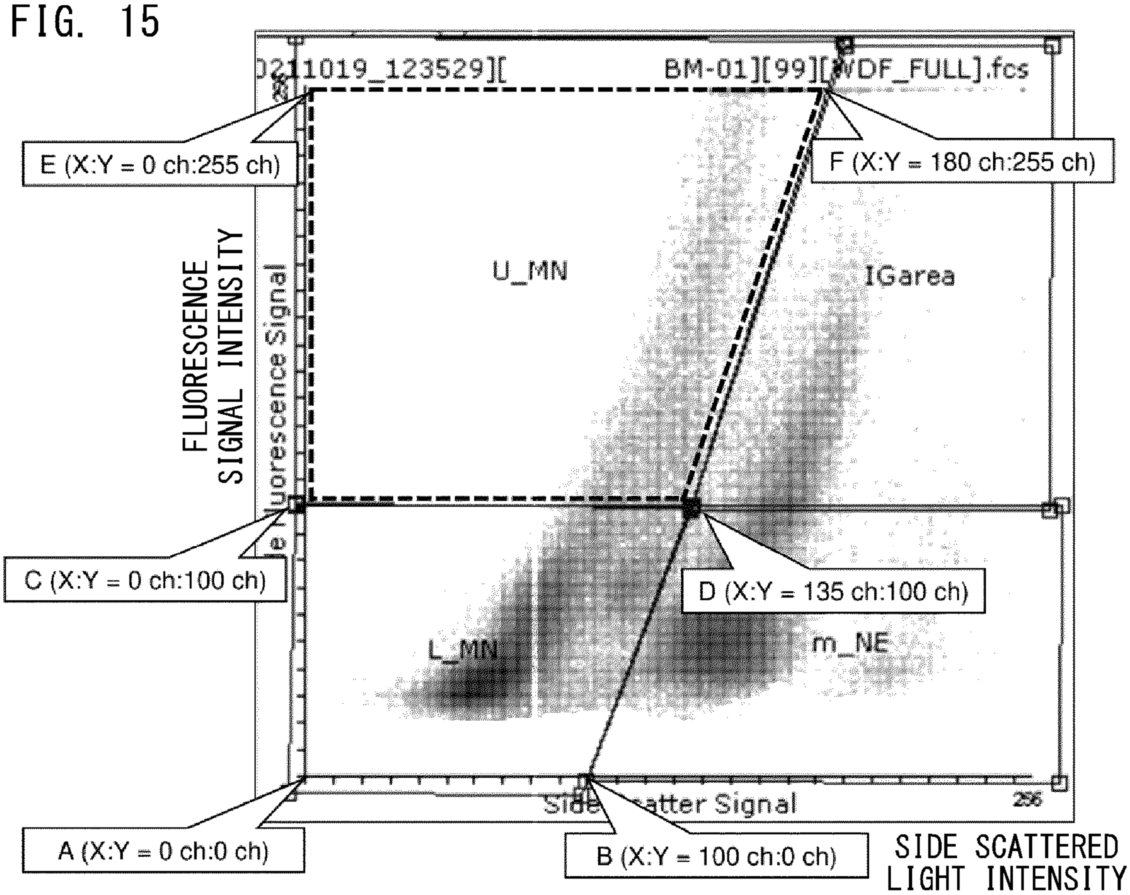
211019_123529][
BM-01][99][WDF_FULL].fcs
E (X:Y = 0 ch:255 ch)
F (X:Y = 180 ch:255 ch)
U_MN
IGarea
FLUORESCENCE SIGNAL INTENSITY
C (X:Y = 0 ch:100 ch)
D (X:Y = 135 ch:100 ch)
L_MN
m_NE
A (X:Y = 0 ch:0 ch)
B (X:Y = 100 ch:0 ch)
SIDE SCATTERED LIGHT INTENSITY

FIG. 16

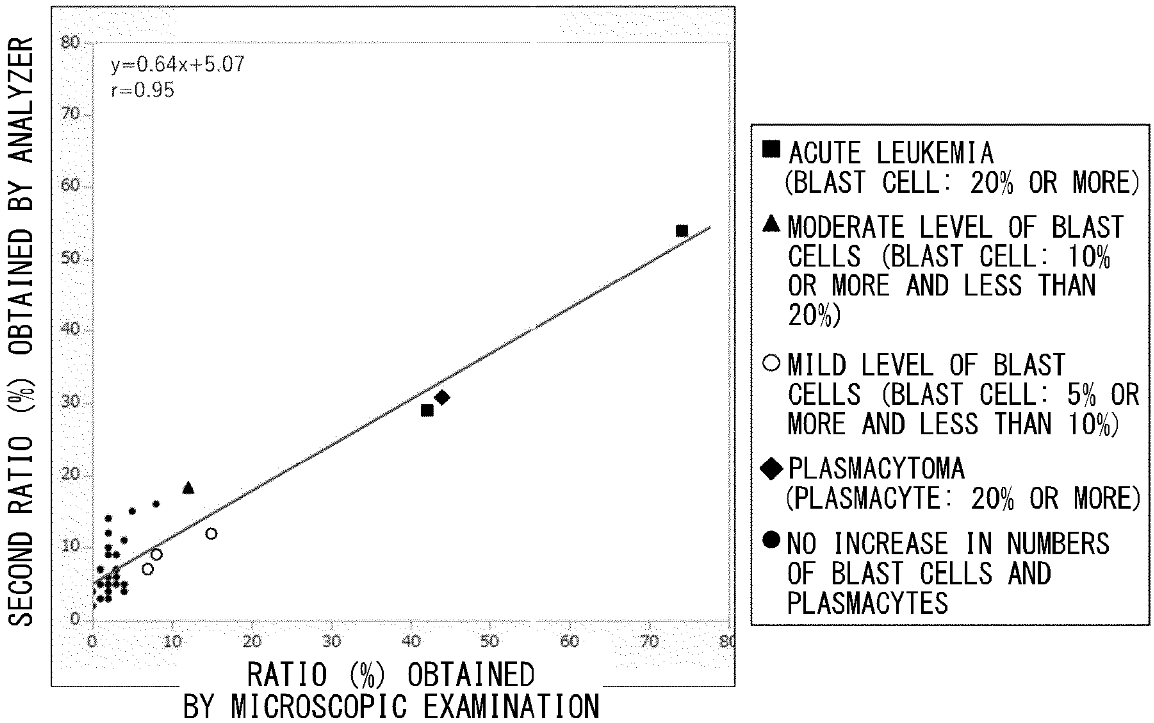
SECOND RATIO (%) OBTAINED BY ANALYZER
y=0.64x+5.07
r=0.95
0
10
20
30
40
50
60
70
80
RATIO (%) OBTAINED BY MICROSCOPIC EXAMINATION
■ ACUTE LEUKEMIA (BLAST CELL: 20% OR MORE)
▲ MODERATE LEVEL OF BLAST CELLS (BLAST CELL: 10% OR MORE AND LESS THAN 20%)
○ MILD LEVEL OF BLAST CELLS (BLAST CELL: 5% OR MORE AND LESS THAN 10%)
◆ PLASMACYTOMA (PLASMACYTE: 20% OR MORE)
● NO INCREASE IN NUMBERS OF BLAST CELLS AND PLASMACYTES

FIG. 17

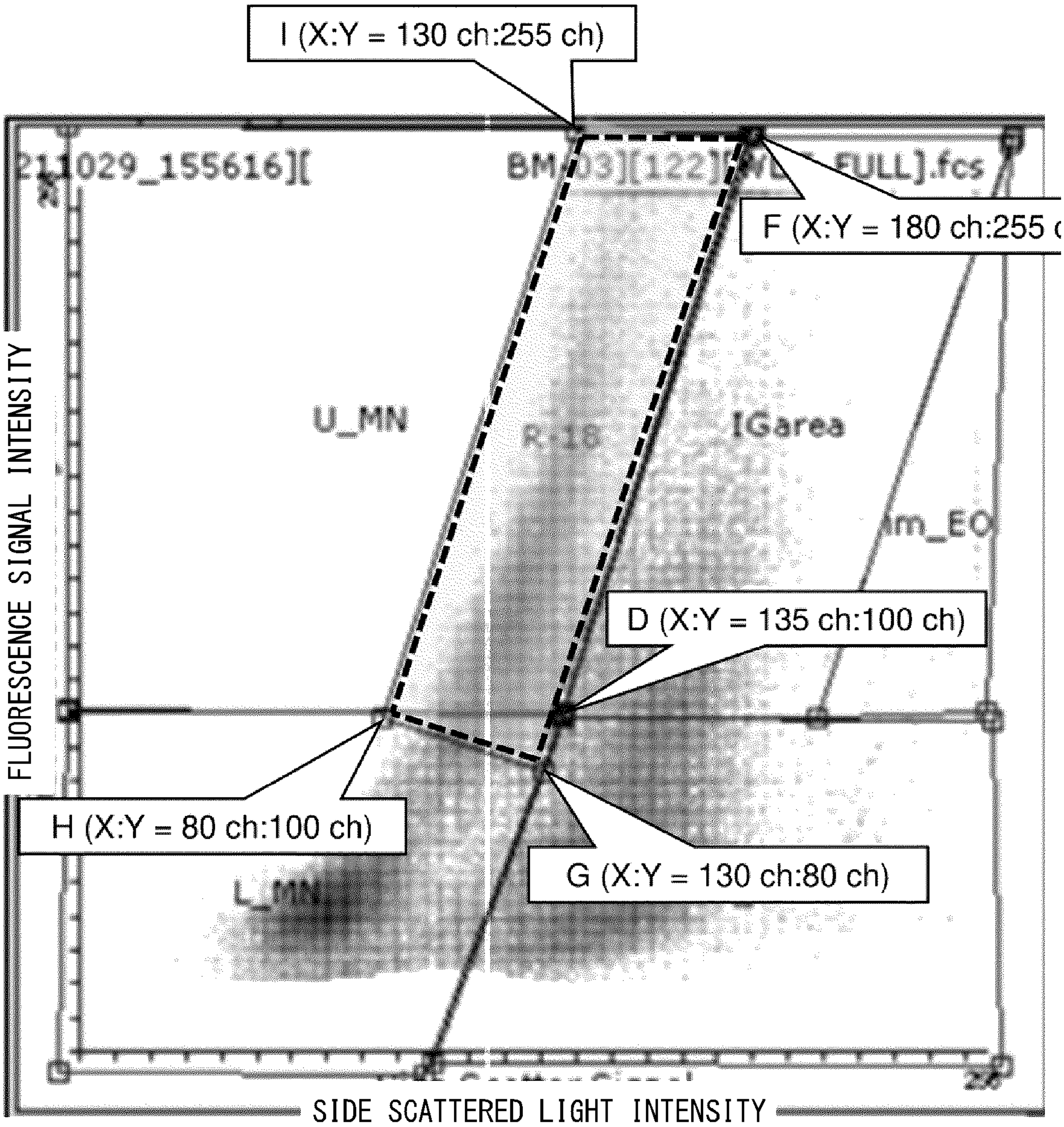
I (X:Y = 130 ch:255 ch)
F (X:Y = 180 ch:255 c
D (X:Y = 135 ch:100 ch)
H (X:Y = 80 ch:100 ch)
G (X:Y = 130 ch:80 ch)
211029_155616][
U_MN
R-18
IGarea
L_MN
FLUORESCENCE SIGNAL INTENSITY
SIDE SCATTERED LIGHT INTENSITY

FIG. 18

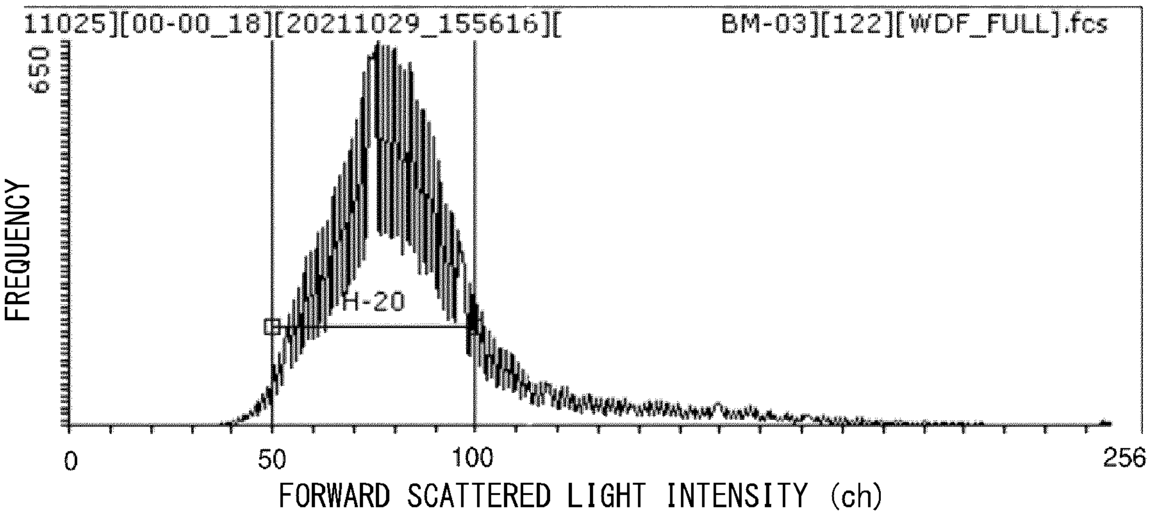
11025][00-00_18][20211029_155616][ BM-03][122][WDF_FULL].fcs
650
FREQUENCY
H-20
0
50
100
256
FORWARD SCATTERED LIGHT INTENSITY (ch)

FIG. 19

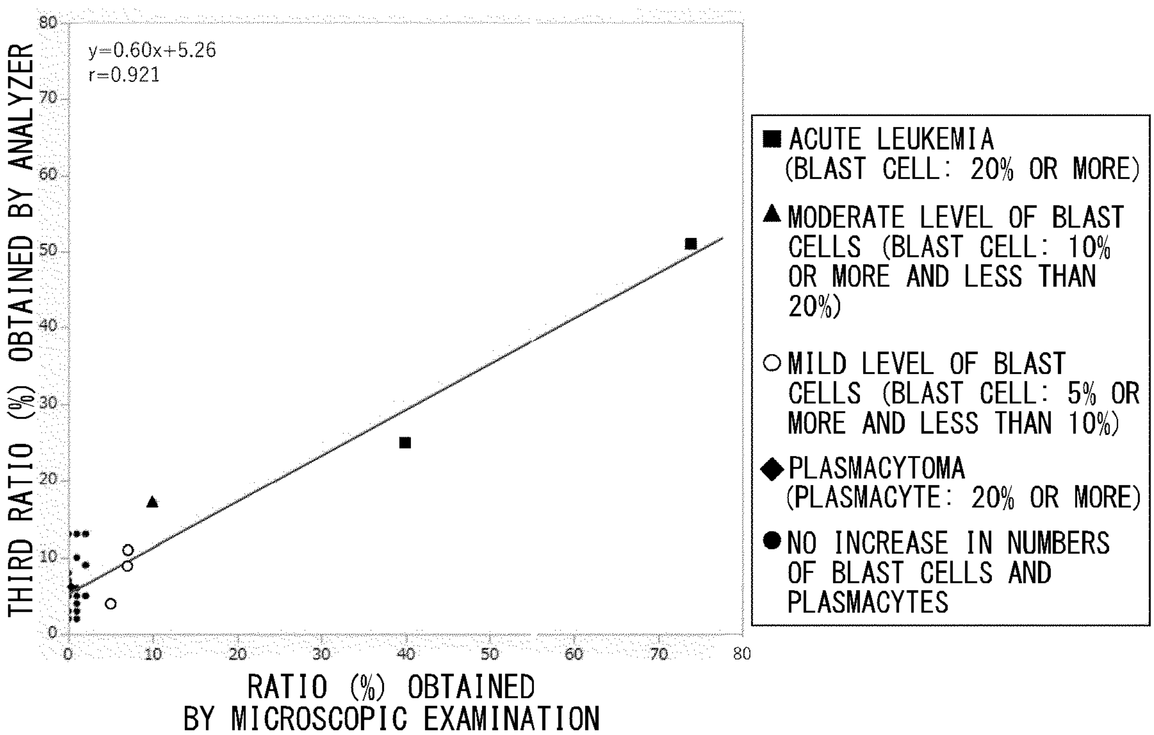
y=0.60x+5.26
r=0.921
THIRD RATIO (%) OBTAINED BY ANALYZER
RATIO (%) OBTAINED BY MICROSCOPIC EXAMINATION
0
10
20
30
40
50
60
70
80
■ ACUTE LEUKEMIA (BLAST CELL: 20% OR MORE)
▲ MODERATE LEVEL OF BLAST CELLS (BLAST CELL: 10% OR MORE AND LESS THAN 20%)
○ MILD LEVEL OF BLAST CELLS (BLAST CELL: 5% OR MORE AND LESS THAN 10%)
◆ PLASMACYTOMA (PLASMACYTE: 20% OR MORE)
● NO INCREASE IN NUMBERS OF BLAST CELLS AND PLASMACYTES

FIG. 20

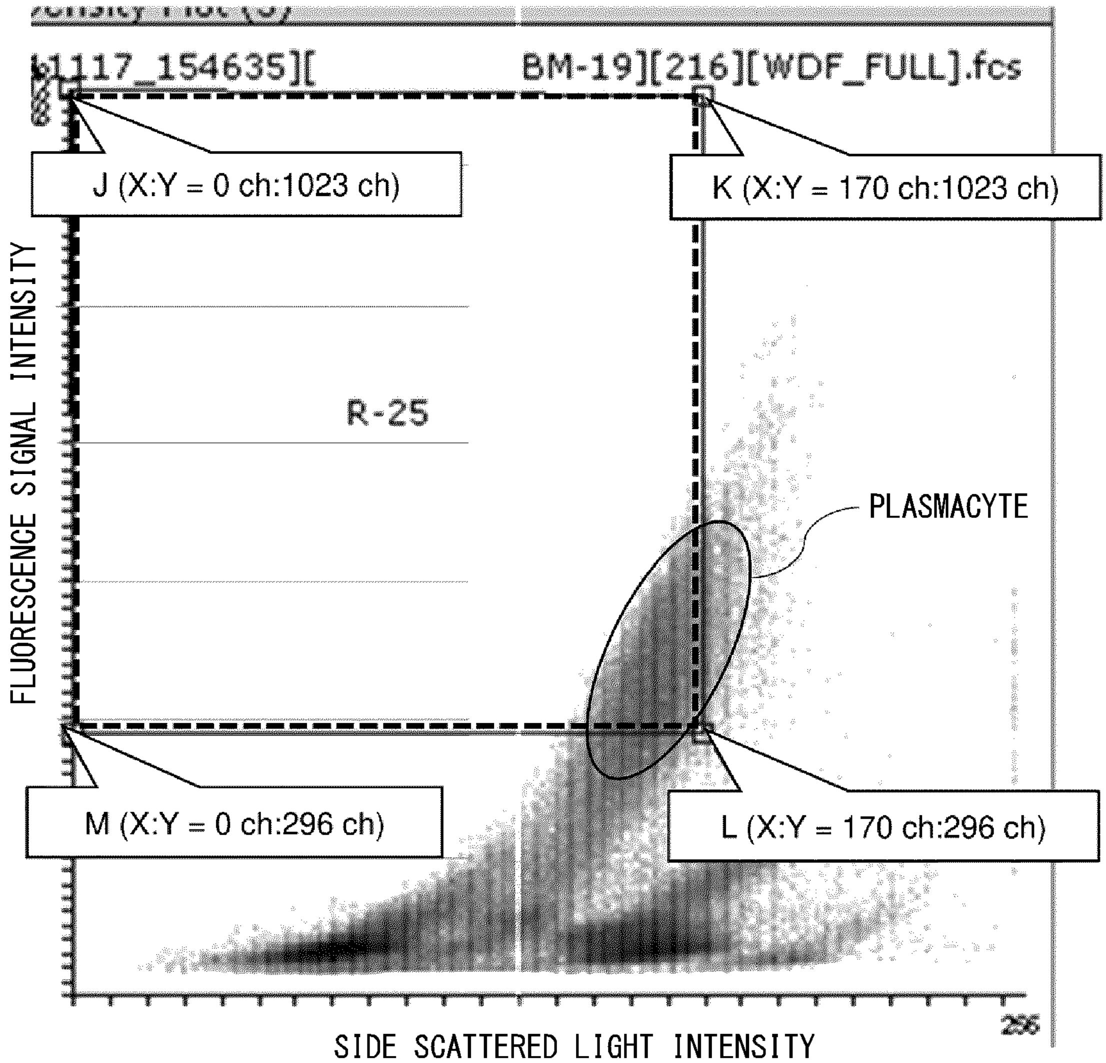
1117_154635][
BM-19][216][WDF_FULL].fcs
J (X:Y = 0 ch:1023 ch)
K (X:Y = 170 ch:1023 ch)
R-25
PLASMACYTE
M (X:Y = 0 ch:296 ch)
L (X:Y = 170 ch:296 ch)
FLUORESCENCE SIGNAL INTENSITY
SIDE SCATTERED LIGHT INTENSITY
256

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022212357 A
- EP 3654014 A1


### Non-patent literature cited in the description


- **BY MORI Y. et al.** Automation of Bone Marrow Aspirate Examination Using the XE-2100 Automated Hematology Analyzer. *Cytometry Part B (Clinical Cytometry)*, 2003, vol. 58B, 25-31